# EUROPEAN PATENT APPLICATION

(11) **EP 4 549 466 A1**
(43) Date of publication of application: **07.05.2025**
(21) Application number: 23830472.9
(22) Date of filing: 29.06.2023
(51) Int. Cl.: C07K 16/28, A61K 39/395, A61P 35/02

(54) **DEVELOPMENT AND USE OF NOVEL MULTISPECIFIC TUMOR INHIBITOR**

(30) Priority: 30.06.2022 CN 202210771160
(71) Applicant: Keymed Biosciences (Chengdu) Co., Ltd., Chengdu, Sichuan 610219 (CN)
(72) Inventor: CHEN, Bo, Chengdu, Sichuan 610219 (CN); WANG, Changyu, Chengdu, Sichuan 610219 (CN); XU, Gang, Chengdu, Sichuan 610219 (CN); LIU, Wei, Chengdu, Sichuan 610219 (CN); SONG, Qin, Chengdu, Sichuan 610219 (CN)
(74) Representative: M. Zardi & Co S.A.
(86) International application number: PCT/CN2023/104243
(87) International publication number: WO 2024/002308

(57) **Abstract**

The present invention relates to an antibody comprising a GPRC5D-binding domain and use thereof. A GPRC5D×CD3 bispecific antibody has a strong killing effect on low-expressing cells and is gentler in activating T cells. A GPRC5D×BCMA×CD3 trispecific antibody can kill BCMA+ cells and GPRC5D+ cells at the same time. The present invention is conducive to overcoming drug resistance caused by single targets and producing more profound and lasting anti-tumor effects in clinical applications

## Description

### TECHNICAL FIELD

The present disclosure relates to an antibody comprising a GPRC5D binding domain and use thereof, and particularly to a multispecific antibody having a GPRC5D binding domain and use thereof.

### BACKGROUND

More and more new therapies are approved and become available, with which the treatment regimen of multiple myeloma is completely changed. For example, ADC therapy Blenrep (belantamab mafodotin) targeting B-cell maturation antigen (BCMA) approved by FDA in 2020, CAR-T therapy Abecma (idecabtagene vicleucel) targeting BCMA approved in 2021, and CAR-T therapy Carvykti (ciltacabtagene autoleucel) targeting BCMA approved recently. However, multiple myeloma is still considered as an incurable disease, and will eventually relapse in most patients. The loss or low expression of BCMA antigen during treatment may lead to the tumor recurrence or drug resistance (van de Donk et al., CAR T-cell therapy for multiple myeloma: state of the art and prospects, Lancet Haematol. 2021 Jun;8(6):e446-e461; Gazeau N et al., Effective anti-BCMA retreatment in multiple myeloma, Blood Adv (2021) 5 (15); and Da Vià MC et al., Homozygous BCMA gene deletion in response to anti-BCMA CAR T cells in a patient with multiple myeloma, Nat Med. 2021 Apr;27(4)). Therefore, it is a reasonable strategy to develop other new targeted therapies independent of BCMA or combination therapies against different targets, to reduce the recurrence or drug resistance of multiple myeloma caused by antigen loss.

G-protein-coupled receptor family C group 5 member D (GPRC5D) is an orphan G-protein-coupled receptor having 7 transmembrane structures (Brauner-Osborne H et al., Cloning and characterization of a human orphan family C G-protein coupled receptor GPRC5D, Biochim Biophys Acta. 2001 Apr 16;1518(3)). The gene is only expressed in cells that produce hard keratin in the hair follicle in skin (Inoue S et al., The RAIG family member, GPRC5D, is associated with hard-keratinized structures, J Invest Dermatol. 2004 Mar;122(3)). In the analysis of gene expression in patients with myeloma, it is found that GPRC5D is highly specifically expressed in multiple myeloma cells and has poor prognosis (Atamaniuk J et al., Overexpression of G protein-coupled receptor 5D in the bone marrow is associated with poor prognosis in patients with multiple myeloma, Eur J Clin Invest. 2012 Sep;42(9); and Cohen Y et al., GPRC5D is a promising marker for monitoring the tumor load and to target multiple myeloma cells, Hematology. 2013 Nov;18(6)). Smith EL et al. further confirmed that GPRC5D receptor protein is selectively and highly expressed in malignant osseous plasmacytoma, which is a new target of immunotherapy independent of the BCMA expression (Smith EL et al., GPRC5D is a target for the immunotherapy of multiple myeloma with rationally designed CAR T cells, Sci Transl Med. 2019 Mar 27;11(485)).

Currently, GPRC5D targeting bispecific antibodies and CAR-T therapy have shown obvious therapeutic effects on multiple myeloma in preclinical studies.

Talquetamab (JNJ-64407564) is a GPRC5D×CD3 bispecific antibody developed by Janssen, which is shown to mediate T cells to effectively kill GPRC5D⁺ myeloma cells in preclinical studies, with a killing effect significantly positively correlated with the expression level of GPRC5D in cells (Pillarisetti K et al., A T-cell-redirecting bispecific G-protein-coupled receptor class 5 member D×CD3 antibody to treat multiple myeloma, Blood. 2020 Apr 9;135(15); and Verkleij CPM et al., Preclinical activity and determinants of response of the GPRC5D×CD3 bispecific antibody Talquetamab in multiple myeloma, Blood Adv. 2021 Apr 27;5(8)).

Kodama T et al. also reported a new GPRC5D×CD3 bispecific antibody, which does not recognize the Cynomolgus GPRC5D receptor and needs to be evaluated by an alternative method for the preclinical safety (Kodama T et al., Anti-GPRC5D/CD3 Bispecific T-Cell-Redirecting Antibody for the Treatment of Multiple Myeloma, Mol Cancer Ther. 2019 Sep;18(9)).

In US20210054094A1, Fertig et al. described 2 anti-GPRC5D antibodies, 5E11 and 5F11 obtained in the immunization of rats, which can bind to human GPRC5D⁺ stably transfected cells with high affinity, but not to Cynomolgus GPRC5D⁺ stably transfected cells; and other two antibodies 10B10 and B72, which bind to Cynomolgus GPRC5D at a low level, and mediate a weak TDCC activity.

Fernández de Larrea C et al. reported that CAR-T therapy targeting both BCMA and GPRC5D can prevent recurrence caused by BCMA escape in a mouse model with transplanted tumor (Fernández de Larrea C et al., Defining an Optimal Dual-Targeted CAR T-cell Therapy Approach Simultaneously Targeting BCMA and GPRC5D to Prevent BCMA Escape-Driven Relapse in Multiple Myeloma, Blood Cancer Discov. 2020 Sep;1(2)).

Although the drugs developed based on the GPRC5D targeting antibody show a therapeutic effect in pre-clinic studies, there is still a need to develop a good clinical treatment method to reduce the recurrence and drug resistance caused by antigen escape, and to reduce the side effects such as cytokine release syndrome and neurotoxicity during the treatment.

### SUMMARY

The present inventor newly finds a number of anti-GPRC5D monoclonal antibodies in studies with different mouse immunization strategies, which can bind to human and Cynomolgus GPRC5D receptors on the cell membrane with high affinity. It is unexpectedly found that a new humanized GPRC5D×CD3 bispecific antibody constructed based on anti-CD3e antibody SP34 (Pessano et al., The T3/T cell receptor complex: antigenic distinction between the two 20-kd T3 (T3-delta and T3-epsilon) subunits, EMBO J. 1985 Feb;4(2)) has a strong killing effect on low expressing cells and is gentle in activating T cells, thus potentially having better clinical efficacy and safety. The present inventor further constructs a tri-specific antibody GPRC5D×BCMA×CD3, which shows a better therapeutic effect and may produce a more lasting response than the BCMA×CD3 bi-specific antibody in a preclinical mouse model.

In an aspect, the present disclosure provides an antibody binding GPRC5D or an antigen binding fragment thereof.

In another aspect, the present disclosure provides a nucleic acid encoding the antibody or antigen binding fragment thereof.

In another aspect, the present disclosure provides a multispecific antibody or an antigen binding fragment thereof, which includes at least:
(a) the antibody binding GPRC5D antigen or antigen binding fragment thereof, which is a first antigen binding portion, where the GPRC5D antigen is a first antigen, the first antigen binding portion includes a first heavy chain variable region and a first light chain variable region, and the first antigen binding portion includes a first binding domain binding the first antigen; and
(b) an antibody binding CD3 antigen or an antigen binding fragment thereof, which is a second antigen binding portion, where the CD3 antigen is a second antigen, the second antigen binding portion includes a second heavy chain variable region and a second light chain variable region, and the second antigen binding portion includes a second binding domain binding the second antigen.

Further preferably the multispecific antibody or antigen binding fragment thereof further includes:
(c) an antibody binding BCMA antigen or an antigen binding fragment thereof, which is a third antigen binding portion, where the BCMA antigen is a third antigen, the third antigen binding portion includes a third heavy chain variable region and a third light chain variable region, and the third antigen binding portion includes a third binding domain binding the third antigen.

In another aspect, the present disclosure provides a nucleic acid encoding the multispecific antibody or antigen binding fragment thereof.

In another aspect, the present disclosure provides a vector carrying the nucleic acid.

In another aspect, the present disclosure provides a cell comprising the vector.

In another aspect, the present disclosure provides a composition comprising the antibody binding GPRC5D or antigen binding fragment thereof, the multispecific antibody or antigen binding fragment thereof, the nucleic acid and/or the cell.

In another aspect, the present disclosure provides a kit comprising the antibody binding GPRC5D or antigen binding fragment thereof, the multispecific antibody or antigen binding fragment thereof, the nucleic acid, the cell and/or the composition.

In another aspect, the present disclosure provides use of the antibody binding GPRC5D or antigen binding fragment thereof, the multispecific antibody or antigen binding fragment thereof, the nucleic acid, the cell and/or the composition in the preparation of a drug or a kit for diagnosing, treating or preventing cancers or autoimmune diseases.

### BRIEF DESCRIPTION OF THE DRAWINGS

Fig. 1 shows the detection results of flow cytometry of human and Cynomolgus GPRC5D HEK293 stably transfected cell line.
Fig. 2 shows the detection results of flow cytometry of human and Cynomolgus GPRC5D CHO stably transfected cell line.
Fig. 3 shows the binding of anti-GPRC5D chimeric antibody with GPRC5D stably transfected cell line, where Fig. 3A shows the binding of anti-GPRC5D chimeric antibody with human GPRC5D stably transfected cell line; and Fig. 3B shows the binding of anti-GPRC5D chimeric antibody with Cynomolgus GPRC5D stably transfected cell line.
Fig. 4 shows the binding of GPRC5D×CD3κλ bispecific antibody with MM.1R tumor cells.
Fig. 5 shows that GPRC5D×CD3κλ bispecific antibody mediates T cells to kill MM.1R tumor cells.
Fig. 6 shows the binding of 2F1 GPRC5D×CD3κλ bispecific antibody and a variant thereof with MM.1R tumor cells.
Fig. 7 shows the binding of 2F1 GPRC5D×CD3κλ bispecific antibody variant with GPRC5D stably transfected cell line, where Fig. 7A shows the binding of 2F1 GPRC5D×CD3κλ bispecific antibody variant with human GPRC5D stably transfected cell line; and Fig. 7B shows the binding of 2F1 GPRC5D×CD3κλ bispecific antibody variant with Cynomolgus GPRC5D stably transfected cell line.
Fig. 8 shows the binding of 2F1 GPRC5D×CD3κλ bispecific antibody variant with Jurkat cells.
Fig. 9 shows TDCC mediated by 2F1 GPRC5D×CD3κλ bispecific antibody variant, where Fig. 9A shows the TDCC activity of T cells mediated by 2F1 GPRC5D×CD3κλ bispecific antibody variant to kill MM.1R tumor cells; and Fig. 9B shows the changes in expression of activated receptors CD69 and CD25 on CD4⁺T and CD8⁺ cell surface in the process of 2F1 GPRC5D×CD3κλ bispecific antibody variant mediating T cells to kill MM.1R tumor cells.
Fig. 10 shows the activation of T cell NFAT singling pathway by 2F1 GPRC5D×CD3κλ bispecific antibody variant.
Fig. 11 shows the inhibition of 2F1 GPRC5D×CD3κλ bispecific antibody variant on tumor growth in a mouse model with transplanted tumor.
Fig. 12 shows the changes in weight of mice after administration of 2F1 GPRC5D×CD3κλ bispecific antibody variant in a mouse model with transplanted tumor.
Fig. 13 shows the structure of GPRC5D×BCMA×CD3 triTCE trispecific antibody.
Fig. 14 shows the binding of GPRC5D×BCMA×CD3 triTCE antibody with GPRC5D⁺ stably transfected cells.
Fig. 15 shows the binding of GPRC5D×BCMA×CD3 triTCE antibody with BCMA⁺ stably transfected cells.
Fig. 16 shows the binding of GPRC5D×BCMA×CD3 triTCE antibody with Jurkat cells.

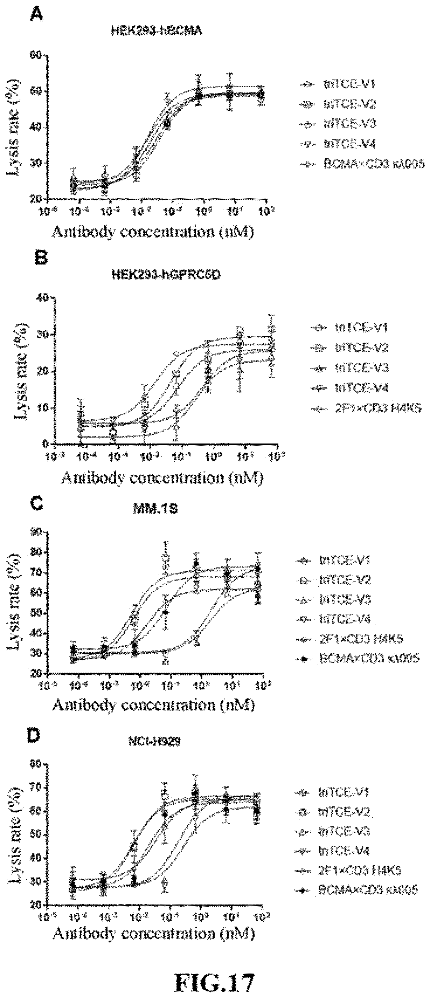

Fig. 17 shows the TDCC activities of GPRC5D×BCMA×CD3 triTCE antibody in GPRC5D⁺ stably transfected cell lines, BCMA⁺ stably transfected cell lines and GPRC5D⁺/BCMA⁺ tumor cells, where Fig. 17A shows the killing effect of GPRC5D×BCMA×CD3 triTCE antibody on BCMA⁺ monopositive target cells; Fig. 17B shows the killing effect of GPRC5D×BCMA×CD3 triTCE antibody on GPRC5D⁺ monopositive target cells; Fig. 17C shows the killing effect of GPRC5D×BCMA×CD3 triTCE antibody on myeloma cell line MM.1S expressing both GPRC5D and BCMA receptors; and Fig. 17D shows the killing effect of GPRC5D×BCMA×CD3 triTCE antibody on myeloma cell line NCI-H929 expressing both GPRC5D and BCMA receptors.

### DETAILED DESCRIPTION

### I. Definitions

In the present disclosure, unless otherwise indicated, scientific and technical terms used herein have the meanings commonly understood by those skilled in the art. In addition, the protein and nucleic acid chemistry, molecular biology, cell and tissue culture, microbiology and immunology related terms and laboratory operation steps used herein are all widely used terms and routine steps in the corresponding art. Meanwhile, for better understanding of the present disclosure, definitions and explanations of related terms are provided below.

An antibody (e.g., monoclonal antibody) that specifically binds to GPRC5D and an antigen-binding fragment thereof are provided herein. Specifically, a monoclonal anti-GPRC5D antibody that specifically binds to GPRC5D is provided herein, where the anti-GPRC5D antibody comprises a variant of a parent antibody. Specifically, an antibody that specifically binds to GPRC5D (for example, human GPRC5D) is provided herein. Specifically, an anti-GPRC5D antibody comprising one or more modification(s) of amino acid residue(s) (for example, 1-3 amino acid residue(s) in the framework region of the heavy chain variable region are replaced) is/are provided herein. Compared with the parent antibody without the modification(s), it retains the affinity to the antigen.

As used herein and unless otherwise specified, the term "about" or "approximate" means that a given value or range is within plus or minus 10% of the value or range. Where an integer is required, the term refers to the closest integer obtained by rounding up or down a given value or range to within plus or minus 10% of the value or range.

For a polypeptide sequence of an antibody chain, the phrase "substantially identical" can be understood as an antibody chain showing at least 60%, 65%, 70%, 75%, 80%, 85%, 90%, 95%, 96%, 97%, 98%, 99%, or more sequence identity with a reference polypeptide sequence. For a nucleic acid sequence, the term can be understood as a nucleotide sequence showing at least 60%, 65%, 70%, 75%, 80%, 85%, 90%, 95%, 96%, 97%, 98%, 99% or higher sequence identity with a reference nucleic acid sequence.

The sequence "similarity" or "identity" has the meaning generally known in the art, and the percentage of sequence similarity between two nucleic acid or polypeptide molecules or regions can be calculated by using a disclosed technology. The sequence similarity can be determined along the full length of a polynucleotide or polypeptide or along a region of the molecule. Although there are many methods to measure the similarity between two polynucleotides or polypeptides, the term "similarity" is well known to the skilled person (Carrillo, H. & Lipman, D., SIAM J Applied Math 48:1073 (1988)).

A "substitution-type" variant is one in which at least one amino acid residue in a naturally occurring sequence is removed and a different amino acid is inserted at the same position. The substitution may be singular, where only one amino acid in the molecule is substituted; or plural, where two or more amino acids in the same molecule are substituted. Multiple substitutions can be occur at consecutive sites. Similarly, one amino acid can be substituted by multiple residues, where such variants include both substitution and insertion. An "insertion-type" variant is one in which one or more amino acids are inserted adjacent to an amino acid at a specific position in a natural sequence. Adjacent to an amino acid means to be linked to the α-carboxyl or α-amino functional group of the amino acid. A "deletion-type" variant is one in which one or more amino acids in a natural amino acid sequence are removed. Usually, a deletion-type variant has one or two amino acids deleted in a specific region of its molecule.

For a variable domain of an antibody, the term "variable" refers to some parts of related molecules with wide sequence differences between antibodies, and is used for specific recognition and binding of particular antibodies to their specific targets. However, variable is not evenly distributed throughout the variable domain of the antibody. Variable is concentrated in regions called complementarity determining regions (CDRs; namely CDR1, CDR2, and CDR3) or hypervariable regions, all of which are located in the variable domains of light chain and heavy chain. A more conserved portion in the variable domain is called the framework region (FR) or framework sequence. Each variable domain of a natural heavy chain and light chain includes four FR regions, which mainly adopt β-folded configuration, and connected by three CDRs, where the CDRs form a loop, which connects the β-folded structures and forms a part of the β-folded structure in some cases. CDRs of each chain are usually connected in the vicinity by the FR region, and promote the formation of a site of the antibody binding to a target (epitope or determinant). As used herein, the numbering of amino acid residues in immunoglobulin is based on the numbering system of amino acid residues in immunoglobulin by Kabat et al., unless otherwise specified. A CDR can have the ability to specifically bind to an associated epitope.

As used herein, the "antibody fragment" or "antigen binding fragment" of an antibody refers to any part of a full-length antibody that is shorter than the full length, but at least contains partial variable region of the antibody that binds an antigen (e.g., one or more CDRs and/or one or more binding sites of the antibody), and thus retains the binding specificity and at least partial specific binding ability of the full-length antibody. Therefore, the antigen binding fragment refers to an antibody fragment containing an antigen-binding portion that binds to the same antigen as the antibody from which the antibody fragment is derived. The antibody fragment includes an antibody derivative produced by enzymatic treatment of a full-length antibody and a synthetic derivative, such as a recombinant derivative. The antibody includes an antibody fragment. Examples of the antibody fragment include, but are not limited to, Fab, Fab', F(ab')₂, single-chain Fv(scFv), Fv, dsFv, diabodies, Fd and Fd' fragment, and other fragments, including modified fragments (see, for example, Methods in Molecular Biology, Vol 207: Recombinant Antibodies for Cancer Therapy Methods and Protocols (2003); Chapter 1; p 3-25, Kipriyanov). The fragment may include multiple chains linked together, for example, by a disulfide bond and/or by a peptide linker. The antibody fragment generally contains at least or about 50 amino acids, and typically at least or about 200 amino acids. The antigen binding fragment includes any antibody fragment that produces an antibody immunospecifically binding to an antigen (at a Ka of at least or at least about 10⁷-10⁸M-1) when inserted into an antibody framework (e.g., by replacing a corresponding region). The "functional fragment" or "analog of an anti-GPRC5D antibody" is a fragment or analog that can prevent or substantially reduce the ability of the receptor to bind the ligand or initiate the signal transduction. As used herein, the functional fragment generally has the same meaning as the "antibody fragment"; and for an antibody, it may refer to a fragment that can prevent or substantially reduce the ability of the receptor to bind a ligand or initiate the signal transduction, such as Fv, Fab, F(ab')₂, and the like. The "Fv" fragment consists of a dimer (V_{H}-V_{L} dimer) formed by non-covalent binding of a variable domain of a heavy chain and a variable domain of a light chain. In this configuration, three CDRs of each variable domain interact to determine a target binding site on the surface of the V_{H}-V_{L} dimer, as does in the case of an intact antibody. The six CDRs together endow the intact antibody with a target binding specificity. However, even a single variable domain (or half Fv only including 3 target-specific CDRs) can still have the ability to recognize and bind a target.

As used herein, the "monoclonal antibody" refers to a group of identical antibodies, where each individual antibody molecule in the monoclonal antibody group is the same as other antibody molecules. This feature is contrary to that of a polyclonal group of antibodies, which contains antibodies with many different sequences. The monoclonal antibody can be prepared by many well-known methods (Smith et al. (2004) J.Clin.Pathol.57, 912-917; and Nelson et al., J Clin Pathol (2000), 53, 111-117). For example, the monoclonal antibody can be prepared by immortalizing B cells, for example, by fusing with myeloma cells to produce a hybridoma cell line or by infecting B cells with viruses such as EBV. The recombinant technology can also be used to prepare an antibody from a clonal population of host cells in vitro by transforming the host cells with a plasmid carrying an artificial sequence of nucleotides encoding the antibody.

As used herein, the full-length antibody is an antibody having two full-length heavy chains (for example, VH-CH1-CH2-CH3 or VH-CH1-CH2-CH3-CH4), two full-length light chains (VL-CL) and a hinge region, such as an antibody naturally produced by antibody secreting B cells and a synthesized antibody with the same domain.

The term "chimeric antibody" refers to an antibody in which the variable region sequence is derived from one species and the constant region sequence is derived from another species, such as an antibody in which the variable region sequence is derived from a mouse antibody and the constant region sequence is derived from a human antibody.

The "humanized" antibody refers to a non-human (e.g. mouse) antibody form, which is a chimeric immunoglobulin, immunoglobulin chain or fragment thereof (e.g. Fv, Fab, Fab', F(ab')₂ or other antigen-binding subsequences of the antibody), and contains the smallest sequence derived from non-human immunoglobulin. Preferably, the humanized antibody is a human immunoglobulin (receptor antibody), in which the residue of the complementarity determining region (CDR) of the receptor antibody is replaced by a CDR residue from a non-human species (donor antibody) with desired specificity, affinity and ability, such as mouse, rat or rabbit.

Additionally, in humanization, it is also possible to mutate the amino acid residues in CDR1, CDR2 and/or CDR3 region of VH and/or VL, thereby improving one or more binding characteristics (such as affinity) of the antibody. Mutations can be introduced by for example PCR-mediated mutations, and their effects on the binding or other functional characteristics of the antibody can be evaluated by in-vitro or in-vivo tests described herein. Generally, conservative mutations are introduced. Such mutations can be amino acid substitutions, additions, or deletions. In addition, there are usually no more than one or two mutations in CDR. Therefore, the humanized antibody described in the present disclosure also covers an antibody containing 1 or 2 amino acid mutations in the CDR.

"Conservative amino acid substitution" may involve substitution of a native amino acid residue with a non-native residue such that there is little or no effect on the polarity or charge of the amino acid residue at the position. Examples of conservative substitutions include the substitution of one nonpolar (hydrophobic) amino acid residue such as isoleucine, valine, leucine, norleucine, alanine or methionine with another nonpolar amino acid residue; and the substitution of one polar (hydrophilic) amino acid residue with another polar amino acid residue, such as substitution of arginine with or for lysine, substitution of glutamine with or for asparagine, and substitution of glycine with or for serine; the substitution of one basic amino acid residue such as lysine, arginine, or histidine with another basic amino acid residue; or the substitution of one acidic residue such as aspartate or glutamate with another acidic residue. The phrase "conservative amino acid substitution" also includes the substitution of a chemically derivatized residue for a non-derivatized residue, provided that the polypeptide exhibits the required biological activity. Other exemplary amino acid substitutions that can be used according to the present invention are shown in Table 1 below.

**Table 1. Some applicable amino acid substitutions**

| Original residue | Exemplary substitution |
|---|---|
| Ala | Val, Leu, Ile |
| Arg | Lys, Gln, Asn |
| Asn | Gln |
| Asp | Glu |
| Cys | Ser, Ala |
| Gln | Asn |
| Glu | Asp |
| Gly | Pro, Ala |
| His | Asn, Gln, Lys, Arg |
| Ile | Leu, Val, Met, Ala, Phe, N-Leucine |
| Leu | norleucine, Ile, Val, Met, Ala, Phe |
| Lys | Arg, 1,4-diamino-butyric acid, Gln, Asn |
| Met | Leu, Phe, Ile |
| Phe | Leu, Val, Ile, Ala, Tyr |
| Pro | Ala |
| Ser | Thr, Ala, Cys |
| Thr | Ser |
| Trp | Tyr, Phe |
| Tyr | Trp, Phe, Thr, Ser |
| Val | Ile, Met, Leu, Phe, Ala, N-Leucine |

As used herein, the term "CDR" refers to a complementarity-determining region, and it is known that each heavy chain or light chain of an antibody molecule has 3 CDRs. CDR is also called hypervariable region and exists in the variable region of each heavy chain and light chain of the antibody, and a highly variable site is present in the primary structure of CDR. In the specification, CDRs of the heavy chain are represented by CDR1, CDR2, and CDR3 at the N terminus of the N-terminal sequence of the heavy chain, and CDRs of the light chain are represented by CDR1, CDR2, and CDR3 at the N terminus of the N-terminal sequence of the light chain. These sites are close to each other in the tertiary structure and determine the specificity to the antigen bound by the antibody.

As used herein, the term "epitope" refers to any antigenic determinant on an antigen to which a complementarity site of an antibody binds. The antigenic determinant usually includes the typing of the chemically active surface of a molecule, such as an amino acid or sugar side chain, and usually has specific three-dimensional structural characteristics and specific charge characteristics.

As used herein, the "specific binding" or "immunospecific binding" with respect to an antibody or antigen-binding fragment thereof are used interchangeably herein, and refer to the ability of an antibody or antigen-binding fragment thereof to form one or more non-covalent bonds with an alloantigen through the noncovalent interactions between the antibody and the antibody-binding site of the antigen. The antigen may be an isolated antigen or present in tumor cells. Generally, an antibody immunospecifically binding (or specifically binds) an antigen binds to the antigen with an affinity constant Ka of about 1 × 10⁷M⁻¹ or 1 × 10⁸M⁻¹ or higher (or a dissociation constant (Kd) of 1 × 10⁻⁷M or 1 × 10⁻⁸M or lower). The affinity constant can be determined by a standard kinetic method for an antibody reaction, such as immunoassay, surface plasmon resonance (SPR) (Rich and Myszka (2000) Curr. Opin.Biotechnol 11:54; Englebienne (1998) Analyst. 123:1599), isothermal titration calorimetry (ITC) or other kinetic interaction assays known in the art (see, for example, Paul, ed., Fundamental Immunology, 2nd ed., Raven Press, New York, pages 332-336(1989); and U.S. Pat. No. 7, 229, 619, in which exemplary SPR and ITC methods for calculating the binding affinity of antibodies are described). Instruments and methods for real-time detection and monitoring of the binding rate are known and commercially available (see Malmqvist (2000) Biochem.Soc.Trans. 27:335).

As used herein, the terms "polynucleotide" and "nucleic acid molecule" refer to an oligomer or polymer containing at least two linked nucleotides or nucleotide derivatives, including deoxyribonucleic acids (DNAs) and ribonucleic acids (RNAs) which are usually linked together by a phosphodiester bond. As used herein, the term "nucleic acid molecule" is intended to include both DNA molecules and RNA molecules. The nucleic acid molecules may be single-stranded or double-stranded, and can be cDNA.

Alternatively, in another embodiment, mutations can be randomly introduced along all or part of the coding sequence of the anti-GPRC5D antibody by, for example, saturation mutagenesis, and the resulting modified anti-GPRC5D antibody can be screened for improved binding activity.

As used herein, "expression" refers to a process of producing a polypeptide through the transcription and translation of a polynucleotide. The expression level of a polypeptide can be evaluated by any method known in the art, including, for example, a method for determining the amount of a polypeptide produced in a host cell. Such methods may include, but are not limited to, quantification of a polypeptide in a cell lysate by ELISA, Coomassie blue staining after gel electrophoresis, Lowry protein assay, and Bradford protein array.

As used herein, the "host cell" is a cell for receiving, maintaining, replicating and amplifying a vector. The host cell can also be used to express a polypeptide encoded by a vector. When the host cell divides, the nucleic acid contained in the vector replicates, thereby amplifying the nucleic acid. The host cell can be a eukaryotic cell or a prokaryotic cell. Suitable host cells include, but are not limited to, CHO cells, various COS cells, HeLa cells and HEK cells such as HEK 293 cells.

As used herein, the "vector" is a replicable nucleic acid. When the vector is transformed into an appropriate host cell, one or more heterologous proteins can be expressed. The vectors include those into which a nucleic acid encoding a polypeptide or a fragment thereof can be introduced by restriction digestion and ligation. The vectors further include those containing a nucleic acid encoding a polypeptide. The vector is used to introduce a nucleic acid encoding a polypeptide into a host cell, to amplify the nucleic acid or to express/present the polypeptide encoded by the nucleic acid. The vector usually remains free, but it can be designed to integrate a gene or a part thereof into a chromosome of the genome. Artificial chromosome vectors are contemplated, such as yeast artificial vectors and mammalian artificial chromosomes. The choice and use of such vehicles are well known to those skilled in the art.

As used herein, the vectors also include "virus vectors" or "viral vectors". The viral vector is an engineered virus, which is operably linked to a foreign gene to transfer (as a vehicle or shuttle) the foreign gene into a cell.

As used herein, the "expression vector" includes a vector capable of expressing DNA operably linked to a regulatory sequence such as a promoter region that affects the expression of such DNA fragments. Such an additional fragment may include a promoter and a terminator sequence, and optionally include one or more origins of replication, one or more selective markers, an enhancer, a polyadenylation signal, and the like. The expression vectors are generally derived from plasmids or viral DNA, or may be elements containing the two. Therefore, the expression vector refers to a recombinant DNA or RNA construct, such as a plasmid, bacteriophage, recombinant virus or other vectors. When introduced into an appropriate host cell, the cloned DNA is expressed. Suitable expression vectors are well known to those skilled in the art, and include expression vectors that are replicable in eukaryotic cells and/or prokaryotic cells as well as expression vectors that remain free or expression vectors that are integrated into the genome of the host cell.

As used herein, "treating" an individual suffering from a disease or disease condition means that the symptoms of the individual are partially or completely relieved, or remain unchanged after treatment. Therefore, the treatment includes prevention, treatment and/or curing. Prevention refers to the prevention of the occurrence of potential diseases and/or the prevention of the deterioration of symptoms or the disease development. The treatment also includes any antibody or antigen-binding fragment thereof provided and any pharmaceutical use of the composition provided herein.

As used herein, "therapeutic effect" means an effect caused by the treatment of an individual, which changes, usually improves or ameliorates the symptoms of a disease or disease condition, or cures the disease or disease condition.

As used herein, "therapeutically effective amount" or "therapeutically effective dose" refers to an amount of a substance, a compound, a material or a composition containing a compound that is at least sufficient to produce a therapeutic effect after being administered to a subject. Therefore, it is an amount necessary to prevent, cure, improve, arrest or partially arrest the symptoms of a disease or disorder.

As used herein, "prophylactically effective amount" or "prophylactically effective dose" refers to an amount of a substance, a compound, a material or a composition containing a compound that produces a desired preventive effect after being administered to a subject. For example, it can prevent or delay the occurrence or recurrence of a disease or symptom, and reduce the possibility of occurrence or recurrence of a disease or symptom. A fully prophylactically effective dose need not occur by administering one dose, and may occur simply after administering a series of doses. Therefore, the prophylactically effective amount can be administered in one or more administrations.

As used herein, the term "patient" or "subject" refers to a mammal, such as a human.

As used herein and unless otherwise specified, the terms "including", "including", "having" and "containing", including their grammatical equivalents, should generally be understood to be open and non-limiting, for example, not excluding other unlisted elements or steps.

### II. Detailed description of specific Implementations

In an aspect, the present disclosure provides an antibody binding GPRC5D antigen or an antigen binding fragment thereof, which comprises:
(1) a heavy chain variable region and a light chain variable region, where
   (i) the heavy chain variable region comprises:
      (a) an HCDR1 sequence having a formula of:
         TSYNMH (SEQ ID NO. 5);
      (b) an HCDR2 sequence having a formula of:
         YIYPGNGGTNYNQX₁FX₂X₃(SEQ ID NO. 242), where
         X₁ is K or N, X₂ is K or Q, and X₃ is A or G; and
      (c) an HCDR3 sequence having a formula of:
         GYGYRYWYFDV(SEQ ID NO. 7); and
   (ii) the light chain variable region comprises:
      (a) an LCDR1 sequence having a formula of:
         X₄ASSSVX₅X₆MH (SEQ ID NO. 243), where
         X₄ is S or R, X₅ is S or R, and X₆ is Y or F;
      (b) an LCDR2 sequence having a formula of:
         DTSKX₇AS(SEQ ID NO. 244), where
         X₇ is V or L; and
      (c) an LCDR3 sequence having a formula of:
         QQWX₈X₉X₁₀PX₁₁T (SEQ ID NO.245), where
         X₈ is S or N, X₉ is S or N, X₁₀ is N, YorK, and X₁₁ is L or P; or
(2) a heavy chain variable region and a light chain variable region, where
   (i) the heavy chain variable region comprises:
      (a) an HCDR1 sequence having a formula of:
         TGYTMN (SEQ ID NO. 25);
      (b) an HCDR2 sequence having a formula of:
         LINPYNGGX₁₂X₁₃YNX₁₄KFX₁₅G (SEQ ID NO.246), where
         X₁₂ is S or T, X₁₃ is R, S or N, X₁₄ is Q or L, and X₁₅ is K or Q; and
      (c) an HCDR3 sequence having a formula of:
         X₁₆X₁₇X₁₈RX₁₉X₂₀MDY (SEQ ID NO. 247), where
         X₁₆ is W or L, X₁₇ is A, P or G, X₁₈ is I or L, X₁₉ is Y or N, and X₂₀ is A or G; and
   (ii) the light chain variable region comprises:
      (a) an LCDR1 sequence having a formula of:
         X₂₁ASQX₂₂X₂₃X₂₄X₂₅NX₂₆X₂₇ (SEQ ID NO. 248), where
         X₂₁ is K, R or Q, X₂₂ is N or S, X₂₃ is V or I, X₂₄ is G or S, X₂₅ is T or N, X₂₆ is V or L, and X₂₇ is A or H;
      (b) an LCDR2 sequence having a formula of:
         X₂₈ASX₂₉X₃₀X₃₁S (SEQ ID NO. 249), where
         X₂₈ is F, Y or S, X₂₉ is Y, L or Q, X₃₀ is R, L or S, and is N or I; and
      (c) an LCDR3 sequence having a formula of:
         QQX₃₂NSX₃₃X₃₄X₃₅T (SEQ ID NO. 250), where
         X₃₂ is Y or S, X₃₃ is Y or W, X₃₄ is L or P, and X₃₅ is L or Q; or
(3) a heavy chain variable region and a light chain variable region, where
   (i) the heavy chain variable region comprises:
      (a) an HCDR1 sequence having a formula of:
         TNFPIE (SEQ ID NO. 42);
      (b) an HCDR2 sequence having a formula of:
         NFHPYNDVTKYNEKFX₃₆G (SEQ ID NO. 251), where
         X₃₆ is K or Q; and
      (c) an HCDR3 sequence having a formula of:
         ITRGY (SEQ ID NO. 44); and
   (ii) the light chain variable region comprises:
      (a) an LCDR1 sequence having a formula of:
         KSSQSLLYSRNQKNSLA (SEQ ID NO. 47);
      (b) an LCDR2 sequence having a formula of:
         WASTRES (SEQ ID NO. 48); and
      (c) an LCDR3 sequence having a formula of:
         QQYYSYPLT (SEQ ID NO.49); or
(4) a heavy chain variable region and a light chain variable region, where
   (i) the heavy chain variable region comprises:
      (a) an HCDR1 sequence having a formula of:
         TSYX₃₇MY (SEQ ID NO. 252), where
         X₃₇ is Y or T;
      (b) an HCDR2 sequence having a formula of:
         X₃₈INPSX₃₉GX₄₀TX₄₁YNX₄₂KFX₄₃X₄₄ (SEQ ID NO. 253), where
         X₃₈ is G or Y, X₃₉ is N or S, X₄₀ is G or Y, X₄₁ is K or N, X₄₂ is Q or E, X₄₃ is K or Q, and X₄₄ is S, D or G; and
      (c) an HCDR3 sequence having a formula of:
         X₄₅X₄₆X₄₇X₄₈X₄₉X₅₀X₅₁YX₅₂X₅₃X₅₄X₅₅DY (SEQ **ID NO.** 254), where
         X₄₅ is G or W, X₄₆ is G or S, X₄₇ is G or L, X₄₈ is R or N, X₄₉ is E or S, X₅₀ is Y or R, X₅₁ is Y or L, X₅₂ is A or Y, X₅₃ is Y or M, X₅₄ is A or absent, and X₅₅ is M or absent; and
   (ii) the light chain variable region comprises:
      (a) an LCDR1 sequence having a formula of:
         X₅₆ASX₅₇X₅₈X₅₉X₆₀X₆₁YX₆₂X₆₃ (SEQ ID NO. 255), where
         X₅₆ is R, Q or S, X₅₇ is Q or S, X₅₈ is D or S, X₅₉ is I or V, X₆₀ is S or absent, X₆₁ is N or S, X₆₂ is L or M, and X₆₃ is N or Y;
      (b) an LCDR2 sequence having a formula of:
         X₆₄TSX₆₅LX₆₆S (SEQ ID NO. 256), where
         X₆₄ is Y or G, X₆₅ is R or N, and X₆₆ is H or A; and
      (c) an LCDR3 sequence having a formula of:
         QQX₆₇X₆₈SX₆₉PX₇₀T (SEQ ID NO.257), where
         X₆₇ is R or G, X₆₈ is Y or S, X₆₉ is L or Y, and X₇₀ is Y or L; or
(5) a heavy chain variable region and a light chain variable region, where
   (i) the heavy chain variable region comprises:
      (a) an HCDR1 sequence having a formula of:
         TX₇₁YVMX₇₂ (SEQ ID NO. 258), where
         X₇₁ is S or N, and X₇₂ is Y or H;
      (b) an HCDR2 sequence having a formula of:
         YINPYNDGTKYNEX₇₃FX₇₄G (SEQ ID NO. 259), where
         X₇₃ is K or R, and X₇₄ is K, T or Q; and
      (c) an HCDR3 sequence having a formula of:
         GGX₇₅X₇₆X₇₇X₇₈X₇₉X₈₀X₈₁X₈₂X₈₃Y (SEQ ID NO. 260), where
         X₇₅ is M or V, X₇₆ is L or R, X₇₇ is T or R, X₇₈ is T or Y, X₇₉ is R or F, X₈₀ is S or absent, X₈₁ is L or absent, X₈₂ is F or absent, and X₈₃ is A, T, V, L or D; and
   (ii) the light chain variable region comprises:
      (a) an LCDR1 sequence having a formula of:
         RASQX₈₄IX₈₅X₈₆X₈₇X₈₈X₈₉ (SEQ ID NO. 261), where
         X₈₄ is R, E or D, X₈₅ is G or S, X₈₆ is G or S, X₈₇ is Y or N, X₈₈ is L or F, and X₈₉ is N or S;
      (b) an LCDR2 sequence having a formula of:
         AX₉₀SX₉₁LDS (SEQ ID NO. 262), where
         X₉₀ is A or T, and X₉₁ is T or S; and
      (c) an LCDR3 sequence having a formula of:
         LQYAX₉₂X₉₃PX₉₄T (SEQ ID NO.263), where
         X₉₂ is S or N, X₉₃ is Y or F, and X₉₄ is P or Y; or
(6) a heavy chain variable region and a light chain variable region, where
   (i) the heavy chain variable region comprises:
      (a) an HCDR1 sequence having a formula of:
         SSYGMS (SEQ ID NO. 58);
      (b) an HCDR2 sequence having a formula of:
         TISSGGSYTYYPDSVKG (SEQ ID NO. 59); and
      (c) an HCDR3 sequence having a formula of:
         HX₉₅X₉₆X₉₇X₉₈X₉₉X₁₀₀X₁₀₀X₁₀₂DY (SEQ ID NO. 264), where
         X₉₅ is G or E, X₉₆ is G or A, X₉₇ is S or R, X₉₈ is S or A, X₉₉ is S or T, X₁₀₀ is Y or F, X₁₀₁ is V or absent, and X₁₀₂ is M or absent; and
   (ii) the light chain variable region comprises:
      (a) an LCDR1 sequence having a formula of:
         RSSKSLLHSNGNTYLY (SEQ ID NO. 63);
      (b) an LCDR2 sequence having a formula of:
         RMSNLAS (SEQ ID NO. 64); and
      (c) an LCDR3 sequence having a formula of:
         MQHLEYPFT (SEQ ID NO. 65); or
(7) a heavy chain variable region and a light chain variable region, where
   (i) the heavy chain variable region comprises:
      (a) an HCDR1 sequence having a formula of:
         TRYWMH (SEQ ID NO. 15);
      (b) an HCDR2 sequence having a formula of:
         EFNPSNGRINYNEKFX₁₀₃X₁₀₄ (SEQ ID NO. 265), where
         X₁₀₃ is K or Q, and X₁₀₄ is N or G; and
      (c) an HCDR3 sequence having a formula of:
         GFAY (SEQ ID NO. 17); and
   (ii) the light chain variable region comprises:
      (a) an LCDR1 sequence having a formula of:
         X₁₀₅ASSTVSYIH (SEQ ID NO. 266), where
         X₁₀₅ is S or R; and
      (b) an LCDR2 sequence having a formula of:
         DTSKLAS (SEQ ID NO. 21); and
      (c) an LCDR3 sequence having a formula of:
         QQWTTNPWT (SEQ ID NO. 22).

Additionally, the present disclosure provides an antibody binding GPRC5D or an antigen binding fragment thereof, which comprises a heavy chain CDR1 selected from an amino acid sequence as shown in SEQ ID NO. 5, 15, 25, 42, 58, 68, 98, 108, 119, or 241, or any variant thereof, a heavy chain CDR2 selected from an amino acid sequence as shown in SEQ ID NO. 6, 16, 26, 35, 43, 52, 59, 69, 78, 89, 99, 115, 133, or 147, or any variant thereof, and a heavy chain CDR3 selected from an amino acid sequence as shown in SEQ ID NO. 7, 17, 27, 44, 60, 70, 79, 90, 100, 109, 116, 120, 134, 139, or 148, or any variant thereof; and a light chain CDR1 selected from an amino acid sequence as shown in SEQ ID NO. 10, 20, 30, 38, 47, 63, 73, 82, 93, 103, 112, 123, 130, 167, 173, 179, 190, 208, or any variant thereof, a light chain CDR2 selected from an amino acid sequence as shown in SEQ ID NO. 11, 21, 31, 48, 64, 74, 83, 94, 104, 124, 151, or 209, or any variant thereof, and a light chain CDR3 selected from an amino acid sequence as shown in SEQ ID NO. 12, 22, 32, 39, 49, 55, 65, 75, 84, 95, 105, 125, or 144, or any variant thereof.

Additionally, the present disclosure provides an antibody binding GPRC5D or an antigen binding fragment thereof, which comprises a combination of heavy chain and light chain CDRs selected from:
(1) comprising heavy chain CDR1, CDR2, and CDR3 sequences having SEQ ID NO. 5, 6, and 7 respectively, and comprising light chain CDR1, CDR2, and CDR3 sequences having SEQ ID NO. 10, 11, and 12 respectively;
(2) comprising heavy chain CDR1, CDR2, and CDR3 sequences having SEQ ID NO. 15, 16, and 17 respectively, and comprising light chain CDR1, CDR2, and CDR3 sequences having SEQ ID NO. 20, 21, and 22 respectively;
(3) comprising heavy chain CDR1, CDR2, and CDR3 sequences having SEQ ID NO. 25, 26, and 27 respectively, and comprising light chain CDR1, CDR2, and CDR3 sequences having SEQ ID NO. 30, 31, and 32 respectively;
(4) comprising heavy chain CDR1, CDR2, and CDR3 sequences having SEQ ID NO. 5, 35, and 7 respectively, and comprising light chain CDR1, CDR2, and CDR3 sequences having SEQ ID NO. 38, 21, and 39 respectively;
(5) comprising heavy chain CDR1, CDR2, and CDR3 sequences having SEQ ID NO. 42, 43, and 44 respectively, and comprising light chain CDR1, CDR2, and CDR3 sequences having SEQ ID NO. 47, 48, and 49 respectively;
(6) comprising heavy chain CDR1, CDR2, and CDR3 sequences having SEQ ID NO. 5, 52, and 7 respectively, and comprising light chain CDR1, CDR2, and CDR3 sequences having SEQ ID NO. 38, 21, and 55 respectively;
(7) comprising heavy chain CDR1, CDR2, and CDR3 sequences having SEQ ID NO. 58, 59, and 60 respectively, and comprising light chain CDR1, CDR2, and CDR3 sequences having SEQ ID NO. 63, 64, and 65 respectively;
(8) comprising heavy chain CDR1, CDR2, and CDR3 sequences having SEQ ID NO. 68, 69, and 70 respectively, and comprising light chain CDR1, CDR2, and CDR3 sequences having SEQ ID NO. 73, 74, and 75 respectively;
(9) comprising heavy chain CDR1, CDR2, and CDR3 sequences having SEQ ID NO. 241, 78, and 79 respectively, and comprising light chain CDR1, CDR2, and CDR3 sequences having SEQ ID NO. 82, 83, and 84 respectively;
(10) comprising heavy chain CDR1, CDR2, and CDR3 sequences having SEQ ID NO. 42, 43, and 44 respectively, and comprising light chain CDR1, CDR2, and CDR3 sequences having SEQ ID NO. 47, 48, and 49 respectively;
(11) comprising heavy chain CDR1, CDR2, and CDR3 sequences having SEQ ID NO. 25, 89, and 90 respectively, and comprising light chain CDR1, CDR2, and CDR3 sequences having SEQ ID NO. 93, 94, and 95 respectively;
(12) comprising heavy chain CDR1, CDR2, and CDR3 sequences having SEQ ID NO. 98, 99, and 100 respectively, and comprising light chain CDR1, CDR2, and CDR3 sequences having SEQ ID NO. 103, 104, and 105 respectively;
(13) comprising heavy chain CDR1, CDR2, and CDR3 sequences having SEQ ID NO. 108, 99, and 109 respectively, and comprising light chain CDR1, CDR2, and CDR3 sequences having SEQ ID NO. 112, 104, and 105 respectively;
(14) comprising heavy chain CDR1, CDR2, and CDR3 sequences having SEQ ID NO. 98, 115, and 116 respectively, and comprising light chain CDR1, CDR2, and CDR3 sequences having SEQ ID NO. 112, 104, and 105 respectively;
(15) comprising heavy chain CDR1, CDR2, and CDR3 sequences having SEQ ID NO. 119, 99, and 120 respectively, and comprising light chain CDR1, CDR2, and CDR3 sequences having SEQ ID NO. 123, 124, and 125 respectively;
(16) comprising heavy chain CDR1, CDR2, and CDR3 sequences having SEQ ID NO. 108, 99, and 116 respectively, and comprising light chain CDR1, CDR2, and CDR3 sequences having SEQ ID NO. 130, 104, and 105 respectively;
(17) comprising heavy chain CDR1, CDR2, and CDR3 sequences having SEQ ID NO. 108, 133, and 134 respectively, and comprising light chain CDR1, CDR2, and CDR3 sequences having SEQ ID NO. 112, 104, and 105 respectively;
(18) comprising heavy chain CDR1, CDR2, and CDR3 sequences having SEQ ID NO. 58, 59, and 139 respectively, and comprising light chain CDR1, CDR2, and CDR3 sequences having SEQ ID NO. 63, 64, and 65 respectively;
(19) comprising heavy chain CDR1, CDR2, and CDR3 sequences having SEQ ID NO. 98, 99, and 109 respectively, and comprising light chain CDR1, CDR2, and CDR3 sequences having SEQ ID NO. 103, 104, and 105 respectively;
(20) comprising heavy chain CDR1, CDR2, and CDR3 sequences having SEQ ID NO. 98, 99, and 109 respectively, and comprising light chain CDR1, CDR2, and CDR3 sequences having SEQ ID NO. 103, 104, and 144 respectively;
(21) comprising heavy chain CDR1, CDR2, and CDR3 sequences having SEQ ID NO. 25, 147, and 148 respectively, and comprising light chain CDR1, CDR2, and CDR3 sequences having SEQ ID NO. 93, 151, and 95 respectively;
(22) comprising heavy chain CDR1, CDR2, and CDR3 sequences having SEQ ID NO. 15, 164, and 17 respectively, and comprising light chain CDR1, CDR2, and CDR3 sequences having SEQ ID NO. 167, 21, and 22 respectively;
(23) comprising heavy chain CDR1, CDR2, and CDR3 sequences having SEQ ID NO. 25, 170, and 27 respectively, and comprising light chain CDR1, CDR2, and CDR3 sequences having SEQ ID NO. 173, 31, and 32 respectively;
(24) comprising heavy chain CDR1, CDR2, and CDR3 sequences having SEQ ID NO. 5, 176, and 7 respectively, and comprising light chain CDR1, CDR2, and CDR3 sequences having SEQ ID NO. 179, 21, and 39 respectively;
(25) comprising heavy chain CDR1, CDR2, and CDR3 sequences having SEQ ID NO. 42, 182, and 44 respectively, and comprising light chain CDR1, CDR2, and CDR3 sequences having SEQ ID NO. 47, 48, and 49 respectively;
(26) comprising heavy chain CDR1, CDR2, and CDR3 sequences having SEQ ID NO. 68, 187, and 70 respectively, and comprising light chain CDR1, CDR2, and CDR3 sequences having SEQ ID NO. 190, 74, and 75 respectively;
(27) comprising heavy chain CDR1, CDR2, and CDR3 sequences having SEQ ID NO. 119, 193, and 120 respectively, and comprising light chain CDR1, CDR2, and CDR3 sequences having SEQ ID NO. 123, 124, and 125 respectively;
(28) comprising heavy chain CDR1, CDR2, and CDR3 sequences having SEQ ID NO. 25, 170, and 27 respectively, and comprising light chain CDR1, CDR2, and CDR3 sequences having SEQ ID NO. 208, 209, and 32 respectively;
(29) comprising heavy chain CDR1, CDR2, and CDR3 sequences having SEQ ID NO. 25, 170, and 27 respectively, and comprising light chain CDR1, CDR2, and CDR3 sequences having SEQ ID NO. 173, 209, and 32 respectively;
(30) comprising heavy chain CDR1, CDR2, and CDR3 sequences having SEQ ID NO. 25, 170, and 27 respectively, and comprising light chain CDR1, CDR2, and CDR3 sequences having SEQ ID NO. 208, 31, and 32 respectively;

Additionally, the present disclosure provides an antibody binding GPRC5D or an antigen binding fragment thereof, which comprises a heavy chain variable region selected from an amino acid sequence as shown in SEQ ID NO. 3, 13, 23, 33, 40, 50, 56, 66, 76, 87, 96, 106, 113, 117, 126, 131, 140, 145, 162, 168, 174, 180, 185, 191, 202, 232, or any variant thereof, and a light chain variable region selected from an amino acid sequence as shown in SEQ ID NO. 8, 18, 28, 36, 45, 53, 61, 71, 80, 85, 91, 101, 110, 121, 128, 135, 142, 149, 165, 171, 177, 183, 188, 194, 206, 212, 216, 220, 230, or any variant thereof.

In a preferred embodiment, the antibody or antigen binding fragment thereof comprises a heavy chain variable region having an amino acid sequence as shown in SEQ ID NO. 3 or any variant thereof, and a light chain variable region having an amino acid sequence as shown in SEQ ID NO. 8 or any variant thereof.

In a preferred embodiment, the antibody or antigen binding fragment thereof comprises a heavy chain variable region having an amino acid sequence as shown in SEQ ID NO. 13 or any variant thereof, and a light chain variable region having an amino acid sequence as shown in SEQ ID NO. 18 or any variant thereof.

In a preferred embodiment, the antibody or antigen binding fragment thereof comprises a heavy chain variable region having an amino acid sequence as shown in SEQ ID NO. 23 or any variant thereof, and a light chain variable region having an amino acid sequence as shown in SEQ ID NO. 28 or any variant thereof.

In a preferred embodiment, the antibody or antigen binding fragment thereof comprises a heavy chain variable region having an amino acid sequence as shown in SEQ ID NO. 33 or any variant thereof, and a light chain variable region having an amino acid sequence as shown in SEQ ID NO. 36 or any variant thereof.

In a preferred embodiment, the antibody or antigen binding fragment thereof comprises a heavy chain variable region having an amino acid sequence as shown in SEQ ID NO. 40 or any variant thereof, and a light chain variable region having an amino acid sequence as shown in SEQ ID NO. 45 or any variant thereof.

In a preferred embodiment, the antibody or antigen binding fragment thereof comprises a heavy chain variable region having an amino acid sequence as shown in SEQ ID NO. 50 or any variant thereof, and a light chain variable region having an amino acid sequence as shown in SEQ ID NO. 53 or any variant thereof.

In a preferred embodiment, the antibody or antigen binding fragment thereof comprises a heavy chain variable region having an amino acid sequence as shown in SEQ ID NO. 56 or any variant thereof, and a light chain variable region having an amino acid sequence as shown in SEQ ID NO. 61 or any variant thereof.

In a preferred embodiment, the antibody or antigen binding fragment thereof comprises a heavy chain variable region having an amino acid sequence as shown in SEQ ID NO. 66 or any variant thereof, and a light chain variable region having an amino acid sequence as shown in SEQ ID NO. 71 or any variant thereof.

In a preferred embodiment, the antibody or antigen binding fragment thereof comprises a heavy chain variable region having an amino acid sequence as shown in SEQ ID NO. 76 or any variant thereof, and a light chain variable region having an amino acid sequence as shown in SEQ ID NO. 80 or any variant thereof.

In a preferred embodiment, the antibody or antigen binding fragment thereof comprises a heavy chain variable region having an amino acid sequence as shown in SEQ ID NO. 40 or any variant thereof, and a light chain variable region having an amino acid sequence as shown in SEQ ID NO. 85 or any variant thereof.

In a preferred embodiment, the antibody or antigen binding fragment thereof comprises a heavy chain variable region having an amino acid sequence as shown in SEQ ID NO. 87 or any variant thereof, and a light chain variable region having an amino acid sequence as shown in SEQ ID NO. 91 or any variant thereof.

In a preferred embodiment, the antibody or antigen binding fragment thereof comprises a heavy chain variable region having an amino acid sequence as shown in SEQ ID NO. 96 or any variant thereof, and a light chain variable region having an amino acid sequence as shown in SEQ ID NO. 101 or any variant thereof.

In a preferred embodiment, the antibody or antigen binding fragment thereof comprises a heavy chain variable region having an amino acid sequence as shown in SEQ ID NO. 106 or any variant thereof, and a light chain variable region having an amino acid sequence as shown in SEQ ID NO. 110 or any variant thereof.

In a preferred embodiment, the antibody or antigen binding fragment thereof comprises a heavy chain variable region having an amino acid sequence as shown in SEQ ID NO. 113 or any variant thereof, and a light chain variable region having an amino acid sequence as shown in SEQ ID NO. 110 or any variant thereof.

In a preferred embodiment, the antibody or antigen binding fragment thereof comprises a heavy chain variable region having an amino acid sequence as shown in SEQ ID NO. 117 or any variant thereof, and a light chain variable region having an amino acid sequence as shown in SEQ ID NO. 121 or any variant thereof.

In a preferred embodiment, the antibody or antigen binding fragment thereof comprises a heavy chain variable region having an amino acid sequence as shown in SEQ ID NO. 126 or any variant thereof, and a light chain variable region having an amino acid sequence as shown in SEQ ID NO. 128 or any variant thereof.

In a preferred embodiment, the antibody or antigen binding fragment thereof comprises a heavy chain variable region having an amino acid sequence as shown in SEQ ID NO. 131 or any variant thereof, and a light chain variable region having an amino acid sequence as shown in SEQ ID NO. 135 or any variant thereof.

In a preferred embodiment, the antibody or antigen binding fragment thereof comprises a heavy chain variable region having an amino acid sequence as shown in SEQ ID NO. 137 or any variant thereof, and a light chain variable region having an amino acid sequence as shown in SEQ ID NO. 61 or any variant thereof.

In a preferred embodiment, the antibody or antigen binding fragment thereof comprises a heavy chain variable region having an amino acid sequence as shown in SEQ ID NO. 140 or any variant thereof, and a light chain variable region having an amino acid sequence as shown in SEQ ID NO. 101 or any variant thereof.

In a preferred embodiment, the antibody or antigen binding fragment thereof comprises a heavy chain variable region having an amino acid sequence as shown in SEQ ID NO. 140 or any variant thereof, and a light chain variable region having an amino acid sequence as shown in SEQ ID NO. 142 or any variant thereof.

In a preferred embodiment, the antibody or antigen binding fragment thereof comprises a heavy chain variable region having an amino acid sequence as shown in SEQ ID NO. 145 or any variant thereof, and a light chain variable region having an amino acid sequence as shown in SEQ ID NO. 149 or any variant thereof.

In a preferred embodiment, the antibody or antigen binding fragment thereof comprises a heavy chain variable region having an amino acid sequence as shown in SEQ ID NO. 162 or any variant thereof, and a light chain variable region having an amino acid sequence as shown in SEQ ID NO. 165 or any variant thereof.

In a preferred embodiment, the antibody or antigen binding fragment thereof comprises a heavy chain variable region having an amino acid sequence as shown in SEQ ID NO. 168 or any variant thereof, and a light chain variable region having an amino acid sequence as shown in SEQ ID NO. 171 or any variant thereof.

In a preferred embodiment, the antibody or antigen binding fragment thereof comprises a heavy chain variable region having an amino acid sequence as shown in SEQ ID NO. 174 or any variant thereof, and a light chain variable region having an amino acid sequence as shown in SEQ ID NO. 177 or any variant thereof.

In a preferred embodiment, the antibody or antigen binding fragment thereof comprises a heavy chain variable region having an amino acid sequence as shown in SEQ ID NO. 180 or any variant thereof, and a light chain variable region having an amino acid sequence as shown in SEQ ID NO. 183 or any variant thereof.

In a preferred embodiment, the antibody or antigen binding fragment thereof comprises a heavy chain variable region having an amino acid sequence as shown in SEQ ID NO. 185 or any variant thereof, and a light chain variable region having an amino acid sequence as shown in SEQ ID NO. 188 or any variant thereof.

In a preferred embodiment, the antibody or antigen binding fragment thereof comprises a heavy chain variable region having an amino acid sequence as shown in SEQ ID NO. 191 or any variant thereof, and a light chain variable region having an amino acid sequence as shown in SEQ ID NO. 194 or any variant thereof.

In a preferred embodiment, the antibody or antigen binding fragment thereof comprises a heavy chain variable region having an amino acid sequence as shown in SEQ ID NO. 202 or any variant thereof, and a light chain variable region having an amino acid sequence as shown in SEQ ID NO. 206 or any variant thereof.

In a preferred embodiment, the antibody or antigen binding fragment thereof comprises a heavy chain variable region having an amino acid sequence as shown in SEQ ID NO. 202 or any variant thereof, and a light chain variable region having an amino acid sequence as shown in SEQ ID NO. 212 or any variant thereof.

In a preferred embodiment, the antibody or antigen binding fragment thereof comprises a heavy chain variable region having an amino acid sequence as shown in SEQ ID NO. 202 or any variant thereof, and a light chain variable region having an amino acid sequence as shown in SEQ ID NO. 216 or any variant thereof.

In a preferred embodiment, the antibody or antigen binding fragment thereof comprises a heavy chain variable region having an amino acid sequence as shown in SEQ ID NO. 202 or any variant thereof, and a light chain variable region having an amino acid sequence as shown in SEQ ID NO. 220 or any variant thereof.

In a preferred embodiment, the antibody or antigen binding fragment thereof comprises a heavy chain variable region having an amino acid sequence as shown in SEQ ID NO. 232 or any variant thereof, and a light chain variable region having an amino acid sequence as shown in SEQ ID NO. 230 or any variant thereof.

In a preferred embodiment, the antibody or antigen binding fragment thereof is humanized.

Additionally, the present disclosure provides a nucleic acid encoding the antibody or antigen binding fragment thereof.

In a preferred embodiment, the nucleic acid comprises a nucleic acid sequence selected from SEQ ID NO. 4, 14, 24, 34, 41, 51, 57, 67, 77, 88, 97, 107, 114, 118, 127, 132, 138, 141, 146, 163, 169, 175, 181, 186, 192, or 203 or any variant thereof, which encodes a heavy chain variable region of the antibody; and a nucleic acid sequence selected from SEQ ID NO. 9, 19, 29, 37, 46, 54, 62, 72, 81, 86, 92, 102, 111, 122, 129, 136, 143, 150, 166, 172, 178, 184, 189, 195, 207, 213, 217, 221 or any variant thereof, which encodes a light chain variable region of the antibody.

In a further preferred embodiment, the nucleic acid comprises a nucleotide sequence as shown in SEQ ID NO. 4 or any variant thereof, which encodes the heavy chain variable region, and a nucleotide sequence as shown in SEQ ID NO. 9 or any variant thereof, which encodes the light chain variable region.

In a further preferred embodiment, the nucleic acid comprises a nucleotide sequence as shown in SEQ ID NO. 14 or any variant thereof, which encodes the heavy chain variable region, and a nucleotide sequence as shown in SEQ ID NO. 19 or any variant thereof, which encodes the light chain variable region.

In a further preferred embodiment, the nucleic acid comprises a nucleotide sequence as shown in SEQ ID NO. 24 or any variant thereof, which encodes the heavy chain variable region, and a nucleotide sequence as shown in SEQ ID NO. 29 or any variant thereof, which encodes the light chain variable region.

In a further preferred embodiment, the nucleic acid comprises a nucleotide sequence as shown in SEQ ID NO. 34 or any variant thereof, which encodes the heavy chain variable region, and a nucleotide sequence as shown in SEQ ID NO. 37 or any variant thereof, which encodes the light chain variable region.

In a further preferred embodiment, the nucleic acid comprises a nucleotide sequence as shown in SEQ ID NO. 41 or any variant thereof, which encodes the heavy chain variable region, and a nucleotide sequence as shown in SEQ ID NO. 46 or any variant thereof, which encodes the light chain variable region.

In a further preferred embodiment, the nucleic acid comprises a nucleotide sequence as shown in SEQ ID NO. 51 or any variant thereof, which encodes the heavy chain variable region, and a nucleotide sequence as shown in SEQ ID NO. 54 or any variant thereof, which encodes the light chain variable region.

In a further preferred embodiment, the nucleic acid comprises a nucleotide sequence as shown in SEQ ID NO. 57 or any variant thereof, which encodes the heavy chain variable region, and a nucleotide sequence as shown in SEQ ID NO. 62 or any variant thereof, which encodes the light chain variable region.

In a further preferred embodiment, the nucleic acid comprises a nucleotide sequence as shown in SEQ ID NO. 67 or any variant thereof, which encodes the heavy chain variable region, and a nucleotide sequence as shown in SEQ ID NO. 72 or any variant thereof, which encodes the light chain variable region.

In a further preferred embodiment, the nucleic acid comprises a nucleotide sequence as shown in SEQ ID NO. 77 or any variant thereof, which encodes the heavy chain variable region, and a nucleotide sequence as shown in SEQ ID NO. 81 or any variant thereof, which encodes the light chain variable region.

In a further preferred embodiment, the nucleic acid comprises a nucleotide sequence as shown in SEQ ID NO. 41 or any variant thereof, which encodes the heavy chain variable region, and a nucleotide sequence as shown in SEQ ID NO. 86 or any variant thereof, which encodes the light chain variable region.

In a further preferred embodiment, the nucleic acid comprises a nucleotide sequence as shown in SEQ ID NO. 88 or any variant thereof, which encodes the heavy chain variable region, and a nucleotide sequence as shown in SEQ ID NO. 92 or any variant thereof, which encodes the light chain variable region.

In a further preferred embodiment, the nucleic acid comprises a nucleotide sequence as shown in SEQ ID NO. 97 or any variant thereof, which encodes the heavy chain variable region, and a nucleotide sequence as shown in SEQ ID NO. 102 or any variant thereof, which encodes the light chain variable region.

In a further preferred embodiment, the nucleic acid comprises a nucleotide sequence as shown in SEQ ID NO. 107 or any variant thereof, which encodes the heavy chain variable region, and a nucleotide sequence as shown in SEQ ID NO. 111 or any variant thereof, which encodes the light chain variable region.

In a further preferred embodiment, the nucleic acid comprises a nucleotide sequence as shown in SEQ ID NO. 114 or any variant thereof, which encodes the heavy chain variable region, and a nucleotide sequence as shown in SEQ ID NO. 111 or any variant thereof, which encodes the light chain variable region.

In a further preferred embodiment, the nucleic acid comprises a nucleotide sequence as shown in SEQ ID NO. 118 or any variant thereof, which encodes the heavy chain variable region, and a nucleotide sequence as shown in SEQ ID NO. 122 or any variant thereof, which encodes the light chain variable region.

In a further preferred embodiment, the nucleic acid comprises a nucleotide sequence as shown in SEQ ID NO. 127 or any variant thereof, which encodes the heavy chain variable region, and a nucleotide sequence as shown in SEQ ID NO. 129 or any variant thereof, which encodes the light chain variable region.

In a further preferred embodiment, the nucleic acid comprises a nucleotide sequence as shown in SEQ ID NO. 132 or any variant thereof, which encodes the heavy chain variable region, and a nucleotide sequence as shown in SEQ ID NO. 136 or any variant thereof, which encodes the light chain variable region.

In a further preferred embodiment, the nucleic acid comprises a nucleotide sequence as shown in SEQ ID NO. 138 or any variant thereof, which encodes the heavy chain variable region, and a nucleotide sequence as shown in SEQ ID NO. 62 or any variant thereof, which encodes the light chain variable region.

In a further preferred embodiment, the nucleic acid comprises a nucleotide sequence as shown in SEQ ID NO. 141 or any variant thereof, which encodes the heavy chain variable region, and a nucleotide sequence as shown in SEQ ID NO. 102 or any variant thereof, which encodes the light chain variable region.

In a further preferred embodiment, the nucleic acid comprises a nucleotide sequence as shown in SEQ ID NO. 141 or any variant thereof, which encodes the heavy chain variable region, and a nucleotide sequence as shown in SEQ ID NO. 143 or any variant thereof, which encodes the light chain variable region.

In a further preferred embodiment, the nucleic acid comprises a nucleotide sequence as shown in SEQ ID NO. 146 or any variant thereof, which encodes the heavy chain variable region, and a nucleotide sequence as shown in SEQ ID NO. 150 or any variant thereof, which encodes the light chain variable region.

In a further preferred embodiment, the nucleic acid comprises a nucleotide sequence as shown in SEQ ID NO. 163 or any variant thereof, which encodes the heavy chain variable region, and a nucleotide sequence as shown in SEQ ID NO. 166 or any variant thereof, which encodes the light chain variable region.

In a further preferred embodiment, the nucleic acid comprises a nucleotide sequence as shown in SEQ ID NO. 169 or any variant thereof, which encodes the heavy chain variable region, and a nucleotide sequence as shown in SEQ ID NO. 172 or any variant thereof, which encodes the light chain variable region.

In a further preferred embodiment, the nucleic acid comprises a nucleotide sequence as shown in SEQ ID NO. 175 or any variant thereof, which encodes the heavy chain variable region, and a nucleotide sequence as shown in SEQ ID NO. 178 or any variant thereof, which encodes the light chain variable region.

In a further preferred embodiment, the nucleic acid comprises a nucleotide sequence as shown in SEQ ID NO. 181 or any variant thereof, which encodes the heavy chain variable region, and a nucleotide sequence as shown in SEQ ID NO. 184 or any variant thereof, which encodes the light chain variable region.

In a further preferred embodiment, the nucleic acid comprises a nucleotide sequence as shown in SEQ ID NO. 186 or any variant thereof, which encodes the heavy chain variable region, and a nucleotide sequence as shown in SEQ ID NO. 189 or any variant thereof, which encodes the light chain variable region.

In a further preferred embodiment, the nucleic acid comprises a nucleotide sequence as shown in SEQ ID NO. 192 or any variant thereof, which encodes the heavy chain variable region, and a nucleotide sequence as shown in SEQ ID NO. 195 or any variant thereof, which encodes the light chain variable region.

In a further preferred embodiment, the nucleic acid comprises a nucleotide sequence as shown in SEQ ID NO. 203 or any variant thereof, which encodes the heavy chain variable region, and a nucleotide sequence as shown in SEQ ID NO. 207 or any variant thereof, which encodes the light chain variable region.

In a further preferred embodiment, the nucleic acid comprises a nucleotide sequence as shown in SEQ ID NO. 203 or any variant thereof, which encodes the heavy chain variable region, and a nucleotide sequence as shown in SEQ ID NO. 213 or any variant thereof, which encodes the light chain variable region.

In a further preferred embodiment, the nucleic acid comprises a nucleotide sequence as shown in SEQ ID NO. 203 or any variant thereof, which encodes the heavy chain variable region, and a nucleotide sequence as shown in SEQ ID NO. 217 or any variant thereof, which encodes the light chain variable region.

In a further preferred embodiment, the nucleic acid comprises a nucleotide sequence as shown in SEQ ID NO. 203 or any variant thereof, which encodes the heavy chain variable region, and a nucleotide sequence as shown in SEQ ID NO. 221 or any variant thereof, which encodes the light chain variable region.

Additionally, the present disclosure provides a multispecific antibody or an antigen binding fragment thereof, which includes at least:
(a) an antibody binding GPRC5D antigen or an antigen binding fragment thereof according to any one of claims 1 to 4, which is a first antigen binding portion, where the GPRC5D antigen is a first antigen, the first antigen binding portion includes a first heavy chain variable region and a first light chain variable region, and the first antigen binding portion includes a first binding domain binding the first antigen.
   In a preferred embodiment, the first binding domain comprises a first heavy chain CDR selected from an amino acid sequence as shown in SEQ ID NO. 25, 170, or 27, or any variant thereof, and a first light chain CDR selected from an amino acid sequence as shown in SEQ ID NO. 31, 32, 173, 208, or 209, or any variant thereof; and
(b) an antibody binding CD3 antigen or an antigen binding fragment thereof, which is a second antigen binding portion, where the CD3 antigen is a second antigen, the second antigen binding portion includes a second heavy chain variable region and a second light chain variable region, and the second antigen binding portion includes a second binding domain binding the second antigen. The second binding domain comprises a second heavy chain CDR1 selected from an amino acid sequence as shown in SEQ ID NO. 159 or any variant thereof, a second heavy chain CDR2 selected from an amino acid sequence as shown in SEQ ID NO. 160 or any variant thereof, and a second heavy chain CDR3 selected from an amino acid sequence as shown in SEQ ID NO. 161 or any variant thereof; and a second light chain CDR1 selected from an amino acid sequence as shown in SEQ ID NO. 154 or any variant thereof, a second light chain CDR2 selected from an amino acid sequence as shown in SEQ ID NO. 155 or any variant thereof, and a second light chain CDR3 selected fromg an amino acid sequence as shown in SEQ ID NO. 156 or any variant thereof.

In a preferred embodiment, the second binding domain comprises a second heavy chain variable region selected from an amino acid sequence as shown in SEQ ID NO. 157 or any variant thereof; and a second light chain variable region selected from an amino acid sequence as shown in SEQ ID NO. 152 or any variant thereof.

In a preferred embodiment, the second heavy chain of the second antigen binding portion comprises an amino acid sequence as shown in SEQ ID NO. 222, and a second light chain of the second antigen binding portion comprises an amino acid sequence as shown in SEQ ID NO. 224.

In a preferred embodiment, the first binding domain comprises a first heavy chain CDR1 selected from an amino acid sequence as shown in SEQ ID NO. 25 or any variant thereof, a first heavy chain CDR2 selected from an amino acid sequence as shown in SEQ ID NO. 170 or any variant thereof, and a first heavy chain CDR3 selected from an amino acid sequence as shown in SEQ ID NO. 27 or any variant thereof; and a first light chain CDR1 selected from an amino acid sequence as shown in SEQ ID NO. 173, 208 or any variant thereof, a first light chain CDR2 selected from an amino acid sequence as shown in SEQ ID NO. 31, 209 or any variant thereof, and a first light chain CDR3 selected from an amino acid sequence as shown in SEQ ID NO. 32 or any variant thereof.

In a preferred embodiment, the first light chain CDR of the first binding domain is selected from: first light chain CDR1, CDR2 and CDR3 sequences comprising an amino acid sequence as shown in SEQ ID NOs. 173, 31, and 32 respectively; first light chain CDR1, CDR2 and CDR3 sequences comprising an amino acid sequence as shown in SEQ ID NOs. 208, 209, and 32 respectively; first light chain CDR1, CDR2 and CDR3 sequences comprising an amino acid sequence as shown in SEQ ID NOs. 173, 209, and 32 respectively; or first light chain CDR1, CDR2 and CDR3 sequences comprising an amino acid sequence as shown in SEQ ID NOs. 208, 31, and 32 respectively; and the first heavy chain CDR of the first binding domain is selected from: first heavy chain CDR1, CDR2 and CDR3 sequences comprising an amino acid sequence as shown in SEQ ID NOs. 25, 170, and 27 respectively.

In a preferred embodiment, the first binding domain comprises a first heavy chain variable region selected from an amino acid sequence as shown in SEQ ID NO. 168, 202, or 232 or any variant thereof; and a first light chain variable region selected from an amino acid sequence as shown in SEQ ID NO. 171, 206, 212, 216, 220, or 230 or any variant thereof.

In a preferred embodiment, the first binding domain comprises a first heavy chain variable region selected from an amino acid sequence as shown in SEQ ID NO. 168 or any variant thereof; and a first light chain variable region selected from an amino acid sequence as shown in SEQ ID NO. 171 or any variant thereof.

In a preferred embodiment, the first binding domain comprises a first heavy chain variable region selected from an amino acid sequence as shown in SEQ ID NO. 202 or any variant thereof; and a first light chain variable region selected from an amino acid sequence as shown in SEQ ID NO. 206 or any variant thereof.

In a preferred embodiment, the first binding domain comprises a first heavy chain variable region selected from an amino acid sequence as shown in SEQ ID NO. 202 or any variant thereof; and a first light chain variable region selected from an amino acid sequence as shown in SEQ ID NO. 212 or any variant thereof.

In a preferred embodiment, the first binding domain comprises a first heavy chain variable region selected from an amino acid sequence as shown in SEQ ID NO. 202 or any variant thereof; and a first light chain variable region selected from an amino acid sequence as shown in SEQ ID NO. 216 or any variant thereof.

In a preferred embodiment, the first binding domain comprises a first heavy chain variable region selected from an amino acid sequence as shown in SEQ ID NO. 202 or any variant thereof; and a first light chain variable region selected from an amino acid sequence as shown in SEQ ID NO. 220 or any variant thereof.

In a preferred embodiment, the first binding domain comprises a first heavy chain variable region selected from an amino acid sequence as shown in SEQ ID NO. 232 or any variant thereof; and a first light chain variable region selected from an amino acid sequence as shown in SEQ ID NO. 230 or any variant thereof.

In a preferred embodiment, the first heavy chain of the first antigen binding portion is selected from an amino acid sequence as shown in SEQ ID NO. 196 or 200; the first light chain of the first antigen binding portion is selected from an amino acid sequence as shown in SEQ ID NO. 198, 204, 210, 214, or 218. Further preferably the first heavy chain of the first antigen binding portion comprises an amino acid sequence as shown in SEQ ID NO. 196; and the first light chain of the first antigen binding portion comprises an amino acid sequence as shown in SEQ ID NO. 198. Further preferably the first heavy chain of the first antigen binding portion comprises an amino acid sequence as shown in SEQ ID NO. 200; and the first light chain of the first antigen binding portion comprises an amino acid sequence as shown in SEQ ID NO. 204. Further preferably, the first heavy chain of the first antigen binding portion comprises an amino acid sequence as shown in SEQ ID NO. 200 and the first light chain of the first antigen binding portion comprises an amino acid sequence as shown in SEQ ID NO. 210. Further preferably the first heavy chain of the first antigen binding portion comprises an amino acid sequence as shown in SEQ ID NO. 200; and the first light chain of the first antigen binding portion comprises an amino acid sequence as shown in SEQ ID NO. 214. Further preferably the first heavy chain of the first antigen binding portion comprises an amino acid sequence as shown in SEQ ID NO. 200; and the first light chain of the first antigen binding portion comprises an amino acid sequence as shown in SEQ ID NO. 218.

In a further preferred embodiment, the multispecific antibody or antigen binding fragment thereof further comprises:
(c) an antibody binding BCMA antigen or an antigen binding fragment thereof, which is a third antigen binding portion, where the BCMA antigen is a third antigen, the third antigen binding portion includes a third heavy chain variable region and a third light chain variable region, and the third antigen binding portion includes a third binding domain binding the third antigen.

In a preferred embodiment, the third binding domain comprises a third heavy chain variable region selected from an amino acid sequence as shown in SEQ ID NO.268 or any variant thereof, and a third light chain variable region selected from an amino acid sequence as shown in SEQ ID NO. 267 or any variant thereof.

In a preferred embodiment, the third antigen binding portion comprises a third heavy chain having an amino acid sequence as shown in SEQ ID NO. 233 or any variant thereof, and a third light chain having an amino acid sequence as shown in SEQ ID NO. 226 or any variant thereof.

In a preferred embodiment, the multispecific antibody is a trispecific antibody. Any two of the first heavy chain variable region, the second heavy chain variable region, and the third heavy chain variable region are connected to a constant region, the remaining heavy chain variable region is not connected to the constant region, and the heavy chain variable region that is not connected to the constant region is connected to any one of the two heavy chain variable regions connected to the constant region, preferably, by a linker. Preferably, the heavy chain variable region that is not connected to the constant region is connected to the corresponding light chain variable region by a linker.

In a preferred embodiment, the first heavy chain variable region or first light chain variable region of the first antigen binding portion is connected to the second heavy chain variable region or second light chain variable region of the second antigen binding portion by a linker. Preferably, the first heavy chain variable region and the first light chain variable region of the first antigen binding portion are connected by a linker. Further preferably, the first light chain variable region of the first antigen binding portion is connected to the second heavy chain variable region of the second antigen binding portion.

In a preferred embodiment, the first heavy chain variable region or first light chain variable region of the first antigen binding portion is connected to the third heavy chain variable region or third light chain variable region of the third antigen binding portion by a linker. Preferably, the first heavy chain variable region and the first light chain variable region of the first antigen binding portion are connected by a linker.

In a preferred embodiment, the linker is a flexible linker.

In a preferred embodiment, the linker is (GGGGS)ₙ, where n=2-5, for example, GGGGSGGGGSGGGGS (SEQ ID NO: 231), GSTSGSGKSSEGKG (SEQ ID NO: 273), GSGGGSG (SEQ ID NO: 274), and GGGSGGSG (SEQ ID NO: 275).

In a further preferred embodiment, the trispecific antibody comprises a light chain 1, a heavy chain 1, a light chain 2, and a heavy chain 2.

In a further preferred embodiment, the light chain 1 comprises a third light chain variable region having an amino acid sequence as shown in SEQ ID NO. 267, the heavy chain 1 comprises a first light chain variable region having an amino acid sequence as shown in SEQ ID NO. 230, a first heavy chain variable region having an amino acid sequence as shown in SEQ ID NO. 232, and a third heavy chain variable region having an amino acid sequence as shown in SEQ ID NO. 268, the light chain 2 comprises a second light chain variable region having an amino acid sequence as shown in SEQ ID NO. 152, and the heavy chain 2 comprises a second heavy chain variable region having an amino acid sequence as shown in SEQ ID NO. 157. Further preferably the light chain 1 comprises an amino acid sequence as shown in SEQ ID NO. 226; the heavy chain 1 comprises an amino acid sequence as shown in SEQ ID NO. 228; the light chain 2 comprises an amino acid sequence as shown in SEQ ID NO. 224; and the heavy chain 2 comprises an amino acid sequence as shown in SEQ ID NO. 222.

In a further preferred embodiment, the light chain 1 comprises a third light chain variable region having an amino acid sequence as shown in SEQ ID NO. 267, the heavy chain 1 comprises a first heavy chain variable region having an amino acid sequence as shown in SEQ ID NO. 232, a first light chain variable region having an amino acid sequence as shown in SEQ ID NO. 230, and a third heavy chain variable region having an amino acid sequence as shown in SEQ ID NO. 268, the light chain 2 comprises a second light chain variable region having an amino acid sequence as shown in SEQ ID NO. 152, and the heavy chain 2 comprises a second heavy chain variable region having an amino acid sequence as shown in SEQ ID NO. 157. Further preferably the light chain 1 comprises an amino acid sequence as shown in SEQ ID NO. 226; the heavy chain 1 comprises an amino acid sequence as shown in SEQ ID NO. 234; the light chain 2 comprises an amino acid sequence as shown in SEQ ID NO. 224; and the heavy chain 2 comprises an amino acid sequence as shown in SEQ ID NO. 222.

In a further preferred embodiment, the light chain 1 comprises a second light chain variable region having an amino acid sequence as shown in SEQ ID NO. 152, the heavy chain 1 comprises a first light chain variable region having an amino acid sequence as shown in SEQ ID NO. 230, a first heavy chain variable region having an amino acid sequence as shown in SEQ ID NO. 232 and a second heavy chain variable region having amino acid sequence as shown in SEQ ID NO. 157, the light chain 2 comprises a third light chain variable region having an amino acid sequence as shown in SEQ ID NO. 267, and the heavy chain 2 comprises a third heavy chain variable region having an amino acid sequence as shown in SEQ ID NO. 268. Further preferably the light chain 1 comprises an amino acid sequence as shown in SEQ ID NO. 224; the heavy chain 1 comprises an amino acid sequence as shown in SEQ ID NO. 236; the light chain 2 comprises an amino acid sequence as shown in SEQ ID NO. 226; and the heavy chain 2 comprises an amino acid sequence as shown in SEQ ID NO. 233.

In a further preferred embodiment, the light chain 1 comprises a second light chain variable region having an amino acid sequence as shown in SEQ ID NO. 152, the heavy chain 1 comprises a first heavy chain variable region having an amino acid sequence as shown in SEQ ID NO. 232, a first light chain variable region having an amino acid sequence as shown in SEQ ID NO. 230, and a second heavy chain variable region having an amino acid sequence as shown in SEQ ID NO. 157, the light chain 2 comprises a third light chain variable region having an amino acid sequence as shown in SEQ ID NO. 267, and the heavy chain 2 comprises a third heavy chain variable region having an amino acid sequence as shown in SEQ ID NO. 268. Further preferably the light chain 1 comprises an amino acid sequence as shown in SEQ ID NO.224; the heavy chain 1 comprises an amino acid sequence as shown in SEQ ID NO. 239; the light chain 2 comprises an amino acid sequence as shown in SEQ ID NO. 226; and the heavy chain 2 comprises an amino acid sequence as shown in SEQ ID NO. 233.

Additionally, the present disclosure provides a nucleic acid encoding the multispecific antibody or antigen binding fragment thereof.

In a preferred embodiment, the multispecific antibody is a bispecific antibody. The nucleic acid encoding the first heavy chain variable region of the first antigen binding portion of the bispecific antibody selected from a nucleotide sequence as shown in SEQ ID NO. 169 or SEQ ID NO. 203, and the nucleic acid encoding the first light chain variable region of the first antigen binding portion selected from a nucleotide sequence as shown in SEQ ID NO. 172, SEQ ID NO. 207, SEQ ID NO. 213, SEQ ID NO. 217, or SEQ ID NO. 221. The nucleic acid encoding the second heavy chain variable region of the second antigen binding portion comprises a nucleotide sequence as shown in SEQ ID NO. 158, and the nucleic acid encoding the second light chain variable region of the second antigen binding portion comprises a nucleotide sequence as shown in SEQ ID NO. 153. Further preferably the nucleic acid encoding the first heavy chain of the first antigen binding portion of the bispecific antibody selected from a nucleotide sequence as shown in SEQ ID NO. 197 or SEQ ID NO. 201, and the nucleic acid encoding the first light chain of the first antigen binding portion has a nucleotide sequence as shown in SEQ ID NO. 199, SEQ ID NO. 205, SEQ ID NO. 211, SEQ ID NO. 215, or SEQ ID NO. 219. The nucleic acid encoding the second heavy chain of the second antigen binding portion comprises a nucleotide sequence as shown in SEQ ID NO. 223, and the nucleic acid encoding the second light chain of the second antigen binding portion comprises a nucleotide sequence as shown in SEQ ID NO. 225.

In a further preferred embodiment, the nucleic acid encoding the first heavy chain variable region of the first antigen binding portion of the bispecific antibody comprises a nucleotide sequence as shown in SEQ ID NO. 169, and the nucleic acid encoding the first light chain variable region of the first antigen binding portion comprises a nucleotide sequence as shown in SEQ ID NO. 172. The nucleic acid encoding the second heavy chain variable region of the second antigen binding portion comprises a nucleotide sequence as shown in SEQ ID NO. 158, and the nucleic acid encoding the second light chain variable region of the second antigen binding portion comprises a nucleotide sequence as shown in SEQ ID NO. 153. Further preferably the nucleic acid encoding the first heavy chain of the first antigen binding portion of the bispecific antibody comprises a nucleotide sequence as shown in SEQ ID NO. 197, and the nucleic acid encoding the first light chain of the first antigen binding portion comprises a nucleotide sequence as shown in SEQ ID NO. 199. The nucleic acid encoding the second heavy chain of the second antigen binding portion comprises a nucleotide sequence as shown in SEQ ID NO. 223, and the nucleic acid encoding the second light chain of the second antigen binding portion comprises a nucleotide sequence as shown in SEQ ID NO. 225.

In a further preferred embodiment, the nucleic acid encoding the first heavy chain variable region of the first antigen binding portion of the bispecific antibody comprises a nucleotide sequence as shown in SEQ ID NO. 203, and the nucleic acid encoding the first light chain variable region of the first antigen binding portion comprises a nucleotide sequence as shown in SEQ ID NO. 207. The nucleic acid encoding the second heavy chain variable region of the second antigen binding portion comprises a nucleotide sequence as shown in SEQ ID NO. 158, and the nucleic acid encoding the second light chain variable region of the second antigen binding portion comprises a nucleotide sequence as shown in SEQ ID NO. 153. Further preferably the nucleic acid encoding the first heavy chain of the first antigen binding portion of the bispecific antibody comprises a nucleotide sequence as shown in SEQ ID NO. 201, and the nucleic acid encoding the first light chain of the first antigen binding portion comprises a nucleotide sequence as shown in SEQ ID NO. 205. The nucleic acid encoding the second heavy chain of the second antigen binding portion comprises a nucleotide sequence as shown in SEQ ID NO. 223, and the nucleic acid encoding the second light chain of the second antigen binding portion comprises a nucleotide sequence as shown in SEQ ID NO. 225.

In a further preferred embodiment, the nucleic acid encoding the first heavy chain variable region of the first antigen binding portion of the bispecific antibody comprises a nucleotide sequence as shown in SEQ ID NO. 203, and the nucleic acid encoding the first light chain variable region of the first antigen binding portion comprises a nucleotide sequence as shown in SEQ ID NO. 213. The nucleic acid encoding the second heavy chain variable region of the second antigen binding portion comprises a nucleotide sequence as shown in SEQ ID NO. 158, and the nucleic acid encoding the second light chain variable region of the second antigen binding portion comprises a nucleotide sequence as shown in SEQ ID NO. 153. Further preferably the nucleic acid encoding the first heavy chain of the first antigen binding portion of the bispecific antibody comprises a nucleotide sequence as shown in SEQ ID NO. 201, and the nucleic acid encoding the first light chain of the first antigen binding portion comprises a nucleotide sequence as shown in SEQ ID NO. 211. The nucleic acid encoding the second heavy chain of the second antigen binding portion comprises a nucleotide sequence as shown in SEQ ID NO. 223, and the nucleic acid encoding the second light chain of the second antigen binding portion comprises a nucleotide sequence as shown in SEQ ID NO. 225.

In a further preferred embodiment, the nucleic acid encoding the first heavy chain variable region of the first antigen binding portion of the bispecific antibody comprises a nucleotide sequence as shown in SEQ ID NO. 203, and the nucleic acid encoding the first light chain variable region of the first antigen binding portion comprises a nucleotide sequence as shown in SEQ ID NO. 217. The nucleic acid encoding the second heavy chain variable region of the second antigen binding portion comprises a nucleotide sequence as shown in SEQ ID NO. 158, and the nucleic acid encoding the second light chain variable region of the second antigen binding portion comprises a nucleotide sequence as shown in SEQ ID NO. 153. Further preferably the nucleic acid encoding the first heavy chain of the first antigen binding portion of the bispecific antibody comprises a nucleotide sequence as shown in SEQ ID NO. 201, and the nucleic acid encoding the first light chain of the first antigen binding portion comprises a nucleotide sequence as shown in SEQ ID NO. 215. The nucleic acid encoding the second heavy chain of the second antigen binding portion comprises a nucleotide sequence as shown in SEQ ID NO. 223, and the nucleic acid encoding the second light chain of the second antigen binding portion comprises a nucleotide sequence as shown in SEQ ID NO. 225.

In a further preferred embodiment, the nucleic acid encoding the first heavy chain variable region of the first antigen binding portion of the bispecific antibody comprises a nucleotide sequence as shown in SEQ ID NO. 203, and the nucleic acid encoding the first light chain variable region of the first antigen binding portion comprises a nucleotide sequence as shown in SEQ ID NO. 221. The nucleic acid encoding the second heavy chain variable region of the second antigen binding portion comprises a nucleotide sequence as shown in SEQ ID NO. 158, and the nucleic acid encoding the second light chain variable region of the second antigen binding portion comprises a nucleotide sequence as shown in SEQ ID NO. 153. Further preferably the nucleic acid encoding the first heavy chain of the first antigen binding portion of the bispecific antibody comprises a nucleotide sequence as shown in SEQ ID NO. 201, and the nucleic acid encoding the first light chain of the first antigen binding portion comprises a nucleotide sequence as shown in SEQ ID NO. 219. The nucleic acid encoding the second heavy chain of the second antigen binding portion comprises a nucleotide sequence as shown in SEQ ID NO. 223, and the nucleic acid encoding the second light chain of the second antigen binding portion comprises a nucleotide sequence as shown in SEQ ID NO. 225.

In a preferred embodiment, the multispecific antibody is a trispecific antibody. The trispecific antibody comprises a light chain 1, a heavy chain 1, a light chain 2, and a heavy chain 2.

In a further preferred embodiment, the nucleic acid encoding the light chain 1 comprises a nucleotide sequence as shown in SEQ ID NO. 269, the nucleic acid encoding the heavy chain 1 comprises a nucleotide sequence as shown in SEQ ID NO. 271, a nucleotide sequence as shown in SEQ ID NO. 272, and a nucleotide sequence as shown in SEQ ID NO. 270, the nucleic acid encoding the light chain 2 comprises a nucleotide sequence as shown in SEQ ID NO. 153, and the nucleic acid encoding the heavy chain 2 comprises a nucleotide sequence as shown in SEQ ID NO. 158. Further preferably the nucleic acid encoding the light chain 1 of the trispecific antibody comprises a nucleotide sequence as shown in SEQ ID NO. 227, the nucleic acid encoding the heavy chain 1 comprises a nucleotide sequence as shown in SEQ ID NO. 229, the nucleic acid encoding the light chain 2 comprises a nucleotide sequence as shown in SEQ ID NO. 225, and the nucleic acid encoding the heavy chain 2 comprises a nucleotide sequence as shown in SEQ ID NO. 223.

In a further preferred embodiment, the nucleic acid encoding the light chain 1 comprises a nucleotide sequence as shown in SEQ ID NO. 269, the nucleic acid encoding the heavy chain 1 comprises a nucleotide sequence as shown in SEQ ID NO. 272, a nucleotide sequence as shown in SEQ ID NO. 271, and a nucleotide sequence as shown in SEQ ID NO. 270, the nucleic acid encoding the light chain 2 comprises a nucleotide sequence as shown in SEQ ID NO. 153, and the nucleic acid encoding the heavy chain 2 comprises a nucleotide sequence as shown in SEQ ID NO. 158. Further preferably the nucleic acid encoding the light chain 1 of the multispecific antibody comprises a nucleotide sequence as shown in SEQ ID NO. 227, the nucleic acid encoding the heavy chain 1 comprises a nucleotide sequence as shown in SEQ ID NO. 235, the nucleic acid encoding the light chain 2 comprises a nucleotide sequence as shown in SEQ ID NO. 225, and the nucleic acid encoding the heavy chain 2 comprises a nucleotide sequence as shown in SEQ ID NO. 223.

In a further preferred embodiment, the nucleic acid encoding the light chain 1 comprises a nucleotide sequence as shown in SEQ ID NO. 153, the nucleic acid encoding the heavy chain 1 comprises a nucleotide sequence as shown in SEQ ID NO. 271, a nucleotide sequence as shown in SEQ ID NO. 272, and a nucleotide sequence as shown in SEQ ID NO. 158, the nucleic acid encoding the light chain 2 comprises a nucleotide sequence as shown in SEQ ID NO. 269, and the nucleic acid encoding the heavy chain 2 comprises a nucleotide sequence as shown in SEQ ID NO. 270. Further preferably the nucleic acid encoding the light chain 1 of the multispecific antibody comprises a nucleotide sequence as shown in SEQ ID NO. 225, the nucleic acid encoding the heavy chain 1 comprises a nucleotide sequence as shown in SEQ ID NO. 237, the nucleic acid encoding the light chain 2 comprises a nucleotide sequence as shown in SEQ ID NO. 227, and the nucleic acid encoding the heavy chain 2 comprises a nucleotide sequence as shown in SEQ ID NO. 238.

In a further preferred embodiment, the nucleic acid encoding the light chain 1 comprises a nucleotide sequence as shown in SEQ ID NO. 153, the nucleic acid encoding the heavy chain 1 comprises a nucleotide sequence as shown in SEQ ID NO. 272, a nucleotide sequence as shown in SEQ ID NO. 271, and a nucleotide sequence as shown in SEQ ID NO. 158, the nucleic acid encoding the light chain 2 comprises a nucleotide sequence as shown in SEQ ID NO. 269, and the nucleic acid encoding the heavy chain 2 comprises a nucleotide sequence as shown in SEQ ID NO. 270. Further preferably the nucleic acid encoding the light chain 1 of the multispecific antibody comprises a nucleotide sequence as shown in SEQ ID NO. 225, the nucleic acid encoding the heavy chain 1 comprises a nucleotide sequence as shown in SEQ ID NO. 240, the nucleic acid encoding the light chain 2 comprises a nucleotide sequence as shown in SEQ ID NO. 227, and the nucleic acid encoding the heavy chain 2 comprises a nucleotide sequence as shown in SEQ ID NO. 238.

Additionally, the present disclosure provides a vector comprising the nucleic acid.

Additionally, the present disclosure provides a cell comprising the nucleic acid or the vector.

Additionally, the present disclosure provides a composition, which comprises the GPRC5D antibody or antigen binding fragment thereof, the multispecific antibody or antigen binding fragment thereof, the nucleic acid, the vector and/or the cell.

Additionally, the present disclosure provides a kit, which comprises the GPRC5D antibody or antigen binding fragment thereof, the multispecific antibody or antigen binding fragment thereof, the nucleic acid, the vector, the cell and/or the composition.

Additionally, the present disclosure provides use of the GPRC5D antibody or antigen binding fragment thereof, the multispecific antibody or antigen binding fragment thereof, the nucleic acid, the vector, the cell and/or the composition in the preparation of a drug or a kit for diagnosing, treating or preventing cancers or autoimmune diseases.

In a preferred embodiment, the cancers are B-cell related cancers, selected from multiple myeloma, malignant plasmacytoma, Hodgkin's lymphoma, nodular lymphocyte predominant Hodgkin's lymphoma, Kahler's disease, myeloid leukemia, plasmacytic leukemia, plasmacytoma, B-cell prolymphocytic leukemia, hairy cell leukemia, B-cell non-Hodgkin's lymphoma (NHL), acute myelogenous leukemia (AML), chronic lymphocytic leukemia (CLL), acute lymphocytic leukemia (ALL), chronic myelogenous leukemia (CML), follicular lymphoma, Burkitt's lymphoma, marginal zone lymphoma, mantle cell lymphoma, large cell lymphoma, precursor B-cell lymphocytic lymphoma, myeloid leukemia, Waldenström's macroglobulinaemia, diffuse large B-cell lymphoma, follicular lymphoma, marginal zone lymphoma, mucosa-associated lymphoid tissue lymphoma, small lymphocytic lymphoma, mantle cell lymphoma, Burkitt's lymphoma, primary mediastinal (thymic) large B-cell lymphoma, lymphoplasmacytic lymphoma, Waldenström's macroglobulinaemia, nodal marginal zone B-cell lymphoma, splenic marginal zone lymphoma, intravascular large B-cell lymphoma, primary effusion lymphoma, lymphomatoid granulomatosis, T-cell/histiocyte-rich large B-cell lymphoma, primary central nervous system lymphoma, primary cutaneous diffuse large B-cell lymphoma (leg type), EBV-positive diffuse large B-cell lymphoma of the elderly, inflammation-related diffuse large B- cell lymphoma\, intravascular large B-cell lymphoma, ALK-positive large B-cell lymphoma, plasmablastic lymphoma, large B-cell lymphoma produced in HHV8-associated multicentric Castleman's disease, unclassifiable B-cell lymphoma with intermediate features between diffuse large B-cell lymphoma and Burkitt's lymphoma, unclassifiable B-cell lymphoma with intermediate features between diffuse large B-cell lymphoma and classical Hodgkin lymphoma, and other B-cell associated lymphoma.

In a preferred embodiment, the autoimmune diseases are selected from immune nephropathy, systemic lupus erythematosus or rheumatoid arthritis.

In an embodiment of the present disclosure, the antibody is administered in a single dose or multiple doses.

If the antibody is administered in multiple doses in the present disclosure, the antibody is preferably administered in at least 3 doses, at least 4 doses, at least 5 doses, at least 6 doses, at least 7 doses, at least 8 doses, at least 9 doses or at least 10 doses and preferably at most 30 doses, 25 doses, 20 doses, 15 doses or 10 doses. The dose of the antibody is preferably administered at intervals of at least 7 days, at least 10 days, at least 14 days or at least 20 days. The dose of the antibody is preferably administered at intervals of 7 to 30 days, 10 to 20 days and preferably about 14 days.

In an aspect, the present disclosure provides use of a therapeutic agent in the preparation of drugs for treating cancer-related diseases in a subject, where the therapeutic agent is selected from a single therapeutic agent or a combination of therapeutic agents.

In some embodiments, the single therapeutic agent is selected from the antibody or antigen binding fragment thereof, the nucleic acid, the vector, the cell, and/or the composition.

In an aspect, the present disclosure provides a kit, which comprises the antibody or antigen binding fragment thereof, the nucleic acid, the vector, the cell, and/or the composition.

The kit according to the present disclosure comprises the antibody of the present disclosure, a fragment, a homologue, or a derivative thereof, such as a labeled or cytotoxic conjugate, and instructions for use of the antibody and a conjugate that kills a specific type of cells, etc. The instructions may include guidelines for in-vitro, in-vivo or ex-vivo use of the antibody and conjugate. The antibody can be in the form of a liquid or solid, and is usually lyophilized. The kit may contain other suitable reagents, such as a buffer, a solution for reconstitution, and other essential components for the intended use. A combination of reagents packaged in a predetermined amount and instructions for use, such as for therapeutic use or for diagnostic determination, are contemplated. When the antibody is labeled, for example, with an enzyme, the kit may include a substrate and cofactor required by the enzyme (e.g., a substrate precursor that provides a detectable chromophore or fluorophore). Additionally, other additives, such as a stabilizer and a buffer (such as a blocking buffer or a lysis buffer) can also be included. The relative amounts of various reagents can be changed to provide a concentrated solution of the reagents, which provides flexibility for users saves space, and saves reagents. These reagents can also be provided in the form of a dry powder, usually in a lyophilized form, including an excipient, which can provide a solution of a reagent with an appropriate concentration when dissolved.

In an aspect, the present disclosure provides use of the antibody or antigen binding fragment thereof, the nucleic acid, the vector, the cell and/or the composition in the preparation of a drug or a kit for diagnosing, treating or preventing cancer-related diseases.

Additionally, the antibody of the present disclosure can also be used in immunoassay, purification methods and other methods using immunoglobulin or fragments thereof. Such uses are well known in the art.

Correspondingly, the present disclosure also provides a composition containing the disclosed anti-GPRC5D antibody or a fragment thereof, where the antibody is conveniently combined with a pharmaceutically acceptable carrier, diluent or excipient, which is a common practice in the art.

As used in the present disclosure, the term "pharmaceutical composition" refers to a preparation of various reagents. A preparation containing a therapeutically effective amount of a multivalent antibody is in a sterile liquid solution, a liquid suspension or a lyophilized form, optionally containing a stabilizer or an excipient.

It is to be understood that the therapeutic agent according to the embodiment will be administered together with a suitable and pharmaceutically acceptable carrier, excipient, and other reagent incorporated into the preparation to provide improved transfer, delivery, and tolerance, etc. A large number of suitable preparations can be found in the pharmacopoeia known to pharmaceutical chemists, including: Remington's Pharmaceutical Sciences (15th edition, Mack Publishing Company, Easton, Pa.(1975)), especially in Chapter 87 of Blaug and Seymour. These preparations include, for example, powders, pastes, ointments, gels, waxes, oils, lipids, lipid-containing (cationic or anionic) carriers (for example, Lipofectin^{™}), DNA conjugates, anhydrous slurries, oil-in-water and water-in-oil emulsions, emulsified polyethylene glycols (polyethylene glycols of various molecular weights), semisolid gels and semisolid mixtures containing polyethylene glycols. Any of the mixtures may be suitable for the treatment or therapy in the present disclosure, provided that the active ingredient in the preparation is not inactivated by the preparation and the preparation is physiologically compatible and tolerant to the route of administration.

For inhalation administration, the compound is delivered as an aerosol spray from a pressurized container, dispenser or sprayer containing a suitable propellant such as carbon dioxide.

It can also be administered systemically through transmucosal or transdermal routes. For transmucosal or transdermal administration, a penetrant suitable for permeation through a barrier is used in the preparation. Such penetrants are generally known in the art and include detergents, bile salts and fusidic acid derivatives, for example, for transmucosal administration. The transmucosal administration can be achieved by using nasal sprays or suppositories. For transdermal administration, one or more of the antibodies can be formulated into a paste, an ointment, a gel, or a cream as commonly known in the art.

The compound can also be prepared into the form of a suppository (e.g., with a conventional suppository base, such as cocoa butter or other glycerides) or a retention enema for rectal delivery.

In an embodiment, the antibody can be prepared with a carrier that prevent it from being rapidly eliminated in the body, into, for example, sustained/controlled-release preparations, including implants and microencapsulated delivery systems. Biodegradable and biocompatible polymers, such as ethylene-vinyl acetate, polyanhydride, polyglycolic acid, collagen, polyorthoester and polylactic acid can be used. Methods for preparing such preparations are obvious to those skilled in the art.

It is particularly advantageous to prepare parenteral compositions in unit dosage form for ease of administration and consistency of dosage. As used herein, unit dosage form refers to a physically discrete unit suitable for use as a unit dose for the subject to be treated. Each unit contains a predetermined amount of one or more of the antibodies calculated to combine with a desired drug carrier to produce a desired therapeutic effect. The specification of the unit dosage form of the embodiment is indicated by and directly depends on the unique characteristics of the antibody, the specific therapeutic effect to be achieved, and the inherent limitations in the art of preparation of such antibodies for treatment in an individual.

The pharmaceutical composition can be packaged in a container, a package, or a dispenser together with the instructions for administration.

The preparations described herein may also contain more than one of the antibodies according to the specific conditions to be treated, preferably those with complementary activities but without negative effects on each other. Alternatively or additionally, the composition may, for example, contain an agent that enhances its function, such as a cytotoxic agent, a cytokine, a chemotherapeutic agent, or a growth inhibitor. Such molecules are suitably combined in an amount effective for the intended purpose. For example, they can be combined in a kit or during use.

In an embodiment, one or more of the antibodies can be administered in a combination therapy, that is, in combination with an additional agent such as therapeutic agent (which can be used to treat pathological conditions or disorders, such as various forms of cancer and inflammatory diseases). The term "combination" herein refers to substantially concurrent, simultaneous or sequential administration of reagents. If administered sequentially, the first of the two compounds is still preferably detectable at an effective concentration at the treatment site when the second compound is administered. In one case, "combination" may also means presence of the antibody of the present disclosure and other therapeutic agents in the kit.

For example, the combination therapy may include the co-preparation and/or coadministration of one or more antibodies described herein with one or more additional therapeutic agents (e. g., one or more cytokines and growth factor inhibitors, immunosuppressants, anti-inflammatory agents, metabolic inhibitors, enzyme inhibitors, and/or cytotoxins or cell growth inhibitors, as described in further detail below). Such combination therapy can advantageously utilize a lower dose of the therapeutic agents administered, to avoid possible toxicity or complications associated with various monotherapies.

Experiments in the present disclosure prove that the anti-GPRC5D antibody provided in the present disclosure can bind to human GPRC5D receptor with a high affinity, and to Cynomolgus GPRC5D with an affinity comparable to that to human GPRC5D receptor. The novel humanized GPRC5Dx CD3 bispecific antibody constructed with the anti-GPRC5D antibody provided in the present disclosure has a strong killing effect on low expressing cells and is gentle in activating T cells. In a mouse model with transplanted tumor, a GPRC5D×BCMA×CD3 trispecific antibody can kill both the BCMA⁺ cells and the GPRC5D⁺ cells. The killing effect on myeloma cells is significantly better than that of BCMA×CD3 or GPRC5D×CD3 bispecific antibody. The present disclosure overcomes the resistance to drugs against single targets, and produces a more potent and lasting anti-tumor effect in clinical use.

For the purpose of clarity and brevity of description, features are described herein as part of some embodiments that are the same or separate. However, it should be understood that the scope of the present disclosure may include some embodiments having a combination of all or some of the described features.

### Example

### Example 1. Production of anti-GPRC5D antibody

### (1) Construction of GPRC5D stably transfected cell line

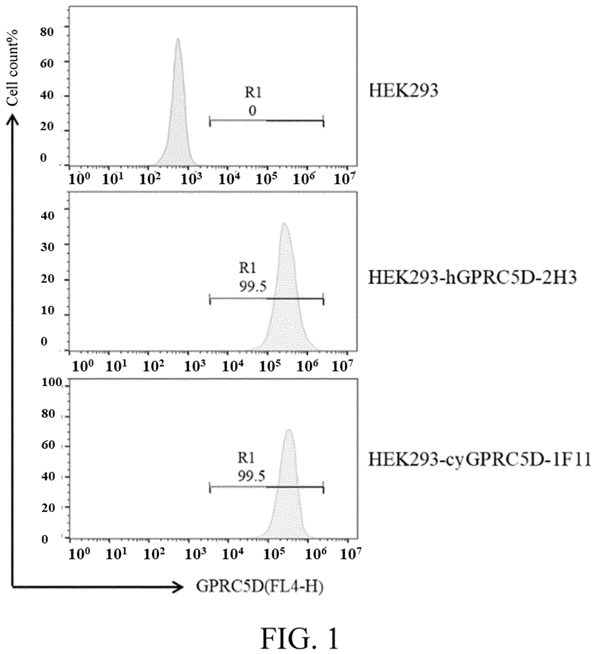

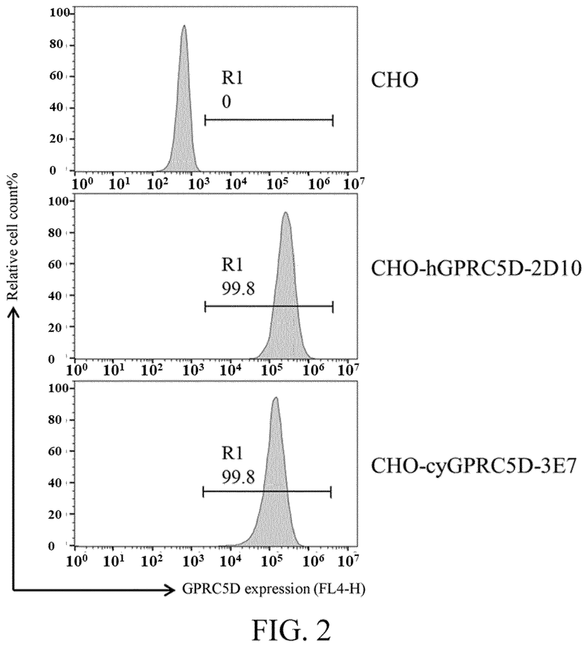

Using human GPRC5D cDNA (Sino Biological, product number: HG24447-UT, and the amino acid sequence of which is as shown in SEQ ID NO. 1) or Cynomolgus GPRC5D cDNA (synthesized by Suzhou Genewiz Inc., and the amino acid sequence of which is as shown in SEQ ID NO. 2) as a template, a lentiviral vector plasmid containing the full-length sequence of human or Cynomolgus GPRC5D was constructed. Following the instructions of a Lenti-Pac HIV Expression Packaging Kit (Gene Copoeia, product number: HPK-LvTR-20), the constructed lentiviral plasmid and a packaging plasmid were co-transfected into HEK293T cells (ATCC CRL-11268), for lentiviral packaging. 48 h after transfection, the culture medium was collected, and the cell debris was removed by centrifugation at 500*g for 10 min, to obtain a culture supernatant containing lentiviral particles. After filtration through a 0.45 µm PES membrane, the culture supernatant was subpackaged into 1.5 mL EP tubes. 10 µL of the culture supernatant was used to infect 1 × 10⁶ HEK293T or CHO cells (ATCC CCL-61). After 48 h, 8 µg/ml puromycin was added to screen stably transfected cells. As a result, monoclonal cell lines HEK293-hGPRC5D (clone 2H3), HEK293-cyGPRC5D (clone 1F11), CHO-hGPRC5D (clone 2D10) and CHO-cyGPRC5D (clone 3E7) highly expressing human or Cynomolgus GPRC5D were obtained, which were simply referred to as HEK293-hGPRC5D, HEK293-cyGPRC5D, CHO-hGPRC5D and CHO-cyGPRC5D respectively. The results are shown in Figs. 1 and 2.

The amino acid sequence of human GPRC5D is as shown in SEQ ID NO. 1:

MYKDCIESTGDYFLLCDAEGPWGIILESLAILGIVVTILLLLAFLFLMRKIQDCSQWNVLPTQLLFLLSVLGLFG
LAFAFIIELNQQTAPVRYFLFGVLFALCFSCLLAHASNLVKLVRGCVSFSWTTILCIAIGCSLLQIIIATEYVTL
IMTRGMMFVNMTPCQLNVDFVVLLVYVLFLMALTFFVSKATFCGPCENWKQHGRLIFITVLFSIIIWVVWISMLL
RGNPQFQRQPQWDDPVVCIALVTNAWVFLLLYIVPELCILYRSCRQECPLQGNACPVTAYQHSFQVENQELSRAR
DSDGAEEDVALTSYGTPIQPQTVDPTQECFIPQAKLSPQQDAGGV

The amino acid sequence of cynomolgus GPRC5Dis as shown in SEQ ID NO. 2:

MYKDCIESTGDYFLPCDSEGPWGIVLESLAILGIVVTILLLLAFLFLMRKIQDCSQWNVLPTQLLFLLSVLGLFG
LAFAFIIQLNQQTAPVRYFLFGVLFALCFSCLLAHASNLVKLVRGRVSFSWTTILCIAIGCSLLQVIIAIEYVTL
IMTRGMMFVHMTPYQLNVDFVVLLVYVLFLMALTFFVSKATFCGPCENWKQHGRLIFITVLFSIIIWVVWISMLL
RGNPQFQRQPQWDDPVVCIALVTNAWVFLLLYIVPELCILYRSCRQECPSQGHACPVTAYQRSFQVENQELSRAR
DSDGAEEDVALTSFGTPIQPQTVDPTQECFIPRAKLSPQQDAGV

### (2) Animal immunization and production of hybridoma

Female Balb/C mice aged 4-6 weeks were used. A eukaryotic expression plasmid containing CMV promoter was constructed with human or Cynomolgus GPRC5D full-length sequence. The mice were alternately immunized by a gene gun using human and Cynomolgus GPRC5D DNA (20 µg plasmid/mouse). After immunization for 4-5 times, the mice with required serum titers were challenged with CHO-hGPRC5D stably transfected cells (5 x 10⁶ cells/animal). After 3 days, the mice were sacrificed by cervical dislocation, and the spleen and some peripheral lymph nodes were harvested from the mice, and ground in a DMEM medium to obtain a B cell-rich suspension. A proper amount of suspension of containing the lymph node and spleen cells was mixed with SP2/0, and the cells were fused by an electrofusion instrument (BTX). After electrofusion, the cells were plated using ClonaCell^{™}-HY Medium D (STEMCELL Technologies), and IgG⁺ positive monoclonal antibodies were picked by Clonepix (Molecular Devices) into a 96 well plate. After 2-3 days of culture, the supernatant was collected and used to screen hybridoma clones binding HEK293-hGPRC5D and HEK293-cyGPRC5D cells.

### Example 2. Screening of hybridoma clones binding human and Cynomolgus GPRC5D

5 x 10⁴ HEK293-hGPRC5D or HEK293-cyGPRC5D cells per well were added into a 96-well U-shaped plate, and centrifuged. The supernatant was discarded. 100 µL/well of the hybridoma culture supernatant was added, and incubated on ice for 60 min. The unbound primary antibody was washed away, and then 50 µL/well of a secondary antibody solution (Anti-mouse IgG, Fc specific-AF647, Jackson Immuno Research) was added, and incubated on ice for 30 min. The remaining secondary antibody was washed away, PBS was added to resuspend the cells, and the binding of the antibody to the cells was detected by a flow cytometer (Intellicyte iQue). A total of 290 hybridoma clones specifically binding hGPRC5D and cyGPRC5D stably transfected cells were obtained after primary screening. The supernatant of the above-mentioned positive clones were enriched and purified, and the clones binding to GPRC5D⁺ tumor cells were further screened. Among them, 77 clones had higher signals of binding to myeloma cells MM. 1S and MM.1R (both purchased from ATCC, product numbers CRL-2974 and CRL-2975).

RNA of candidate hybridoma monoclonal cells was extracted by the TRNzol lysis method, and then reversely transcribed to synthesize a single-stranded cDNA, which was used as a template to amplify the variable region sequence of the antibody in the hybridoma monoclonal cells. The light chain variable region of the antibody was cloned into a vector containing a human light chain constant region, and the heavy chain variable region was cloned into a vector containing the human heavy chain constant region. The plasmids were co-transfected into CHO-S cells. A complete IgG antibody was expressed and secreted into the culture medium. After 7 days of expression at 37°C with 5%CO₂ at 125 rpm, the supernatant was collected. The chimeric IgG was purified by Protein A chromatography. The concentration of IgG was determined by spectrophotometry. The control antibody GC5B596 was synthesized as described in the patent document US10562968B2, expressed in CHO cells and purified by ProteinA chromatography.

The binding of the antibodies to human and Cynomolgus GPRC5D stably transfected cells was detected by flow cytometry. The cell suspension of CHO-hGPRC5D or CHO-cyGPRC5D stably transfected cells prepared in Example 1 was adjusted to a concentration of 5 x 10⁵ cells/ml. Then, 100 µl/ well of the cell suspension was added to a 96-well U-shaped plate, and centrifuged at 300g for 5 min. The supernatant was discarded. 100 µL per well of serially diluted antibody was added (initial concentration: 200 nM, 3-fold dilution, 12 gradients), and incubated at 4°C for 45 min. The secondary antibody 50 µL/well of Alexa Fluro647 labeled goat anti-human IgG Fc (1:300 dilution) was added, incubated on ice for 45 min, and detected by flow cytometry.

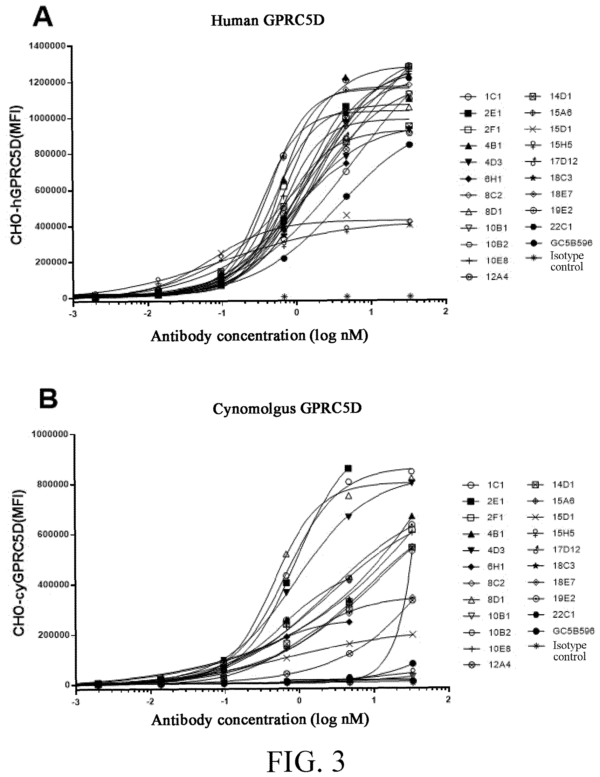

Fig. 3 shows the binding of anti-GPRC5D chimeric antibody with GPRC5D stably transfected cell line. Fig. 3A shows the result of binding of the chimeric antibody with human GPRC5D stably transfected cell line detected by flow cytometry, indicating that the activities of all chimeric binding are significantly better than GC5B596. Fig. 3B shows the result of binding of the chimeric antibody with Cynomolgus GPRC5D stably transfected cells detected by flow cytometry, where clones 2E1, 2F1, 4B 1, 4D3, 8D1, 10B2, 10E8, 15D1 and 19E2 have strong binding ability, and GC5B596 only weakly binds to the Cynomolgus GPRC5D stably transfected cells at the highest concentration of 200 nM. The results are shown in Table 1.

**Table 1. Binding of anti-GPRC5D chimeric antibody to stably transfected cell lines and tumor cells**

| Clone | Human GPRC5D | Cynomolgus GPRC5D |
|---|---|---|
| 1C1 | ++++ | + |
| 2E1 | ++++ | ++++ |
| 2F1 | ++++ | ++++ |
| 4B1 | ++++ | ++++ |
| 4D3 | ++++ | ++++ |
| 6H1 | ++++ | ++ |
| 8C2 | ++++ | ++ |
| 8D1 | ++++ | ++++ |
| 10B1 | ++++ | + |
| 10B2 | ++++ | ++++ |
| 10E8 | ++++ | ++++ |
| 12A4 | ++++ | ++ |
| 14D1 | ++++ | +++ |
| 15A6 | ++++ | +++ |
| 15D1 | ++++ | ++ |
| 15H5 | ++++ | + |
| 17D12 | ++++ | +++ |
| 18C3 | ++++ | + |
| 18E7 | ++++ | +++ |
| 19E2 | ++++ | ++++ |
| 22C1 | ++++ | + |
| GC5B596 | +++ | + |
| Isotype control | Not binding | Not binding |

The variable region sequence of the anti-GPRC5D monoclonal antibody is shown below:

**Table 2. Variable region sequence of murine GPRC5D antibody**

| GPRC5D Murine antibody | HCVR | | HCDR1 | HCDR2 | HCDR3 | LCVR | | LCDR1 | LCDR2 | LCDR3 |
|---|---|---|---|---|---|---|---|---|---|---|
| | Amino acid sequence | Nucleotide sequence | Amino acid sequence | Amino acid sequence | Amino acid sequence | Amino acid sequence | Nucleotide sequence | Amino acid sequence | Amino acid sequence | Amino acid sequence |
| 1C1 | 3 | 4 | 5 | 6 | 7 | 8 | 9 | 10 | 11 | 12 |
| 2E1 | 13 | 14 | 15 | 16 | 17 | 18 | 19 | 20 | 21 | 22 |
| 2F1 | 23 | 24 | 25 | 26 | 27 | 28 | 29 | 30 | 31 | 32 |
| 4B1 | 33 | 34 | 5 | 35 | 7 | 36 | 37 | 38 | 21 | 39 |
| 4D3 | 40 | 41 | 42 | 43 | 44 | 45 | 46 | 47 | 48 | 49 |
| 6H1 | 50 | 51 | 5 | 52 | 7 | 53 | 54 | 38 | 21 | 55 |
| 8C2 | 56 | 57 | 58 | 59 | 60 | 61 | 62 | 63 | 64 | 65 |
| 8D1 | 66 | 67 | 68 | 69 | 70 | 71 | 72 | 73 | 74 | 75 |
| 10B1 | 76 | 77 | 241 | 78 | 79 | 80 | 81 | 82 | 83 | 84 |
| 10B2 | 40 | 41 | 42 | 43 | 44 | 85 | 86 | 47 | 48 | 49 |
| 10E8 | 87 | 88 | 25 | 89 | 90 | 91 | 92 | 93 | 94 | 95 |
| 12A4 | 96 | 97 | 98 | 99 | 100 | 101 | 102 | 103 | 104 | 105 |
| 14D1 | 106 | 107 | 108 | 99 | 109 | 110 | 111 | 112 | 104 | 105 |
| 15A6 | 113 | 114 | 98 | 115 | 116 | 110 | 111 | 112 | 104 | 105 |
| 15D1 | 117 | 118 | 119 | 99 | 120 | 121 | 122 | 123 | 124 | 125 |
| 15H5 | 126 | 127 | 108 | 99 | 116 | 128 | 129 | 130 | 104 | 105 |
| 17D12 | 131 | 132 | 108 | 133 | 134 | 135 | 136 | 112 | 104 | 105 |
| 18C3 | 137 | 138 | 58 | 59 | 139 | 61 | 62 | 63 | 64 | 65 |
| 18E7 | 140 | 141 | 98 | 99 | 109 | 101 | 102 | 103 | 104 | 105 |
| 19E2 | 140 | 141 | 98 | 99 | 109 | 142 | 143 | 103 | 104 | 144 |
| 22C1 | 145 | 146 | 25 | 147 | 148 | 149 | 150 | 93 | 151 | 95 |

### 1C1

The amino acid sequence of VH is as shown in SEQ ID NO. 3, the coding nucleic acid of which is as shown in SEQ ID NO. 4, and the HCDR1, HCDR2, and HCDR3 of which are respectively as shown in SEQ ID NOs. 5, 6, and 7.

QAYLQQSGAELVRSGASVKMSCKASGYTFTSYNMHWVKQTPGQGLEWIGYIYPGNGGTNYNQKFKAKATLTADTSSS
TAYMQISSLTSEDSAVYFCARGYGYRYWYFDVWGAGTTVTVSS

### Nucleic acid sequence

CAGGCTTATCTACAGCAGTCTGGGGCTGAGCTGGTGAGGTCTGGGGCCTCAGTGAAGATGTCCTGCAAGGCTTCTGG
CTACACATTTACCAGTTACAATATGCACTGGGTAAAGCAGACACCTGGGCAGGGCCTGGAATGGATTGGATATATTT
ATCCTGGAAATGGTGGTACTAACTACAATCAGAAGTTCAAGGCCAAGGCCACATTGACTGCAGACACATCCTCCAGC
ACAGCCTACATGCAGATCAGCAGCCTGACATCTGAAGACTCTGCGGTCTATTTCTGTGCAAGAGGGTATGGTTACCG
CTACTGGTACTTCGATGTCTGGGGCGCAGGGACCACGGTCACCGTCTCCTCA

The amino acid sequence of VL is as shown in SEQ ID NO. 8, the coding nucleic acid of which is as shown in SEQ ID NO. 9, and the LCDR1, LCDR2, and LCDR3 of which are respectively as shown in SEQ ID NOs. 10, 11, and 12.

NIVLTQSPAIMSASPGEKVTMTCSASSSVRFMHWYQQKSGTSPKRWIYDTSKVASGVPARFSGSGSGTSYSLTIRSM
EAEDAATYYCQQWSSNPLTFGAGTKLELK

### Nucleic acid sequence

AATATTGTGCTAACTCAGTCTCCAGCAATCATGTCTGCATCTCCAGGGGAGAAGGTCACCATGACCTGCAGTGCCAG
CTCAAGTGTACGTTTCATGCACTGGTACCAGCAGAAGTCAGGCACCTCCCCCAAAAGATGGATTTATGATACATCCA
AAGTGGCTTCTGGAGTCCCTGCTCGCTTCAGTGGCAGTGGGTCTGGGACCTCTTACTCTCTCACAATCAGAAGCATG
GAGGCTGAAGATGCTGCCACTTATTACTGCCAGCAGTGGAGTAGTAACCCGCTCACGTTCGGTGCTGGGACCAAGCT
GGAGCTGAAA

### 2E1

The amino acid sequence of VH is as shown in SEQ ID NO. 13, the coding nucleic acid of which is as shown in SEQ ID NO. 14, and the HCDR1, HCDR2, and HCDR3 of which are respectively as shown in SEQ ID NOs. 15, 16, and 17.

QVQLQQPGAELVKPGASVKLSCKASGYTFTRYWMHWVKQRPGQGLDWIGEFNPSNGRINYNEKFKNKATLTIDKSSS
TAYMQLSSLTSEDSAVYYCTRGFAYWGQGTLVTVSA

### Nucleic acid sequence

CAGGTCCAACTGCAGCAGCCTGGGGCTGAACTGGTGAAGCCTGGGGCTTCAGTGAAGCTGTCCTGCAAGGCTTCTGG
CTACACCTTCACCCGCTACTGGATGCACTGGGTGAAGCAGAGGCCTGGACAAGGCCTTGATTGGATTGGAGAGTTTA
ATCCTAGTAACGGTCGTATTAACTACAATGAGAAGTTCAAGAACAAGGCCACACTGACTATAGACAAGTCCTCCAGC
ACTGCCTACATGCAACTCAGCAGCCTGACATCTGAGGACTCTGCGGTCTATTACTGTACAAGAGGGTTTGCTTACTG
GGGCCAAGGGACTCTGGTCACTGTCTCTGCA

The amino acid sequence of VL is as shown in SEQ ID NO. 18, the coding nucleic acid of which is as shown in SEQ ID NO. 19, and the LCDR1, LCDR2, and LCDR3 of which are respectively as shown in SEQ ID NOs. 20, 21, and 22.

QIVLTQSPAIMSASPGERVTMTCSASSTVSYIHWYQQKSGTSPKRWIYDTSKLASGVPARFSGSGSGTSYSLTISSM
EAEDAATYYCQQWTTNPWTFGGGTKLEIK

### Nucleic acid sequence

CAAATTGTTCTCACCCAGTCTCCAGCAATCATGTCTGCATCTCCAGGGGAGAGGGTCACCATGACCTGCAGTGCCAG
CTCAACTGTAAGTTACATACACTGGTACCAGCAGAAGTCAGGCACCTCCCCCAAAAGATGGATTTATGACACATCCA
AACTGGCTTCTGGAGTCCCTGCTCGCTTCAGTGGCAGTGGGTCTGGGACCTCTTACTCTCTCACAATCAGCAGCATG
GAGGCTGAAGATGCTGCCACTTATTACTGCCAGCAGTGGACTACTAACCCGTGGACGTTCGGTGGAGGCACCAAGCT
GGAAATCAAA

### 2F1

The amino acid sequence of VH is as shown in SEQ ID NO. 23, the coding nucleic acid of which is as shown in SEQ ID NO. 24, and the HCDR1, HCDR2, and HCDR3 of which are respectively as shown in SEQ ID NOs. 25, 26, and 27.

EVLLQQSGPELVKPGTSMKISCKASGYSFTGYTMNWVKQRHGKNLEWIGLINPYNGGTRYNQKFKGRATLTVDKSSS
TAYMELLSLTSEDSAVYYCARWALRYGMDYWGQGTSVTVSS

### Nucleic acid sequence

GAGGTCCTGCTGCAACAGTCTGGACCTGAACTGGTGAAGCCTGGAACTTCAATGAAGATATCCTGCAAGGCTTCTGG
TTACTCATTCACTGGCTACACCATGAACTGGGTGAAACAGAGACATGGAAAGAACCTTGAGTGGATTGGACTTATCA
ATCCTTACAATGGTGGTACTAGGTACAACCAGAAGTTCAAGGGCAGGGCCACATTAACTGTGGACAAGTCATCCAGC
ACAGCCTACATGGAACTCCTCAGTCTGACATCTGAGGACTCTGCAGTCTATTACTGTGCAAGATGGGCCCTACGGTA
TGGTATGGACTACTGGGGTCAAGGAACCTCAGTCACCGTCTCCTCA

The amino acid sequence of VL is as shown in SEQ ID NO. 28, the coding nucleic acid of which is as shown in SEQ ID NO. 29, and the LCDR1, LCDR2, and LCDR3 of which are respectively as shown in SEQ ID NOs. 30, 31, and 32.

DIVMTQSQKFMSTSVGDRVSVTCKASQNVGTNVAWYQQKPGHSPKALIYSASYRNSGVPDRFTGSGSGTDFTLTISN
VQSEDLAEYFCQQYNSYLLTFGGGTKLELK

### Nucleic acid sequence

GACATTGTGATGACCCAGTCTCAAAAATTCATGTCCACATCAGTAGGAGACAGGGTCAGCGTCACCTGCAAGGCCAG
TCAGAACGTGGGTACTAATGTAGCCTGGTATCAACAGAAACCAGGGCATTCTCCTAAAGCACTGATTTACTCGGCAT
CCTACCGGAATAGTGGAGTCCCTGATCGCTTCACAGGCAGTGGCTCTGGGACAGATTTCACTCTCACCATCAGCAAT
GTGCAGTCTGAAGACTTGGCAGAGTATTTCTGTCAGCAATATAACAGCTATCTTCTCACGTTCGGAGGGGGGACCAA
GCTGGAGCTGAAA

### 4B1

The amino acid sequence of VH is as shown in SEQ ID NO. 33, the coding nucleic acid of which is as shown in SEQ ID NO. 34, and the HCDR1, HCDR2, and HCDR3 of which are respectively as shown in SEQ ID NOs. 5, 35, and 7.

QAYLQQSGAELVRSGASVKMSCKASGYTFTSYNMHWVKQTPGQGLEWIGYIYPGNGGTNYNQNFKGKATLTADTSSS
TAYMQISSLTSEDSAVYFCARGYGYRYWYFDVWGAGTTVTVSS

### Nucleic acid sequence

CAGGCTTATCTACAGCAGTCTGGGGCTGAGCTGGTGAGGTCTGGGGCCTCAGTGAAGATGTCCTGTAAGGCTTCTGG
CTACACATTTACCAGTTACAATATGCACTGGGTAAAGCAGACACCTGGACAGGGCCTGGAATGGATTGGATATATTT
ATCCTGGAAATGGTGGTACTAACTACAATCAGAATTTCAAGGGCAAGGCCACATTGACTGCAGACACTTCCTCCAGC
ACAGCCTACATGCAGATCAGCAGTCTGACATCTGAAGACTCTGCGGTCTATTTCTGTGCAAGAGGGTATGGTTACCG
CTACTGGTACTTCGATGTCTGGGGCGCAGGGACCACGGTCACCGTCTCCTCA

The amino acid sequence of VL is as shown in SEQ ID NO. 36, the coding nucleic acid of which is as shown in SEQ ID NO. 37, and the LCDR1, LCDR2, and LCDR3 of which are respectively as shown in SEQ ID NOs. 38, 21, and 39.

QIVLTQSPAIMSASPGEKVTMTCSASSSVSYMHWYQQKSGTSPKRWIYDTSKLASGVPVRFSGSGSGTSYSLTISSM
EAEDAATYYCQQWSNYPPTFGSGTKLEIK

### Nucleic acid sequence

CAAATTGTTCTCACCCAGTCTCCAGCAATCATGTCTGCATCTCCAGGGGAGAAGGTCACCATGACCTGCAGTGCCAG
CTCAAGTGTAAGTTACATGCACTGGTACCAGCAGAAGTCAGGCACCTCCCCCAAAAGATGGATTTATGACACATCCA
AACTGGCTTCTGGAGTCCCTGTTCGTTTCAGTGGCAGTGGGTCTGGGACCTCTTACTCTCTCACAATCAGCAGCATG
GAGGCTGAAGATGCTGCCACTTATTACTGCCAGCAGTGGAGTAATTACCCACCCACGTTCGGCTCGGGGACAAAGTT
GGAAATAAAA

### 4D3

The amino acid sequence of VH is as shown in SEQ ID NO. 40, the coding nucleic acid of which is as shown in SEQ ID NO. 41, and the HCDR1, HCDR2, and HCDR3 of which are respectively as shown in SEQ ID NOs. 42, 43, and 44.

QVQLQQSGAELVKPGASVKMSCKAFGYTFTNFPIEWMKQNRGRSLEWIGNFHPYNDVTKYNEKFKGKAKLTVEKSSS
TVYLELSRLTSDDSAVYYCALITRGYWGQGTTLTVSS

### Nucleic acid sequence

CAGGTTCAGCTGCAACAGTCTGGGGCTGAGCTGGTGAAGCCTGGGGCCTCAGTGAAGATGTCCTGCAAGGCTTTTGG
CTACACCTTCACTAACTTTCCAATAGAGTGGATGAAGCAGAATCGTGGGAGGAGCCTAGAGTGGATTGGAAATTTTC
ATCCTTACAATGATGTTACTAAGTACAATGAAAAATTCAAGGGCAAGGCCAAATTGACTGTAGAGAAATCCTCTAGC
ACAGTCTACTTGGAGCTCAGCCGATTAACATCTGATGACTCTGCTGTTTATTACTGTGCCCTTATTACACGGGGCTA
CTGGGGCCAAGGCACCACTCTCACAGTCTCCTCA

The amino acid sequence of VL is as shown in SEQ ID NO. 45, the coding nucleic acid of which is as shown in SEQ ID NO. 46, and the LCDR1, LCDR2, and LCDR3 of which are respectively as shown in SEQ ID NOs. 47, 48, and 49.

DIVMSQSPSSLGVSVGEKVTMSCKSSQSLLYSRNQKNSLAWYQQKPGQSPKLLIYWASTRESGVPDRFTGSGSGTDF
TLTISSVKAEDLAVYYCQQYYSYPLTFGAGTKLELK

### Nucleic acid sequence

GACATTGTGATGTCACAGTCTCCATCCTCCCTAGGTGTGTCAGTTGGAGAGAAGGTTACTATGAGCTGCAAGTCCAG
TCAGAGCCTTTTATATAGTCGCAATCAAAAGAACTCCTTGGCCTGGTACCAGCAGAAACCTGGGCAGTCTCCTAAAC
TGCTGATTTACTGGGCATCCACTAGGGAATCTGGGGTCCCTGATCGCTTCACAGGCAGTGGATCTGGGACAGATTTC
ACTCTCACCATCAGCAGTGTGAAGGCTGAAGACCTGGCAGTTTATTACTGTCAGCAATATTATAGCTATCCGCTCAC
GTTCGGTGCTGGGACCAAGCTGGAGCTGAAA

### 6H1

The amino acid sequence of VH is as shown in SEQ ID NO. 50, the coding nucleic acid of which is as shown in SEQ ID NO. 51, and the HCDR1, HCDR2, and HCDR3 of which are respectively as shown in SEQ ID NOs. 5, 52, and 7.

QAYLQQSGAELVRSGASVKMSCKASGYTFTSYNMHWVKQTPGQGLEWIGYIYPGNGGTNYNQKFKGKATLTADTSSS
TAYMQISSLTSEDSAVYFCARGYGYRYWYFDVWGTGTTVTVSS

### Nucleic acid sequence

CAGGCTTATCTACAGCAGTCTGGGGCTGAGCTGGTGAGGTCTGGGGCCTCAGTGAAGATGTCCTGCAAGGCTTCTGG
CTACACATTTACCAGTTATAATATGCACTGGGTAAAGCAGACACCTGGGCAGGGCCTGGAATGGATTGGATATATTT
ATCCTGGAAATGGTGGTACTAACTACAATCAGAAATTCAAGGGCAAGGCCACATTGACTGCAGACACATCCTCCAGC
ACAGCCTACATGCAGATCAGCAGCCTGACATCTGAAGACTCTGCGGTCTATTTCTGTGCAAGAGGGTATGGTTACCG
CTACTGGTACTTCGATGTCTGGGGCACAGGGACCACGGTCACCGTCTCCTCA

The amino acid sequence of VL is as shown in SEQ ID NO. 53, the coding nucleic acid of which is as shown in SEQ ID NO. 54, and the LCDR1, LCDR2, and LCDR3 of which are respectively as shown in SEQ ID NOs. 38, 21, and 55.

QIVLTQSPAIMSASPGEKVTMTCSASSSVSYMHWYQQKSGTSPKRWIYDTSKLASGVPARFSGSGSGTSYSLTISSM
EAEDAATYYCQQWNSKPPTFGSGTKLEIK

### Nucleic acid sequence

CAAATTGTTCTCACCCAGTCTCCAGCAATCATGTCTGCATCTCCAGGGGAGAAGGTCACCATGACCTGCAGTGCCAG
CTCAAGTGTAAGTTACATGCACTGGTACCAGCAGAAGTCAGGCACCTCCCCCAAAAGATGGATTTATGACACATCCA
AACTGGCTTCTGGAGTCCCTGCTCGCTTCAGTGGCAGTGGGTCTGGGACCTCATACTCTCTCACAATCAGCAGCATG
GAGGCTGAAGATGCTGCCACTTATTACTGCCAACAGTGGAATAGTAAGCCACCCACGTTCGGCTCGGGGACAAAGTT
GGAAATAAAA

### 8C2

The amino acid sequence of VH is as shown in SEQ ID NO. 56, the coding nucleic acid of which is as shown in SEQ ID NO. 57, and the HCDR1, HCDR2, and HCDR3 of which are respectively as shown in SEQ ID NOs. 58, 59, and 60.

EVQLVESGGDLVKPGGSLKLSCAASGFTFSSYGMSWVRQTPDKRLEWVATISSGGSYTYYPDSVKGRFTISRDNAKN
TLYLQMSSLKSEDTAMYYCARHGGSSSYVMDYWGQGTSVTVSS

### Nucleic acid sequence

GAGGTGCAGCTGGTGGAGTCTGGGGGAGACTTAGTGAAGCCTGGAGGGTCCCTGAAACTCTCCTGTGCAGCCTCTGG
ATTCACTTTCAGTAGCTATGGCATGTCTTGGGTTCGCCAGACTCCAGACAAGAGGCTGGAGTGGGTCGCAACCATTA
GTAGTGGTGGTAGTTACACCTACTATCCAGACAGTGTGAAGGGGCGATTCACCATCTCCAGGGACAATGCCAAGAAC
ACCCTGTACCTGCAAATGAGCAGTCTGAAGTCTGAGGACACAGCCATGTATTACTGTGCAAGACATGGGGGTAGTAG
CTCTTATGTTATGGACTACTGGGGTCAAGGAACCTCAGTCACCGTCTCCTCA

The amino acid sequence of VL is as shown in SEQ ID NO. 61, the coding nucleic acid of which is as shown in SEQ ID NO. 62, and the LCDR1, LCDR2, and LCDR3 of which are respectively as shown in SEQ ID NOs. 63, 64, and 65.

DIVMTQAAPSVPVTPGESVSISCRSSKSLLHSNGNTYLYWFLQRPGQSPQLLIYRMSNLASGVPDRFSGSGSGTAFT
LRISRVEAEDVGVYYCMQHLEYPFTFGSGTKLEIK

### Nucleic acid sequence

GATATTGTGATGACTCAGGCTGCACCCTCTGTACCTGTCACTCCTGGAGAGTCAGTATCCATCTCCTGCAGGTCTAG
TAAGAGTCTCCTGCATAGTAATGGCAACACTTACTTGTATTGGTTCCTGCAGAGGCCAGGCCAGTCTCCTCAGCTCC
TGATATATCGGATGTCCAACCTTGCCTCAGGAGTCCCAGACAGGTTCAGTGGCAGTGGGTCAGGAACTGCTTTCACA
CTGAGAATCAGTAGAGTGGAGGCTGAGGATGTGGGTGTTTATTACTGTATGCAACATCTAGAATATCCTTTCACGTT
CGGCTCGGGGACAAAGTTGGAAATAAAA

### 8D1

The amino acid sequence of VH is as shown in SEQ ID NO. 66, the coding nucleic acid of which is as shown in SEQ ID NO. 67, and the HCDR1, HCDR2, and HCDR3 of which are respectively as shown in SEQ ID NOs. 68, 69, and 70.

QVQLQQPGAELVKPGASVKLSCKASGYTFTSYYMYWVKQRPGQGLEWIGGINPSNGGTKYNEKFKSKATLTVDKSSS
TAFMQLSSLTSEDSAVYYCTRGSLRSYYYAMDYWGQGTSVTVSS

### Nucleic acid sequence

CAGGTCCAACTGCAGCAGCCTGGGGCTGAACTGGTGAAGCCTGGGGCTTCAGTGAAGTTGTCCTGCAAGGCTTCTGG
CTACACCTTCACCAGCTATTATATGTACTGGGTGAAGCAGAGGCCTGGACAAGGCCTTGAGTGGATTGGGGGGATTA
ATCCTAGCAATGGTGGTACTAAGTACAATGAGAAGTTCAAGAGCAAGGCCACACTGACTGTAGACAAATCCTCCAGC
ACAGCCTTCATGCAACTCAGCAGCCTGACATCTGAGGACTCTGCGGTCTATTACTGTACAAGAGGATCACTACGGTC
TTATTACTATGCTATGGACTACTGGGGTCAAGGAACCTCAGTCACCGTCTCCTCA

The amino acid sequence of VL is as shown in SEQ ID NO. 71, the coding nucleic acid of which is as shown in SEQ ID NO. 72, and the LCDR1, LCDR2, and LCDR3 of which are respectively as shown in SEQ ID NOs. 73, 74, and 75.

DIQMTQTTSSLSASLGDRVTISCRASQDISNYLNWYQQKPDGTVKLLIYYTSRLHSGVPSRFSGSGSGTDYSLTISN
LEQEDLATYFCQQGYSLPYTFGGGTKLEIK

### Nucleic acid sequence

GATATCCAGATGACACAGACTACATCCTCCCTGTCTGCCTCTCTGGGAGACAGAGTCACCATCAGTTGCAGGGCAAG
TCAGGACATTAGTAATTATTTAAACTGGTATCAGCAGAAACCAGATGGAACTGTTAAACTCCTGATCTACTACACAT
CAAGATTACATTCAGGAGTCCCATCAAGGTTCAGTGGCAGTGGGTCTGGAACAGATTATTCTCTCACCATTAGCAAC
CTGGAGCAAGAAGATCTTGCCACTTATTTTTGCCAACAGGGTTATTCGCTTCCGTACACGTTCGGAGGGGGGACCAA
GCTGGAAATAAAA

### 10B1

The amino acid sequence of VH is as shown in SEQ ID NO. 76, the coding nucleic acid of which is as shown in SEQ ID NO. 77, and the HCDR1, HCDR2, and HCDR3 of which are respectively as shown in SEQ ID NOs. 241, 78, and 79.

QVQLQQSGAELARPGASVKMSCKASGYIFTSYTMYWVKQRPGQGLEWIGYINPSSGYTNYNQKFKDKATLTADKSSS
TAYMQLSSLTSEDSAVYYCARWGGNERLYYYAMDYWGQGTSVTVSS

### Nucleic acid sequence

CAGGTCCAGCTGCAGCAGTCTGGGGCTGAACTGGCAAGACCTGGGGCCTCAGTGAAGATGTCCTGCAAGGCTTCTGG
CTACATCTTTACTAGCTACACGATGTACTGGGTAAAACAGAGGCCTGGACAGGGTCTGGAATGGATTGGATACATTA
ATCCTAGCAGTGGTTATACTAATTACAATCAGAAGTTCAAGGACAAGGCCACATTGACTGCAGACAAATCCTCCAGC
ACAGCCTACATGCAACTGAGCAGCCTGACATCTGAGGACTCTGCAGTCTATTACTGTGCAAGATGGGGTGGTAACGA
ACGCCTATATTACTATGCTATGGACTACTGGGGTCAAGGAACCTCAGTCACCGTCTCCTCA

The amino acid sequence of VL is as shown in SEQ ID NO. 80, the coding nucleic acid of which is as shown in SEQ ID NO. 81, and the LCDR1, LCDR2, and LCDR3 of which are respectively as shown in SEQ ID NOs. 82, 83, and 84.

QIVLTQSPAIMSASPGEKVTITCSASSSVSYMYWFQQKPGTSPKLWIYGTSNLASGVPARFSGSGSGTSYSLTISRM
EAEDAATYYCQQRSSYPLTFGAGTKLELK

### Nucleic acid sequence

CAAATTGTTCTCACCCAGTCTCCAGCAATCATGTCTGCATCTCCAGGGGAGAAGGTCACCATAACCTGCAGTGCCAG
CTCAAGTGTAAGTTACATGTACTGGTTCCAGCAGAAGCCAGGCACTTCTCCCAAACTCTGGATTTATGGCACATCCA
ACCTGGCTTCTGGAGTCCCTGCTCGCTTCAGTGGCAGTGGATCTGGGACCTCTTACTCTCTCACAATCAGCCGAATG
GAGGCTGAAGATGCTGCCACTTATTACTGCCAGCAGAGGAGCAGTTACCCGCTCACGTTCGGTGCTGGGACCAAGCT
GGAGCTGAAA

### 10B2

The amino acid sequence of VH is as shown in SEQ ID NO. 40, the coding nucleic acid of which is as shown in SEQ ID NO. 41, and the HCDR1, HCDR2, and HCDR3 of which are respectively as shown in SEQ ID NOs. 42, 43, and 44.

QVQLQQSGAELVKPGASVKMSCKAFGYTFTNFPIEWMKQNRGRSLEWIGNFHPYNDVTKYNEKFKGKAKLTVEKSSS
TVYLELSRLTSDDSAVYYCALITRGYWGQGTTLTVSS

### Nucleic acid sequence

CAGGTTCAGCTGCAACAGTCTGGGGCTGAGCTGGTGAAGCCTGGGGCCTCAGTGAAGATGTCCTGCAAGGCTTTTGG
CTACACCTTCACTAACTTTCCAATAGAGTGGATGAAGCAGAATCGTGGGAGGAGCCTAGAGTGGATTGGAAATTTTC
ATCCTTACAATGATGTTACTAAGTACAATGAAAAATTCAAGGGCAAGGCCAAATTGACTGTAGAGAAATCCTCTAGC
ACAGTCTACTTGGAGCTCAGCCGATTAACATCTGATGACTCTGCTGTTTATTACTGTGCCCTTATTACACGGGGCTA
CTGGGGCCAAGGCACCACTCTCACAGTCTCCTCA

The amino acid sequence of VL is as shown in SEQ ID NO. 85, the coding nucleic acid of which is as shown in SEQ ID NO. 86, and the LCDR1, LCDR2, and LCDR3 of which are respectively as shown in SEQ ID NOs. 47, 48, and 49.

DIVMSQSPSSLAVSVGEKVTMSCKSSQSLLYSRNQKNSLAWYQQKPGQSPKLLLYWASTRESGVPDRFTGSGSGTDF
TLTISSVKAEDLAVYYCQQYYSYPLTFGAGTKLELK

### Nucleic acid sequence

GACATTGTGATGTCACAGTCTCCATCCTCCCTAGCTGTGTCAGTTGGAGAGAAGGTTACTATGAGCTGCAAGTCCAG
TCAGAGCCTTTTATATAGTCGCAATCAAAAGAACTCCTTGGCCTGGTACCAGCAGAAACCTGGGCAGTCTCCTAAAC
TGCTGCTTTACTGGGCATCCACTAGGGAATCTGGGGTCCCTGATCGCTTCACAGGCAGTGGATCTGGGACAGATTTC
ACTCTCACCATCAGCAGTGTGAAGGCTGAAGACCTGGCAGTTTATTACTGTCAGCAATATTATAGCTATCCGCTCAC
GTTCGGTGCTGGGACCAAGCTGGAGCTGAAA

### 10E8

The amino acid sequence of VH is as shown in SEQ ID NO. 87, the coding nucleic acid of which is as shown in SEQ ID NO. 88, and the HCDR1, HCDR2, and HCDR3 of which are respectively as shown in SEQ ID NOs. 25, 89, and 90.

EVQLQQSGPELVKPGASMKISCKASGYSFTGYTMNWVKQRHGKNLEWIGLINPYNGGTSYNQKFKGKATLTVDKSSS
TAYMELLSLTSEDSAVYYCARLPIRNGMDYWGQGTSVTVSS

### Nucleic acid sequence

GAGGTCCAGCTGCAGCAGTCTGGACCTGAGCTGGTGAAGCCTGGAGCTTCAATGAAGATATCCTGCAAGGCTTCTGG
TTACTCATTCACTGGCTACACCATGAACTGGGTGAAGCAGAGGCATGGAAAGAACCTTGAGTGGATTGGACTTATTA
ATCCTTACAATGGAGGTACTAGCTATAACCAGAAGTTCAAGGGCAAGGCCACATTAACTGTAGACAAGTCATCCAGC
ACAGCCTACATGGAGCTCCTCAGTCTGACATCTGAGGACTCTGCAGTCTATTACTGTGCAAGATTACCTATCCGAAA
TGGTATGGACTACTGGGGTCAAGGAACCTCAGTCACCGTCTCCTCA

The amino acid sequence of VL is as shown in SEQ ID NO. 91, the coding nucleic acid of which is as shown in SEQ ID NO. 92, and the LCDR1, LCDR2, and LCDR3 of which are respectively as shown in SEQ ID NOs. 93, 94, and 95.

DIVLTQSPATLSVTPGDSVSLSCRASQSISNNLHWYQQKSHESPRLLVKFASLSISGIPSRFSGSGSGTDFTLSINS
VETEDFGMYFCQQSNSWPQTFGGGTKLEIK

### Nucleic acid sequence

GATATTGTGCTAACTCAGTCTCCAGCCACCCTGTCTGTGACTCCAGGAGATAGCGTCAGTCTTTCCTGCAGGGCCAG
CCAAAGTATTAGCAACAACCTACACTGGTATCAACAAAAATCACATGAGTCTCCAAGGCTTCTCGTCAAATTTGCTT
CCCTGTCCATCTCTGGGATCCCCTCCAGGTTCAGTGGCAGTGGATCAGGGACAGATTTCACTCTCAGTATCAACAGT
GTGGAGACTGAAGATTTTGGAATGTATTTCTGTCAACAGAGTAACAGCTGGCCTCAGACGTTCGGTGGAGGCACCAA
GCTGGAAATCAAA

### 12A4

The amino acid sequence of VH is as shown in SEQ ID NO. 96, the coding nucleic acid of which is as shown in SEQ ID NO. 97, and the HCDR1, HCDR2, and HCDR3 of which are respectively as shown in SEQ ID NOs. 98, 99, and 100.

EVQLQQSGPELVKPGASVKMSCKASEYTFTNYVMYWVKQKPGQGLEWIGYINPYNDGTKYNEKFKGKATLTSDKSSS
TAYMELSSLTSEDSAVYYCVRGGMLTTRSLFTYWGQGTLVTVSA

### Nucleic acid sequence

GAGGTCCAGCTGCAGCAGTCTGGACCTGAGCTGGTAAAGCCTGGGGCTTCAGTGAAGATGTCCTGCAAGGCTTCTGA
ATACACATTCACTAATTATGTTATGTATTGGGTGAAGCAGAAGCCTGGGCAGGGCCTTGAGTGGATTGGATATATTA
ATCCTTACAATGATGGTACTAAGTACAATGAGAAGTTCAAAGGCAAGGCCACACTGACTTCAGACAAATCCTCCAGC
ACAGCCTACATGGAGCTCAGCAGCCTGACCTCTGAGGACTCTGCGGTCTATTACTGTGTGAGAGGGGGAATGCTTAC
GACGCGTTCCCTTTTTACTTACTGGGGCCAAGGGACTCTGGTCACTGTCTCTGCA

The amino acid sequence of VL is as shown in SEQ ID NO. 101, the coding nucleic acid of which is as shown in SEQ ID NO. 102, and the LCDR1, LCDR2, and LCDR3 of which are respectively as shown in SEQ ID NOs. 103, 104, and 105.

DIQMTQSPSSLSASLGERVSLTCRASQRISGYLSWLQQKPDGTIKRLIYAASTLDSGVPKRFSGSRSGSDYSLTISS
LESEDFADYYCLQYASYPPTFGGGTKLEIK

### Nucleic acid sequence

GACATCCAGATGACCCAGTCTCCATCCTCCTTATCTGCCTCTCTGGGAGAAAGAGTCAGTCTCACTTGTCGGGCAAG
TCAAAGAATTAGTGGTTACTTAAGCTGGCTTCAGCAAAAACCAGATGGAACTATTAAACGCCTGATCTACGCCGCAT
CCACTTTAGATTCTGGTGTCCCAAAAAGGTTCAGTGGCAGTAGGTCTGGGTCAGATTATTCTCTCACCATCAGCAGC
CTTGAGTCTGAAGATTTTGCAGACTATTACTGTCTACAATATGCTAGTTATCCTCCGACGTTCGGTGGAGGCACCAA
GCTGGAAATCAAA

### 14D1

The amino acid sequence of VH isas shown in SEQ ID NO. 106, the coding nucleic acid of which is as shown in SEQ ID NO. 107, and the HCDR1, HCDR2, and HCDR3 of which are respectively as shown in SEQ ID NOs. 108, 99, and 109.

EVQLQQSGPDLVKPGASVKMSCKASGYKFTSYVMYWVKQKTGQGLEWIGYINPYNDGTKYNEKFKGKATLTSDKSSS
TAYMEFSSLTSEDSAVYYCVRGGMLTTRSLFAYWGQGTLVTVSA

### Nucleic acid sequence

GAGGTCCAGCTGCAGCAGTCTGGACCTGACCTGGTAAAGCCTGGGGCTTCAGTGAAGATGTCCTGCAAGGCTTCTGG
ATACAAATTCACTAGCTATGTTATGTACTGGGTGAAGCAGAAGACTGGGCAGGGCCTTGAGTGGATTGGATATATTA
ATCCTTACAATGATGGTACTAAGTACAATGAGAAGTTCAAAGGCAAGGCCACACTGACTTCAGACAAATCCTCCAGC
ACAGCCTACATGGAGTTCAGCAGCCTGACCTCTGAGGACTCTGCGGTCTATTACTGTGTAAGAGGGGGAATGCTTAC
GACGCGTTCCCTTTTTGCTTACTGGGGCCAAGGGACTCTGGTCACTGTCTCTGCA

The amino acid sequence of VLis as shown in SEQ ID NO. 110, the coding nucleic acid of which is as shown in SEQ ID NO. 111, and the LCDR1, LCDR2, and LCDR3 of which are respectively as shown in SEQ ID NOs. 112, 104, and 105.

DIQMTQSPSSLSASLGERVSLTCRASQEISGYFSWLQQKPDGTIKRLIYAASTLDSGVPKRFSGSRSGSDYSLTISS
LESEDFADYYCLQYASYPPTFGGGTKLEIK

### Nucleic acid sequence

GACATCCAGATGACCCAGTCTCCATCCTCCTTATCTGCCTCTCTGGGAGAAAGAGTCAGTCTCACTTGTCGGGCAAG
TCAGGAAATTAGTGGTTACTTTAGCTGGCTTCAGCAGAAACCAGATGGAACTATTAAACGCCTGATCTACGCCGCAT
CCACTTTAGATTCTGGTGTCCCAAAAAGGTTCAGTGGCAGTAGGTCTGGGTCAGATTATTCTCTCACCATCAGCAGC
CTTGAGTCTGAAGATTTTGCAGACTATTACTGTCTACAATATGCTAGTTATCCTCCGACGTTCGGTGGAGGCACCAA
GCTGGAAATCAAA

### 15A6

The amino acid sequence of VH is as shown in SEQ ID NO. 113, the coding nucleic acid of which is as shown in SEQ ID NO. 114, and the HCDR1, HCDR2, and HCDR3 of which are respectively as shown in SEQ ID NOs. 98, 115, and 116.

EVQLQQSGPELVKPGASVKMSCKASGYTFTNYVMYWVKQKPGQVLEWIGYINPYNDGTKYNEKFTGKATLTSDKSSS
TAYMELSSLTSEDSAVYYCARGGMLTTRSLFVYWGQGTLVTVSA

### Nucleic acid sequence

GAGGTCCAGCTGCAGCAGTCTGGACCTGAGCTGGTAAAGCCTGGGGCTTCAGTGAAGATGTCCTGCAAGGCTTCTGG
ATACACATTCACTAACTATGTTATGTACTGGGTGAAACAGAAGCCTGGGCAGGTCCTTGAATGGATTGGATATATTA
ATCCTTACAATGATGGTACTAAGTACAATGAGAAGTTCACAGGCAAGGCCACACTGACTTCAGACAAATCCTCCAGC
ACAGCCTACATGGAGCTCAGCAGCCTGACCTCTGAGGACTCTGCGGTCTATTACTGTGCAAGAGGGGGAATGCTTAC
GACGCGTTCCCTTTTTGTTTACTGGGGCCAAGGGACTCTGGTCACTGTCTCTGCA

The amino acid sequence of VL is as shown in SEQ ID NO. 110, the coding nucleic acid of which is as shown in SEQ ID NO. 111, and the LCDR1, LCDR2, and LCDR3 of which are respectively as shown in SEQ ID NOs. 112, 104, and 105.

DIQMTQSPSSLSASLGERVSLTCRASQEISGYFSWLQQKPDGTIKRLIYAASTLDSGVPKRFSGSRSGSDYSLTISS
LESEDFADYYCLQYASYPPTFGGGTKLEIK

### Nucleic acid sequence

GACATCCAGATGACCCAGTCTCCATCCTCCTTATCTGCCTCTCTGGGAGAAAGAGTCAGTCTCACTTGTCGGGCAAG
TCAGGAAATTAGTGGTTACTTTAGCTGGCTTCAGCAGAAACCAGATGGAACTATTAAACGCCTGATCTACGCCGCAT
CCACTTTAGATTCTGGTGTCCCAAAAAGGTTCAGTGGCAGTAGGTCTGGGTCAGATTATTCTCTCACCATCAGCAGC
CTTGAGTCTGAAGATTTTGCAGACTATTACTGTCTACAATATGCTAGTTATCCTCCGACGTTCGGTGGAGGCACCAA
GCTGGAAATCAAA

### 15D1

The amino acid sequence of VH is as shown in SEQ ID NO. 117, the coding nucleic acid of which is as shown in SEQ ID NO. 118, and the HCDR1, HCDR2, and HCDR3 of which are respectively as shown in SEQ ID NOs. 119, 99, and 120.

EVQLQQSGPELGKPGASVKMSCKASGYTFTNYVMHWVKQKPGQGLEWIGYINPYNDGTKYNEKFKGKATLTSDKSSN
TAYMELSSLTSEDSAVYYCARGGVRRYFDYWGQGTTLTVSS

### Nucleic acid sequence

GAGGTCCAGCTGCAGCAGTCTGGACCTGAGCTGGGAAAGCCTGGGGCTTCAGTGAAGATGTCCTGCAAGGCTTCTGG
ATACACATTCACTAACTATGTTATGCACTGGGTGAAGCAGAAGCCTGGGCAGGGCCTTGAGTGGATTGGATATATTA
ATCCTTACAATGATGGTACTAAGTACAATGAGAAGTTCAAAGGCAAGGCCACACTGACTTCAGACAAATCCTCCAAC
ACAGCCTACATGGAGCTCAGCAGCCTGACCTCTGAGGACTCTGCGGTCTATTACTGTGCAAGAGGAGGGGTACGACG
CTACTTTGACTACTGGGGCCAAGGCACCACTCTCACAGTCTCCTCA

The amino acid sequence of VL is as shown in SEQ ID NO. 121, the coding nucleic acid of which is as shown in SEQ ID NO. 122, and the LCDR1, LCDR2, and LCDR3 of which are respectively as shown in SEQ ID NOs. 123, 124, and 125.

DIQMTQSPSSLSASLGERVSLTCRASQDIGSNLNWLQQEPDGTIKRLIYATSSLDSGVPKRFSGSRSGSDYSLTISS
LESEDFVDYYCLQYASFPYTFGGGTKLEIK

### Nucleic acid sequence

GACATCCAGATGACCCAGTCTCCATCCTCCTTATCTGCCTCTCTGGGAGAAAGAGTCAGTCTCACTTGTCGGGCAAG
TCAGGACATTGGTAGTAACTTAAACTGGCTTCAGCAGGAACCAGATGGAACTATTAAACGCCTGATCTACGCCACAT
CCAGTTTAGATTCTGGTGTCCCCAAAAGGTTCAGTGGCAGTAGGTCTGGGTCAGATTATTCTCTCACCATCAGCAGC
CTTGAGTCTGAAGATTTTGTAGACTATTACTGTCTACAATATGCTAGTTTTCCGTACACGTTCGGAGGGGGGACCAA
GCTGGAAATAAAA

### 15H5

The amino acid sequence of VH is as shown in SEQ ID NO. 126, the coding nucleic acid of which is as shown in SEQ ID NO. 127, and the HCDR1, HCDR2, and HCDR3 of which are respectively as shown in SEQ ID NOs. 108, 99, and 116.

EVQLQQSGPELVKPGASVKMSCMASGYTFTSYVMYWVKQKPGQGLEWIGYINPYNDGTKYNEKFKGKATLTSDKSSS
TAYMELSSLTSEDSAVYYCARGGMLTTRSLFVYWGHGTLVTVSA

### Nucleic acid sequence

GAGGTCCAGCTGCAGCAGTCTGGACCTGAACTGGTAAAGCCTGGGGCTTCAGTGAAGATGTCCTGCATGGCTTCTGG
ATACACATTCACTAGCTATGTTATGTACTGGGTGAAGCAGAAGCCTGGGCAGGGCCTTGAGTGGATTGGATATATTA
ATCCTTACAATGATGGTACTAAGTACAATGAGAAGTTCAAAGGCAAGGCCACACTGACTTCAGACAAATCCTCCAGC
ACAGCCTACATGGAGCTCAGCAGCCTGACCTCTGAGGACTCTGCGGTCTATTACTGTGCAAGAGGGGGAATGCTTAC
GACGCGTTCCCTTTTTGTTTACTGGGGCCACGGGACTCTGGTCACTGTCTCTGCA

The amino acid sequence of VL is as shown in SEQ ID NO. 128, the coding nucleic acid of which is as shown in SEQ ID NO. 129, and the LCDR1, LCDR2, and LCDR3 of which are respectively as shown in SEQ ID NOs. 130, 104, and 105.

DIQMTQSPSSLSASLGERVSLTCRASQEISGYLSWLQQKPDGTIKRLIYAASTLDSGVPKRFSGSRSGSDYSLTISS
LESEDFADYYCLQYASYPPTYGGGTKLEIK

### Nucleic acid sequence

GACATCCAGATGACCCAGTCTCCATCCTCCTTATCTGCCTCTCTGGGAGAAAGAGTCAGTCTCACTTGTCGGGCAAG
TCAGGAAATTAGTGGTTACTTAAGCTGGCTTCAGCAGAAACCAGATGGAACTATTAAACGCCTGATCTACGCCGCAT
CCACTTTAGATTCTGGTGTCCCAAAAAGGTTCAGTGGCAGTAGGTCTGGGTCAGATTATTCTCTCACCATCAGCAGC
CTTGAGTCTGAAGATTTTGCAGACTATTACTGTCTACAATATGCTAGTTATCCTCCGACGTACGGTGGAGGCACCAA
GCTGGAAATCAAA

### 17D12

The amino acid sequence of VH is as shown in SEQ ID NO. 131, the coding nucleic acid of which is as shown in SEQ ID NO. 132, and the HCDR1, HCDR2, and HCDR3 of which are respectively as shown in SEQ ID NOs. 108, 133, and 134.

EVQLQQSGPELVKPGASVKMSCKASGYTFTSYVMYWVKQKPGQVLEWIGYINPYNDGTKYNERFTGKATLTSDKSSS
TAYMELSSLTSEDSAVYYCARGGMLTTRSLFLYWGQGTLVTVSA

### Nucleic acid sequence

GAGGTCCAGCTGCAGCAGTCTGGACCTGAGCTGGTAAAGCCTGGGGCTTCAGTGAAGATGTCCTGCAAGGCTTCTGG
ATACACATTCACTAGTTATGTTATGTACTGGGTGAAACAGAAGCCTGGGCAGGTCCTTGAATGGATTGGATATATTA
ATCCTTACAATGATGGTACTAAGTACAATGAGAGGTTCACAGGCAAGGCCACACTGACTTCAGACAAATCCTCCAGC
ACAGCCTACATGGAGCTCAGCAGCCTGACCTCTGAGGACTCTGCGGTCTATTACTGTGCAAGAGGGGGAATGCTTAC
GACGCGTTCCCTTTTTCTTTACTGGGGCCAAGGGACTCTGGTCACTGTCTCTGCA

The amino acid sequence of VL is as shown in SEQ ID NO. 135, the coding nucleic acid of which is as shown in SEQ ID NO. 136, and the LCDR1, LCDR2, and LCDR3 of which are respectively as shown in SEQ ID NOs. 112, 104, and 105.

DIVMSQSPSSLSASLGERVSLTCRASQEISGYFSWLQQKPDGTIKRLIYAASTLDSGVPKRFSGSRSGSDYSLTISS
LESEDFADYYCLQYASYPPTFGGGTKLEIK

### Nucleic acid sequence

GACATTGTGATGTCACAGTCTCCATCCTCCTTATCTGCCTCTCTGGGAGAAAGAGTCAGTCTCACTTGTCGGGCAAG
TCAGGAAATTAGTGGTTACTTTAGCTGGCTTCAGCAGAAACCAGATGGAACTATTAAACGCCTGATCTACGCCGCAT
CCACTTTAGATTCTGGTGTCCCAAAAAGGTTCAGTGGCAGTAGGTCTGGGTCAGATTATTCTCTCACCATCAGCAGC
CTTGAGTCTGAAGATTTTGCAGACTATTACTGTCTACAATATGCTAGTTATCCTCCGACGTTCGGTGGAGGCACCAA
GCTGGAAATAAAA

### 18C3

The amino acid sequence of VH is as shown in SEQ ID NO. 137, the coding nucleic acid of which is as shown in SEQ ID NO. 138, and the HCDR1, HCDR2, and HCDR3 of which are respectively as shown in SEQ ID NOs. 58, 59, and 139.

EVQLVESGGDLVKPGGSLKLSCAASGFTFSSYGMSWVRQTPDKRLEWVATISSGGSYTYYPDSVKGRFTISRDNAKN
TLYLQMSSLKSEDTAMYYCARHEARATFDYWGQGTTLTVSS

### Nucleic acid sequence

GAGGTGCAGCTGGTGGAGTCTGGGGGAGACTTAGTGAAGCCTGGAGGGTCCCTGAAACTCTCCTGTGCAGCCTCTGG
ATTCACTTTCAGTAGCTATGGCATGTCTTGGGTTCGCCAGACTCCAGACAAGAGGCTGGAGTGGGTCGCAACCATTA
GTAGTGGTGGTAGTTACACCTACTATCCAGACAGTGTGAAGGGGCGATTCACCATCTCCAGAGACAATGCCAAGAAC
ACCCTGTACCTGCAAATGAGCAGTCTGAAGTCTGAGGACACAGCCATGTATTACTGTGCAAGACATGAGGCTCGGGC
TACCTTTGACTACTGGGGCCAAGGCACCACTCTCACAGTCTCCTCA

The amino acid sequence of VL is as shown in SEQ ID NO. 61, the coding nucleic acid of which is as shown in SEQ ID NO. 62, and the LCDR1, LCDR2, and LCDR3 of which are respectively as shown in SEQ ID NOs. 63, 64, and 65.

DIVMTQAAPSVPVTPGESVSISCRSSKSLLHSNGNTYLYWFLQRPGQSPQLLIYRMSNLASGVPDRFSGSGSGTAFT

LRISRVEAEDVGVYYCMQHLEYPFTFGSGTKLEIK

### Nucleic acid sequence

GATATTGTGATGACTCAGGCTGCACCCTCTGTACCTGTCACTCCTGGAGAGTCAGTATCCATCTCCTGCAGGTCTAG
TAAGAGTCTCCTGCATAGTAATGGCAACACTTACTTGTATTGGTTCCTGCAGAGGCCAGGCCAGTCTCCTCAGCTCC
TGATATATCGGATGTCCAACCTTGCCTCAGGAGTCCCAGACAGGTTCAGTGGCAGTGGGTCAGGAACTGCTTTCACA
CTGAGAATCAGTAGAGTGGAGGCTGAGGATGTGGGTGTTTATTACTGTATGCAACATCTAGAATATCCTTTCACGTT
CGGCTCGGGGACAAAGTTGGAAATAAAA

### 18E7

The amino acid sequence of VH is as shown in SEQ ID NO. 140, the coding nucleic acid of which is as shown in SEQ ID NO. 141, and the HCDR1, HCDR2, and HCDR3 of which are respectively as shown in SEQ ID NOs. 98, 99, and 109.

EVQLQQSGPELVKPGASVKMSCKASEYTFTNYVMYWVKQKPGQGLEWIGYINPYNDGTKYNEKFKGKATLTSDKSSS
TAYMELSSLTSEDSAVYYCVRGGMLTTRSLFAYWGQGTLVTVSA

### Nucleic acid sequence

GAGGTCCAGCTGCAGCAGTCTGGACCTGAGCTGGTAAAGCCTGGGGCTTCAGTGAAGATGTCCTGCAAGGCTTCTGA
ATACACATTCACTAATTATGTTATGTATTGGGTGAAGCAGAAGCCTGGGCAGGGCCTTGAGTGGATTGGATATATTA
ATCCTTACAATGATGGTACTAAGTACAATGAGAAGTTCAAAGGCAAGGCCACACTGACTTCAGACAAATCCTCCAGC
ACAGCCTACATGGAGCTCAGCAGCCTGACCTCTGAGGACTCTGCGGTCTATTACTGTGTGAGAGGGGGAATGCTTAC
GACGCGTTCCCTTTTTGCTTACTGGGGCCAAGGGACTCTGGTCACTGTCTCTGCA

The amino acid sequence of VL is as shown in SEQ ID NO. 101, the coding nucleic acid of which is as shown in SEQ ID NO. 102, and the LCDR1, LCDR2, and LCDR3 of which are respectively as shown in SEQ ID NOs. 103, 104, and 105.

DIQMTQSPSSLSASLGERVSLTCRASQRISGYLSWLQQKPDGTIKRLIYAASTLDSGVPKRFSGSRSGSDYSLTISS
LESEDFADYYCLQYASYPPTFGGGTKLEIK

### Nucleic acid sequence

GACATCCAGATGACCCAGTCTCCATCCTCCTTATCTGCCTCTCTGGGAGAAAGAGTCAGTCTCACTTGTCGGGCAAG
TCAAAGAATTAGTGGTTACTTAAGCTGGCTTCAGCAAAAACCAGATGGAACTATTAAACGCCTGATCTACGCCGCAT
CCACTTTAGATTCTGGTGTCCCAAAAAGGTTCAGTGGCAGTAGGTCTGGGTCAGATTATTCTCTCACCATCAGCAGC
CTTGAGTCTGAAGATTTTGCAGACTATTACTGTCTACAATATGCTAGTTATCCTCCGACGTTCGGTGGAGGCACCAA
GCTGGAAATCAAA

### 19E2

The amino acid sequence of VH is as shown in SEQ ID NO. 140, the coding nucleic acid of which is as shown in SEQ ID NO. 141, and the HCDR1, HCDR2, and HCDR3 of which are respectively as shown in SEQ ID NOs. 98, 99, and 109.

EVQLQQSGPELVKPGASVKMSCKASEYTFTNYVMYWVKQKPGQGLEWIGYINPYNDGTKYNEKFKGKATLTSDKSSS
TAYMELSSLTSEDSAVYYCVRGGMLTTRSLFAYWGQGTLVTVSA

### Nucleic acid sequence

GAGGTCCAGCTGCAGCAGTCTGGACCTGAGCTGGTAAAGCCTGGGGCTTCAGTGAAGATGTCCTGCAAGGCTTCTGA
ATACACATTCACTAATTATGTTATGTATTGGGTGAAGCAGAAGCCTGGGCAGGGCCTTGAGTGGATTGGATATATTA
ATCCTTACAATGATGGTACTAAGTACAATGAGAAGTTCAAAGGCAAGGCCACACTGACTTCAGACAAATCCTCCAGC
ACAGCCTACATGGAGCTCAGCAGCCTGACCTCTGAGGACTCTGCGGTCTATTACTGTGTGAGAGGGGGAATGCTTAC
GACGCGTTCCCTTTTTGCTTACTGGGGCCAAGGGACTCTGGTCACTGTCTCTGCA

The amino acid sequence of VL is as shown in SEQ ID NO. 142, the coding nucleic acid of which is as shown in SEQ ID NO. 143, and the LCDR1, LCDR2, and LCDR3 of which are respectively as shown in SEQ ID NOs. 103, 104, and 144.

DIQMTQSPSSLSASLGERVSLTCRASQRISGYLSWLQQKPDGTIKRLIYAASTLDSGVPKRFSGSRSGSDYSLTI
SSLESEDFADYYCLQYANYPPTFGGGTKLEIK

### Nucleic acid sequence

GACATCCAGATGACCCAGTCTCCATCCTCCTTATCTGCCTCTCTGGGAGAAAGAGTCAGTCTCACTTGTCGGGCAAG
TCAAAGAATTAGTGGTTACTTAAGCTGGCTTCAGCAAAAACCAGATGGAACTATTAAACGCCTGATCTACGCCGCAT
CCACTTTAGATTCTGGTGTCCCAAAAAGGTTCAGCGGCAGTAGGTCTGGGTCAGATTATTCTCTCACCATCAGCAGC
CTTGAGTCTGAAGATTTTGCAGACTATTACTGTCTACAATATGCTAATTATCCTCCGACGTTCGGTGGAGGCACCAA
GCTGGAAATCAAA

### 22C1

The amino acid sequence of VH is as shown in SEQ ID NO. 145, the coding nucleic acid of which is as shown in SEQ ID NO. 146, and the HCDR1, HCDR2, and HCDR3 of which are respectively as shown in SEQ ID NOs. 25, 147, and 148.

EVQLQQSGPELVKPGTSMKISCKASGYSFTGYTMNWVKQSHGKNLEWIGLINPYNGGSNYNLKFKGKATLTVDKSSN
TAYMELLSLTSEDSAVYYCARWGLRNAMDYWGQGTSVTVSS

### Nucleic acid sequence

GAGGTCCAGCTGCAGCAGTCTGGACCTGAGCTGGTGAAGCCTGGAACTTCAATGAAGATATCCTGCAAGGCTTCTGG
TTACTCATTCACTGGCTACACCATGAACTGGGTGAAGCAGAGCCATGGAAAGAACCTTGAGTGGATTGGACTTATTA
ATCCTTACAATGGTGGTTCTAACTACAACCTGAAGTTCAAGGGCAAGGCCACATTAACTGTAGACAAGTCATCCAAC
ACAGCCTACATGGAACTCCTCAGTCTGACATCTGAGGACTCTGCAGTCTATTACTGTGCAAGATGGGGATTACGGAA
TGCTATGGACTACTGGGGTCAAGGAACCTCAGTCACCGTCTCCTCA

The amino acid sequence of VL is as shown in SEQ ID NO. 149, the coding nucleic acid of which is as shown in SEQ ID NO. 150, and the LCDR1, LCDR2, and LCDR3 of which are respectively as shown in SEQ ID NOs. 93, 151, and 95.

DIVVTQSPATLSVTPGDSVSLSCRASQSISNNLHWFQQKSHESPRLLIKYASQSISGIPSRFSGSGSGTDFTLSINS
VETEDFGMYFCQQSNSWPQTFGGGTKLEIK

### Nucleic acid sequence

GATATTGTGgTAACTCAGTCTCCAGCCACCCTGTCTGTGACTCCAGGAGATAGCGTCAGTCTTTCCTGCAGGGCCAG
TCAAAGTATTAGCAACAACCTACACTGGTTTCAACAAAAATCACATGAGTCTCCAAGGCTTCTCATCAAGTATGCTT
CCCAGTCCATCTCTGGGATCCCCTCCAGGTTCAGTGGCAGTGGATCAGGGACAGATTTCACTCTCAGTATCAACAGT
GTGGAGACTGAAGATTTTGGAATGTATTTCTGTCAACAGAGTAACAGTTGGCCTCAGACGTTCGGTGGAGGCACCAA
GCTGGAAATCAAA

### Example 3. Construction of GPRC5D×CD3κλ bispecific antibody

Clones 2E1, 2F1, 4B1, 4D3, 8D1 and 15D1 with high affinity in binding GPRC5D were used, and their CDRs were transplanted into the genes of human germline gene with high homology, and constructed into bispecific antibodies with humanized anti-CD3e antibody VH9VL5. The Fc part of the bispecific antibody had a human IgG4 knob-into-hole structure to realize heterodimer pairing (Atwell et al., Stable Heterodimers from Remodeling the Domain Interface of a Homodimer using a Phage Display Library, J. Mol. Biol. (1997) 270). By mutations Ser228Pro, Leu235Glu, and Pro329Ala, the hinge region was kept stable, and the interaction with the Fcγ receptor and C1q was completely removed.

The sequence information of humanized anti-CD3e antibody VH9VL5 is shown below:
The amino acid sequence of VL is as shown in SEQ ID NO. 152, the coding nucleic acid of which is as shown in SEQ ID NO. 153, and the LCDR1, LCDR2, and LCDR3 of which are respectively as shown in SEQ ID NOs. 154, 155, and 156.

QAVVTQEPSLTVSPGGTVTLTCRSSTGAVTTSNYANWVQEKPGQAPRGLIGGTNKRAPWTPARFSGSLLGGKAALTI
TGAQAEDEAEYYCVLWYSNLWVFGGGTKLTVL

### Nucleic acid sequence

CAGGCTGTGGTCACACAAGAGCCTAGCCTGACAGTGTCTCCTGGCGGCACAGTGACCCTGACCTGTAGATCTTCTAC
AGGCGCCGTGACCACCAGCAACTACGCTAATTGGGTGCAGgAGAAGCCCGGCCAGGCTCCTAGAGGACTGATCGGCG
GAACAAACAAGAGAGCCCCTTGGACACCCGCCAGATTCTCTGGATCTCTGCTCGGCGGAAAGGCCGCTCTGACAATC
ACTGGTGCTCAGGCTGAGGACGAGGCCGAGTACTATTGTGTGCTGTGGTACAGCAACCTGTGGGTGTTCGGCGGAGG
CACCAAACTGACAGTTCTG

The amino acid sequence of VH isas shown in SEQ ID NO. 157, the coding nucleic acid of which is as shown in SEQ ID NO. 158, and the HCDR1, HCDR2, and HCDR3 of which are respectively as shown in SEQ ID NOs. 159, 160, and 161.

EVQLVESGGGLVQPGGSLRLSCAASGFTFNTYAMNWVRKAPGKGLEWVGRIRSKYNNYATYYADSVKDRFTISRDDS
KNSLYLQMNSLKTEDTAVYYCVRHGNFGNSYVSWFAYWGQGTLVTVSS

### Nucleic acid sequence

GAGGTGCAGCTGGTTGAATCTGGCGGAGGACTGGTTCAGCCTGGCGGATCTCTGAGACTGTCTTGTGCCGCCAGCGG
CTTCACCTTCAACACCTACGCTATGAACTGGGTCCGAAAGGCCCCTGGCAAAGGACTGGAATGGGTGGGAAGAATCA
GGTCCAAGTACAACAACTACGCCACCTACTACGCCGACAGCGTGAAGGACAGATTCACCATCAGCAGGGACGACAGC
AAGAACAGCCTGTACCTGCAGATGAACAGCCTGAAAACCGAGGACACCGCCGTGTACTACTGTGTCAGACACGGCAA
CTTCGGCAACAGCTATGTGTCTTGGTTTGCCTACTGGGGCCAGGGCACACTGGTCACAGTTAGCTCT

**Table 3. Variable region sequence of humanized GPRC5D antibody**

| GPRC5D Humanized antibody | HCVR | | HCDR1 | HCDR2 | HCDR3 | LCVR | | LCDR1 | LCDR2 | LCDR3 |
|---|---|---|---|---|---|---|---|---|---|---|
| | Amino acid sequence | Nucleotide sequence | Amino acid sequence | Amino acid sequence | Amino acid sequence | Amino acid sequence | Nucleotide sequence | Amino acid sequence | Amino acid sequence | Amino acid sequence |
| 2E1 | 162 | 163 | 15 | 164 | 17 | 165 | 166 | 167 | 21 | 22 |
| 2F1 | 168 | 169 | 25 | 170 | 27 | 171 | 172 | 173 | 31 | 32 |
| 4B1 | 174 | 175 | 5 | 176 | 7 | 177 | 178 | 179 | 21 | 39 |
| 4D3 | 180 | 181 | 42 | 182 | 44 | 183 | 184 | 47 | 48 | 49 |
| 8D1 | 185 | 186 | 68 | 187 | 70 | 188 | 189 | 190 | 74 | 75 |
| 15D1 | 191 | 192 | 119 | 193 | 120 | 194 | 195 | 123 | 124 | 125 |

### Humanized antibody 2E1

The amino acid sequence of VH is as shown in SEQ ID NO. 162, the coding nucleic acid of which is as shown in SEQ ID NO. 163, and the HCDR1, HCDR2, and HCDR3 of which are respectively as shown in SEQ ID NOs. 15, 164, and 17.

QVQLVQSGAEVKKPGSSVKVSCKASGYTFTRYWMHWVREAPGQGLEWMGEFNPSNGRINYNEKFQGRVTLTIDKSSS
TAYMELSSLRSEDTAVYYCTRGFAYWGQGTLVTVSS

### Nucleic acid sequence

CAGGTTCAGCTGGTTCAGTCTGGCGCCGAAGTGAAGAAACCTGGCAGCAGCGTGAAGGTGTCCTGCAAGGCTAGCGG
CTACACATTCACCAGATACTGGATGCACTGGGTCCGAGAGGCCCCTGGACAAGGACTTGAGTGGATGGGCGAGTTCA
ACCCCAGCAACGGCAGAATCAACTACAACGAGAAGTTCCAGGGCAGAGTGACCCTGACCATCGACAAGAGCAGCAGC
ACCGCCTACATGGAACTGAGCAGCCTGAGAAGCGAGGACACCGCCGTGTACTACTGCACAAGAGGCTTCGCTTACTG
GGGCCAGGGAACCCTGGTTACCGTTAGCTCT

The amino acid sequence of VL is as shown in SEQ ID NO. 165, the coding nucleic acid of which is as shown in SEQ ID NO. 166, and the LCDR1, LCDR2, and LCDR3 of which are respectively as shown in SEQ ID NOs. 167, 21, and 22.

EIVLTQSPATLSLSPGERATLSCRASSTVSYIHWYQKKPGQAPRRLIYDTSKLASGIPARFSGSGSGTDYTLTISSL
EPEDFAVYYCQQWTTNPWTFGGGTKVEIK

### Nucleic acid sequence

GAGATCGTGCTGACACAGAGCCCTGCCACACTGTCACTGTCTCCAGGCGAGAGAGCCACACTGAGCTGTAGAGCCAG
CAGCACCGTGTCCTACATCCACTGGTATCAGAAGAAGCCCGGCCAGGCTCCTAGAAGGCTGATCTACGACACAAGCA
AGCTGGCCTCTGGCATCCCCGCTAGATTTTCTGGCAGCGGCAGCGGAACCGACTACACCCTGACAATCAGCAGCCTG
GAACCTGAGGACTTCGCCGTGTACTACTGCCAGCAGTGGACCACCAATCCTTGGACATTCGGCGGAGGCACCAAGGT
GGAAATCAAG

### Humanized antibody 2F1

The amino acid sequence of VH is as shown in SEQ ID NO. 168, the coding nucleic acid of which is as shown in SEQ ID NO. 169, and the HCDR1, HCDR2, and HCDR3 of which are respectively as shown in SEQ ID NOs. 25, 170, and 27.

QVQLVQSGAEVKKPGSSVKVSCKASGYSFTGYTMNWVREAPGQGLEWMGLINPYNGGTRYNQKFQGRVTLTVDKSSS
TAYMELSSLRSEDTAVYYCARWALRYGMDYWGQGTLVTVSS

### Nucleic acid sequence

CAGGTTCAGCTGGTTCAGTCTGGCGCCGAAGTGAAGAAACCTGGCAGCAGCGTGAAGGTGTCCTGCAAGGCTAGCGG
CTACAGCTTCACCGGCTACACCATGAACTGGGTCCGAGAGGCTCCTGGACAGGGACTCGAATGGATGGGCCTGATCA
ACCCTTACAACGGCGGCACCAGATACAACCAGAAATTCCAGGGCAGAGTGACCCTGACCGTGGACAAGTCTAGCAGC
ACCGCCTACATGGAACTGAGCAGCCTGAGAAGCGAGGACACCGCCGTGTACTACTGTGCCAGATGGGCTCTGAGATA
CGGCATGGACTACTGGGGCCAGGGAACCCTGGTTACCGTTAGCTCT

The amino acid sequence of VL is as shown in SEQ ID NO. 171, the coding nucleic acid of which is as shown in SEQ ID NO. 172, and the LCDR1, LCDR2, and LCDR3 of which are respectively as shown in SEQ ID NOs. 173, 31, and 32.

DIQMTQSPSSLSASVGDRVTITCRASQNVGTNVAWYQKKPGKAPKALIYSASYRNSGVPSRFSGSGSGTDFTLTISS
LQPEDFATYFCQQYNSYLLTFGGGTKVEIK

### Nucleic acid sequence

GACATCCAGATGACACAGAGCCCTAGCAGCCTGTCTGCCAGCGTGGGAGACAGAGTGACCATCACCTGTAGAGCCAG
CCAGAACGTGGGCACAAACGTGGCCTGGTATCAGAAGAAGCCTGGCAAGGCCCCTAAGGCTCTGATCTACAGCGCCA
GCTACAGAAACAGCGGCGTGCCCTCTAGATTCAGCGGCTCTGGCTCTGGCACCGACTTCACCCTGACAATCTCTAGC
CTGCAGCCTGAGGACTTCGCTACCTACTTCTGCCAGCAGTACAACAGCTACCTGCTGACCTTCGGCGGAGGCACCAA
GGTGGAAATCAAG

### Humanized antibody 4B1

The amino acid sequence of VH is as shown in SEQ ID NO. 174, the coding nucleic acid of which is as shown in SEQ ID NO. 175, and the HCDR1, HCDR2, and HCDR3 of which are respectively as shown in SEQ ID NOs. 5, 176, and 7.

QAQLVQSGAEVKKPGSSVKVSCKASGYTFTSYNMHWVREAPGQGLEWMGYIYPGNGGTNYNQKFQGRVTLTADTSSS
TAYMELSSLRSEDTAVYFCARGYGYRYWYFDVWGQGTLVTVSS

### Nucleic acid sequence

CAGGCTCAGCTGGTTCAGTCTGGCGCCGAAGTGAAGAAACCTGGCAGCAGCGTGAAGGTGTCCTGCAAGGCTAGCGG
CTACACATTCACCAGCTACAACATGCACTGGGTCCGAGAGGCCCCTGGACAGGGACTTGAGTGGATGGGCTACATCT
ACCCTGGCAACGGCGGCACCAACTACAACCAGAAATTCCAGGGCAGAGTGACCCTGACCGCCGACACATCTAGCAGC
ACCGCCTACATGGAACTGAGCAGCCTGAGAAGCGAGGATACCGCCGTGTACTTCTGTGCCAGAGGCTACGGATACCG
GTACTGGTACTTCGACGTGTGGGGCCAGGGAACCCTGGTCACAGTTTCTTCT

The amino acid sequence of VL is as shown in SEQ ID NO. 177, the coding nucleic acid of which is as shown in SEQ ID NO. 178, and the LCDR1, LCDR2, and LCDR3 of which are respectively as shown in SEQ ID NOs. 179, 21, and 39.

EIVLTQSPATLSLSPGERATLSCRASSSVSYMHWYQKKPGQAPRRLIYDTSKLASGIPARFSGSGSGTDYTLTISSL
EPEDFAVYYCQQWSNYPPTFGGGTKVEIK

### Nucleic acid sequence

GAGATCGTGCTGACACAGAGCCCTGCCACACTGTCACTGTCTCCAGGCGAGAGAGCCACACTGAGCTGTAGAGCCAG
CAGCAGCGTGTCCTACATGCACTGGTATCAGAAGAAGCCCGGCCAGGCTCCTAGAAGGCTGATCTACGACACAAGCA
AGCTGGCCTCTGGCATCCCCGCTAGATTTTCTGGCAGCGGCAGCGGAACCGACTACACCCTGACAATCAGCAGCCTG
GAACCTGAGGACTTCGCCGTGTACTACTGCCAGCAGTGGTCTAACTACCCTCCTACCTTCGGCGGAGGCACCAAGGT
GGAAATCAAG

### Humanized antibody 4D3

The amino acid sequence of VH is as shown in SEQ ID NO. 180, the coding nucleic acid of which is as shown in SEQ ID NO. 181, and the HCDR1, HCDR2, and HCDR3 of which are respectively as shown in SEQ ID NOs. 42, 182, and 44.

QVQLVQSGAEVKKPGSSVKVSCKASGYTFTNFPIEWMREAPGQGLEWMGNFHPYNDVTKYNEKFQGRVTLTVDKSSS
TVYMELSSLRSEDTAVYYCALITRGYWGQGTLVTVSS

### Nucleic acid sequence

CAGGTTCAGCTGGTTCAGTCTGGCGCCGAAGTGAAGAAACCTGGCAGCAGCGTGAAGGTGTCCTGCAAGGCTAGCGG
CTACACATTCACAAACTTCCCCATCGAGTGGATGAGAGAGGCCCCTGGACAGGGCCTTGAGTGGATGGGAAACTTTC
ACCCCTACAACGACGTGACCAAGTACAACGAGAAGTTCCAGGGCAGAGTGACCCTGACCGTGGACAAGAGCAGCAGC
ACCGTGTACATGGAACTGAGCAGCCTGAGAAGCGAGGACACCGCCGTGTACTACTGTGCCCTGATCACCAGAGGCTA
CTGGGGCCAGGGAACACTGGTCACAGTCAGCTCT

The amino acid sequence of VL is as shown in SEQ ID NO. 183, the coding nucleic acid of which is as shown in SEQ ID NO. 184, and the LCDR1, LCDR2, and LCDR3 of which are respectively as shown in SEQ ID NOs. 47, 48, and 49.

DIVMTQSPDSLAVSLGERATINCKSSQSLLYSRNQKNSLAWYQKKPGQPPKLLIYWASTRESGVPDRFSGSGSGTDF
TLTISSLQAEDVAVYYCQQYYSYPLTFGQGTKLEIK

### Nucleic acid sequence

GACATCGTGATGACACAGAGCCCTGATAGCCTGGCCGTGTCTCTGGGAGAGAGAGCCACCATCAACTGCAAGAGCAG
CCAGAGCCTGCTGTACTCCAGAAACCAGAAGAACAGCCTGGCCTGGTATCAGAAGAAGCCCGGCCAGCCTCCTAAGC
TGCTGATCTACTGGGCCAGCACCAGAGAAAGCGGCGTGCCAGATAGATTCAGCGGCAGCGGCTCTGGAACCGACTTC
ACCCTGACAATCAGCTCCCTGCAGGCCGAGGATGTGGCCGTGTACTACTGCCAGCAGTACTACAGCTACCCTCTGAC
CTTCGGCCAGGGCACCAAGCTGGAAATCAAG

### Humanized antibody 8D1

The amino acid sequence of VH is as shown in SEQ ID NO. 185, the coding nucleic acid of which is as shown in SEQ ID NO. 186, and the HCDR1, HCDR2, and HCDR3 of which are respectively as shown in SEQ ID NOs. 68, 187, and 70.

QVQLVQSGAEVKKPGSSVKVSCKASGYTFTSYYMYWVREAPGQGLEWMGGINPSNGGTKYNEKFQGRVTLTVDKSSS
TAYMELSSLRSEDTAVYYCTRGSLRSYYYAMDYWGQGTLVTVSS

### Nucleic acid sequence

CAGGTTCAGCTGGTTCAGTCTGGCGCCGAAGTGAAGAAACCTGGCAGCAGCGTGAAGGTGTCCTGCAAGGCTAGCGG
CTACACATTCACCAGCTACTACATGTACTGGGTCCGAGAGGCTCCTGGACAGGGACTCGAATGGATGGGCGGCATCA
ACCCTTCTAACGGCGGCACCAAGTACAACGAGAAGTTCCAGGGCAGAGTGACCCTGACCGTGGACAAGTCTAGCAGC
ACCGCCTACATGGAACTGAGCAGCCTGAGAAGCGAGGACACCGCCGTGTACTACTGTACCAGAGGCTCCCTGAGATC
CTACTACTACGCCATGGACTACTGGGGCCAGGGCACACTGGTTACCGTTAGCTCT

The amino acid sequence of VL is as shown in SEQ ID NO. 188, the coding nucleic acid of which is as shown in SEQ ID NO. 189, and the LCDR1, LCDR2, and LCDR3 of which are respectively as shown in SEQ ID NOs. 190, 74, and 75.

DIQMTQSPSSLSASVGDRVTITCQASQDISNYLNWYQKKPGKAPKLLIYYTSRLHSGVPSRFSGSGSGTDYTFTISS
LQPEDIATYFCQQGYSLPYTFGQGTKLEIK

### Nucleic acid sequence

GACATCCAGATGACACAGAGCCCTAGCAGCCTGTCTGCCAGCGTGGGAGACAGAGTGACCATCACCTGTCAGGCCAG
CCAGGACATCAGCAACTACCTGAACTGGTATCAGAAGAAGCCCGGCAAGGCCCCTAAGCTGCTGATCTACTACACCA
GCAGACTGCACAGCGGCGTGCCCTCTAGATTTTCTGGCAGCGGCTCTGGCACCGACTACACCTTCACAATCAGCAGC
CTGCAGCCTGAGGATATCGCTACCTACTTCTGCCAGCAAGGCTACAGCCTGCCTTACACATTCGGCCAGGGCACCAA
GCTGGAAATCAAG

### Humanized antibody 15D1

The amino acid sequence of VH is as shown in SEQ ID NO. 191, the coding nucleic acid of which is as shown in SEQ ID NO. 192, and the HCDR1, HCDR2, and HCDR3 of which are respectively as shown in SEQ ID NOs. 119, 193, and 120.

QVQLVQSGAEVKKPGSSVKVSCKASGYTFTNYVMHWVKEKPGQGLEWMGYINPYNDGTKYNEKFQGRVTLTSDKSSN
TAYMELSSLRSEDTAVYYCARGGVRRYFDYWGQGTLVTVSS

### Nucleic acid sequence

CAGGTTCAGCTGGTTCAGTCTGGCGCCGAAGTGAAGAAACCTGGCAGCAGCGTGAAGGTGTCCTGCAAGGCTAGCGG
CTACACATTCACCAACTACGTGATGCACTGGGTCAAAGAGAAGCCCGGCCAGGGACTTGAGTGGATGGGCTACATCA
ACCCCTACAACGACGGCACCAAGTACAACGAGAAGTTCCAGGGCAGAGTGACCCTGACCAGCGACAAGTCTAGCAAC
ACCGCCTACATGGAACTGAGCAGCCTGAGAAGCGAGGACACCGCCGTGTACTACTGTGCTAGAGGCGGAGTGCGGAG
ATACTTCGACTACTGGGGACAGGGCACCCTGGTCACAGTCTCTTCT

The amino acid sequence of VL isas shown in SEQ ID NO. 194, the coding nucleic acid of which is as shown in SEQ ID NO. 195, and the LCDR1, LCDR2 and LCDR3 of which are respectively as shown in SEQ ID NO. 123, 124, and 125.

DIQMTQSPSSLSASVGDRVTITCRASQDIGSNLNWLQKEPGKAPKRLIYATSSLDSGVPSRFSGSRSGSDYTLTISS
LQPEDFATYYCLQYASFPYTFGQGTKLEIK

### Nucleic acid sequence

GACATCCAGATGACACAGAGCCCTAGCAGCCTGTCTGCCAGCGTGGGAGACAGAGTGACCATCACCTGTAGAGCCAG
CCAGGACATCGGCAGCAACCTGAACTGGCTGCAGAAAGAGCCTGGCAAGGCCCCTAAGAGACTGATCTACGCCACAA
GCAGCCTGGACAGCGGAGTGCCTTCTAGATTCAGCGGCAGCAGAAGCGGCAGCGACTACACCCTGACAATCAGCAGT
CTGCAGCCCGAGGACTTCGCCACCTACTACTGTCTGCAGTACGCTAGCTTCCCCTACACCTTCGGCCAGGGCACCAA
GCTGGAAATCAAG

Plasmids encoding the corresponding antibody fragments were mixed according to the ratio of κ light chain: λ light chain: heavy chain 1: heavy chain 2 = 2:2:1:1. After mixing with 3 mg/mL PEI, CHO-S cells were co-transfected and cultured in 500 mL CD CHO AGT medium (Gibco#12490-001) at 37°C with 5%CO₂ at 150 rpm. On days 2, 4 and 6 after transient transfection, 4% CHO Feed C+ (Gibco #A25031-05) was added. When the cell survival rate decreased to about 85%, the fermentation broth was harvested and purified by Protein A affinity chromatography. SEC-HPLC showed that the monomer content was higher than 90%. The control GPRC5D×CD3 bispecific antibody GCDB72 was synthesized and assembled as described in the patent document US10562968B2, and the isotype controls anti-KLH×CD3, anti-KLH antibody and anti-CD3e antibody VH9VL5 were constructed according to the method in WO2022042661A1.

### Example 4. Binding activity and TDCC killing activity of GPRC5D× CD3κ λ bispecific antibody

### (1) Binding of GPRC5D×CD3κλ bispecific antibody to GPRC5D⁺ tumor cells

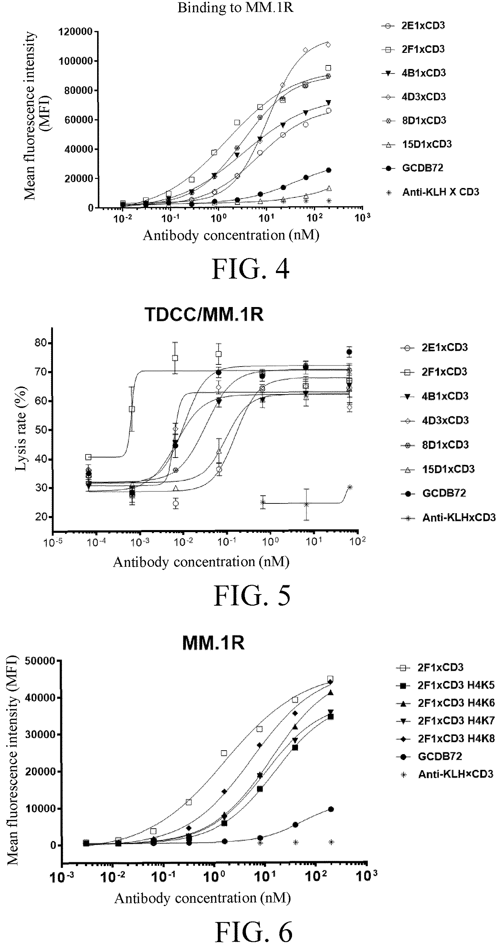

MM.1R tumor cells in logarithmic growth phase was added with 200 µg/mL mouse IgG (Jackson ImmunoResearch, 115-005-03) and blocked for 30 min in an ice bath. The cell suspension was adjusted to a concentration of 5 × 10⁵ cells/mL, and 100 µL per well was added to a 96-well U-shaped plate, centrifuged at 300 g for 5 min. The supernatant was discarded. 100 µL per well of serially diluted antibody was added (initial concentration: 200 nM, 3-fold dilution, 10 gradients), and incubated at 4°C for 60 min. The primary antibody was washed away, and 50 µL/well Alexa Fluro647 labeled goat anti-human IgG FC (1:300 dilution) was added, and incubated for 20 min. After washing once, 50 µL/well of PI was added, incubated for 5 min, and detected by flow cytometry. The results are shown in Fig. 4. The GPRC5D×CD3 κλ bispecific antibodies can all recognize the MM.1R tumor cells. The GPRC5D×CD3 κλ bispecific antibodies including 2E1×CD3, 2F1×CD3, 4B1×CD3, 4D3×CD3 and 8D1×CD3 have significantly higher binding activity to tumor cells than GCDB72, and the average fluorescence intensity in binding MM.1R at the highest concentration of 200 nM is 2.60-4.4 time of GCDB72 (Table 4).

### (2) TDCC effect mediated by GPRC5D×CD3κλ bispecific antibodies

Freshly isolated PBMCs were mixed with MM.1R tumor cells in logarithmic growth phase at a ratio of effector cell: target cell =10:1. 50 µL per well of serially diluted antibody was added (initial antibody concentration: 66.7 nM, 10-fold dilution, 7 gradients), and cultured at 37°C with 5%CO₂ for 24 hrs. After culture, 50 µL of the supernatant was transferred to a new black microplate, and 50 µL/well of substrate for LDH detection was added. After 10 min, the reaction was terminated and the release of LDH was detected. The results are shown in Fig. 5. The 2F1 GPRC5D×CD3κλ bispecific antibody has strong killing activity on tumor cells (EC50=0.65 pM), which is significantly better than GCDB72 (Table 4).

**Table 4. In-vitro activity of GPRC5D×CD3κλ bispecific antibodies**

| GPRC5D×CD3 κλ bispecific antibody | Binding to MM.1R | | TDCC |
|---|---|---|---|
| | EC50 (nM) | Maximum fluorescence intensity (MFI, 200 nM) | EC50 (pM) |
| 2E1×CD3 | 6.88 | 65655 | 161.4 |
| 2F1×CD3 | 1.51 | 94610 | 0.65 |
| 4B1×CD3 | 3.35 | 70907 | 6.95 |
| 4D3×CD3 | 10.65 | 110302 | 6.18 |
| 8D1×CD3 | 3.34 | 89031 | 32.9 |
| 15D1×CD3 | weak | 12807 | 96.5 |
| GCDB72 | 38.73 | 25080 | 10.5 |
| anti-KLH×CD3 | - | 4231 | - |

### Example 5. Optimization of 2F1 GPRC5D×CD3 κλ humanized bispecific antibodies

### (1) Constuction of 2F1 GPRC5D×CD3 κλ bispecific antibody variants

2F1 GPRC5D×CD3 κλ bispecific antibody had high TDCC activity. Different VK1 and VH1 human germline family genes (VK1-33, VK1-39, VH1-46, and VH1-69) were used to optimize the affinity, surface charge distribution and isoelectric point of humanized 2F1 antibody, and different variants of 2F1×CD3 κλ bispecific antibody were obtained (see Table 5 and Table 6). The plasmids encoding the light and heavy chains of the GPRC5D and CD3 antibodies were mixed according to a ratio of κlight chain: λ light chain: heavy chain 1: heavy chain 2 = 2:2:1:1. After mixing with 3 mg/mL PEI, CHO-S cells were co-transfected and cultured in CD CHO AGT medium at 37°C with 5%CO₂ at 150 rpm. On days 2, 4 and 6 after transient transfection, 4% CHO Feed C+ was added. When the cell survival rate decreased to about 85%, the fermentation broth was harvested, filtered and preliminarily purified by Protein A affinity chromatography. SEC-HPLC showed the monomer content was higher than 90%. After further purification by Capto S ImpAct ion exchange chromatography, the monomer content was increased to > 98-99%.

**Table 5. Light and heavy chain sequences of different 2F1×CD3 κλ bispecific antibodies and their variants**

| 2F1×CD3 κλ bispecific antibody and variants thereof | GPRC5D antibody | | | | | | | | | | | | | |
|---|---|---|---|---|---|---|---|---|---|---|---|---|---|---|
| | Heavy chain | | | | | | | Light chain | | | | | | |
| | Amino acid sequence | Nucleic acid sequence | HCVR | | HCDR1 | HCDR2 | HCDR3 | Amino acid sequence | Nucleic acid sequence | LCVR | | LCDR1 | LCDR2 | LCDR3 |
| | | | Amino acid sequence | Nucleic acid sequence | | | | | | Amino acid sequence | Nucleic acid sequence | | | |
| 2F1×CD3 | 196 | 197 | 168 | 169 | 25 | 170 | 27 | 198 | 199 | 171 | 172 | 173 | 31 | 32 |
| 2F1×CD3 H4K5 | 200 | 201 | 202 | 203 | 25 | 170 | 27 | 204 | 205 | 206 | 207 | 208 | 209 | 32 |
| 2F1×CD3 H4K6 | 200 | 201 | 202 | 203 | 25 | 170 | 27 | 210 | 211 | 212 | 213 | 173 | 209 | 32 |
| 2F1×CD3 H4K7 | 200 | 201 | 202 | 203 | 25 | 170 | 27 | 214 | 215 | 216 | 217 | 208 | 31 | 32 |
| 2F1×CD3 H4K8 | 200 | 201 | 202 | 203 | 25 | 170 | 27 | 218 | 219 | 220 | 221 | 173 | 31 | 32 |
| | | | | | | | | | | | | | | |

| 2F1×CD3 κλ Bispecific antibody and variant thereof | CD3 antibody | | | | | | | | | | | | | |
|---|---|---|---|---|---|---|---|---|---|---|---|---|---|---|
| | Heavy chain | | | | | | | Light chain | | | | | | |
| | Amino acid sequence | Nucleic acid sequence | HCVR | | HCDR1 | HCDR2 | HCDR3 | Amino acid sequence | Nucleic acid sequence | LCVR | | LCDR1 | LCDR2 | LCDR3 |
| | | | Amino acid sequence | Nucleic acid sequence | | | | | | Amino acid sequence | Nucleic acid sequence | | | |
| 2F1×CD3 | 222 | 223 | 157 | 158 | 159 | 160 | 161 | 224 | 225 | 152 | 153 | 154 | 155 | 156 |
| 2F1×CD3 H4K5 | 222 | 223 | 157 | 158 | 159 | 160 | 161 | 224 | 225 | 152 | 153 | 154 | 155 | 156 |
| 2F1×CD3 H4K6 | 222 | 223 | 157 | 158 | 159 | 160 | 161 | 224 | 225 | 152 | 153 | 154 | 155 | 156 |
| 2F1×CD3 H4K7 | 222 | 223 | 157 | 158 | 159 | 160 | 161 | 224 | 225 | 152 | 153 | 154 | 155 | 156 |
| 2F1×CD3 H4K8 | 222 | 223 | 157 | 158 | 159 | 160 | 161 | 224 | 225 | 152 | 153 | 154 | 155 | 156 |

**Table 6. Specific light and heavy chain sequences of different 2F1×CD3 κλ bispecific antibodies and their variants**

| 2F1×CD3 κλ Bispecific antibody and variant thereof | GPRC5D antibody | CD3 antibody |
|---|---|---|
| 2F1×CD3 | Heavy chain: the amino acid sequence thereof is as shown in SEQ ID NO. 196, and a coding nucleic acid therefor is as shown in SEQ ID NO. 197 | Heavy chain: the amino acid sequence thereof is as shown in SEQ ID NO. 222, and a coding nucleic acid therefor is as shown in SEQ ID NO. 223 |
| | QVQLVQSGAEVKKPGSSVKVSCKASGYSFTGY TMNWVREAPGQGLEWMGLINPYNGGTRYNQKF QGRVTLTVDKSSSTAYMELSSLRSEDTAVYYC ARWALRYGMDYWGQGTLVTVSSASTKGPSVFP LAPCSRSTSESTAALGCLVKDYFPEPVTVSWN SGALTSGVHTFPAVLQSSGLYSLSSVVTVPSS SLGTKTYTCNVDHKPSNTKVDKRVESKYGPPC PPCPAPEFEGGPSVFLFPPKPKDTLMISRTPE VTCVVVDVSQEDPEVQFNWYVDGVEVHNAKTK PREEQFNSTYRVVSVLTVLHQDWLNGKEYKCK VSNKGLASSIEKTISKAKGQPREPQVCTLPPS QEEMTKNQVSLSCAVKGFYPSDIAVEWESNGQ PENNYKTTPPVLDSDGSFFLVSKLTVDKSRWQ EGNVFSCSVMHEALHNHYTQKSLSLSLGK | EVQLVESGGGLVQPGGSLRLSCAASGFTFNTYA MNWVRKAPGKGLEWVGRIRSKYNNYATYYADSV KDRFTISRDDSKNSLYLQMNSLKTEDTAVYYCV RHGNFGNSYVSWFAYWGQGTLVTVSSASTKGPS VFPLAPCSRSTSESTAALGCLVKDYFPEPVTVS WNSGALTSGVHTFPAVLQSSGLYSLSSVVTVPS SSLGTKTYTCNVDHKPSNTKVDKRVESKYGPPC PPCPAPEFEGGPSVFLFPPKPKDTLMISRTPEV TCVVVDVSQEDPEVQFNWYVDGVEVHNAKTKPR EEQFNSTYRVVSVLTVLHQDWLNGKEYKCKVSN KGLASSIEKTISKAKGQPREPQVYTLPPCQEEM TKNQVSLWCLVKGFYPSDIAVEWESNGQPENNY KTTPPVLDSDGSFFLYSKLTVDKSRWQEGNVFS CSVMHEALHNHYTQKSLSLSLGK |
| | Light chain: the amino acid sequence thereof is as shown in SEQ ID NO. 198, and a coding nucleic acid therefor is as shown in SEQ ID NO. 199 | Light chain: the amino acid sequence thereof is as shown in SEQ ID NO. 224, and a coding nucleic acid therefor is as shown in SEQ ID NO. 225 |
| | DIQMTQSPSSLSASVGDRVTITCRASQNVGTN VAWYQKKPGKAPKALIYSASYRNSGVPSRFSG SGSGTDFTLTISSLQPEDFATYFCQQYNSYLL TFGGGTKVEIKRTVAAPSVFIFPPSDEQLKSG TASVVCLLNNFYPREAKVQWKVDNALQSGNSQ ESVTEQDSKDSTYSLSSTLTLSKADYEKHKVY ACEVTHQGLSSPVTKSFNRGEC | QAVVTQEPSLTVSPGGTVTLTCRSSTGAVTTSN YANWVQEKPGQAPRGLIGGTNKRAPWTPARFSG SLLGGKAALTITGAQAEDEAEYYCVLWYSNLWV FGGGTKLTVLGQPKAAPSVTLFPPSSEELQANK ATLVCLISDFYPGAVTVAWKADSSPVKAGVETT TPSKQSNNKYAASSYLSLTPEQWKSHRSYSCQV THEGSTVEKTVAPTECS |
| 2F1×CD3 H4K5 | Heavy chain: the amino acid sequence thereof is as shown in SEQ ID NO. 200, and a coding nucleic acid therefor is as shown in SEQ ID NO. 201 | Heavy chain: the amino acid sequence thereof is as shown in SEQ ID NO. 222, and a coding nucleic acid therefor is as shown in SEQ ID NO. 223 |
| | QVQLVQSGAEVKKPGSSVKVSCKASGYSFTGY TMNWVREAPGQGLEWMGLINPYNGGTRYNQKF QGRVTITADESTSTAYMELSSLRSEDTAVYYC ARWALRYGMDYWGQGTLVTVSSASTKGPSVFP LAPCSRSTSESTAALGCLVKDYFPEPVTVSWN SGALTSGVHTFPAVLQSSGLYSLSSVVTVPSS SLGTKTYTCNVDHKPSNTKVDKRVESKYGPPC PPCPAPEFEGGPSVFLFPPKPKDTLMISRTPE VTCVVVDVSQEDPEVQFNWYVDGVEVHNAKTK PREEQFNSTYRVVSVLTVLHQDWLNGKEYKCK VSNKGLASSIEKTISKAKGQPREPQVCTLPPS QEEMTKNQVSLSCAVKGFYPSDIAVEWESNGQ PENNYKTTPPVLDSDGSFFLVSKLTVDKSRWQ EGNVFSCSVMHEALHNHYTQKSLSLSLGK | EVQLVESGGGLVQPGGSLRLSCAASGFTFNTYA MNWVRKAPGKGLEWVGRIRSKYNNYATYYADSV KDRFTISRDDSKNSLYLQMNSLKTEDTAVYYCV RHGNFGNSYVSWFAYWGQGTLVTVSSASTKGPS VFPLAPCSRSTSESTAALGCLVKDYFPEPVTVS WNSGALTSGVHTFPAVLQSSGLYSLSSVVTVPS SSLGTKTYTCNVDHKPSNTKVDKRVESKYGPPC PPCPAPEFEGGPSVFLFPPKPKDTLMISRTPEV TCVVVDVSQEDPEVQFNWYVDGVEVHNAKTKPR EEQFNSTYRVVSVLTVLHQDWLNGKEYKCKVSN KGLASSIEKTISKAKGQPREPQVYTLPPCQEEM TKNQVSLWCLVKGFYPSDIAVEWESNGQPENNY KTTPPVLDSDGSFFLYSKLTVDKSRWQEGNVFS CSVMHEALHNHYTQKSLSLSLGK |
| | Light chain: the amino acid sequence thereof is as shown in SEQ ID NO. 204, and a coding nucleic acid therefor is as shown in SEQ ID NO. 205 | Light chain: the amino acid sequence thereof is as shown in SEQ ID NO. 224, and a coding nucleic acid therefor is as shown in SEQ ID NO. 225 |
| | DIQMTQSPSSLSASVGDRVTITCQASQNVGTN VAWYQKKPGQAPKALIYSASYLNSGVPSRFSG SGSGTDFTFTISSLQPEDIATYFCQQYNSYLL TFGGGTKVEIKRTVAAPSVFIFPPSDEQLKSG TASVVCLLNNFYPREAKVQWKVDNALQSGNSQ ESVTEQDSKDSTYSLSSTLTLSKADYEKHKVY ACEVTHQGLSSPVTKSFNRGEC | QAVVTQEPSLTVSPGGTVTLTCRSSTGAVTTSN YANWVQEKPGQAPRGLIGGTNKRAPWTPARFSG SLLGGKAALTITGAQAEDEAEYYCVLWYSNLWV FGGGTKLTVLGQPKAAPSVTLFPPSSEELQANK ATLVCLISDFYPGAVTVAWKADSSPVKAGVETT TPSKQSNNKYAASSYLSLTPEQWKSHRSYSCQV THEGSTVEKTVAPTECS |
| 2F1×CD3 H4K6 | Heavy chain: the amino acid sequence thereof is as shown in SEQ ID NO. 200, and a coding nucleic acid therefor is as shown in SEQ ID NO. 201 | Heavy chain: the amino acid sequence thereof is as shown in SEQ ID NO. 222, and a coding nucleic acid therefor is as shown in SEQ ID NO. 223 |
| | QVQLVQSGAEVKKPGSSVKVSCKASGYSFTGY TMNWVREAPGQGLEWMGLINPYNGGTRYNQKF QGRVTITADESTSTAYMELSSLRSEDTAVYYC ARWALRYGMDYWGQGTLVTVSSASTKGPSVFP LAPCSRSTSESTAALGCLVKDYFPEPVTVSWN SGALTSGVHTFPAVLQSSGLYSLSSVVTVPSS SLGTKTYTCNVDHKPSNTKVDKRVESKYGPPC PPCPAPEFEGGPSVFLFPPKPKDTLMISRTPE VTCVVVDVSQEDPEVQFNWYVDGVEVHNAKTK PREEQFNSTYRVVSVLTVLHQDWLNGKEYKCK VSNKGLASSIEKTISKAKGQPREPQVCTLPPS QEEMTKNQVSLSCAVKGFYPSDIAVEWESNGQ PENNYKTTPPVLDSDGSFFLVSKLTVDKSRWQ EGNVFSCSVMHEALHNHYTQKSLSLSLGK | EVQLVESGGGLVQPGGSLRLSCAASGFTFNTYA MNWVRKAPGKGLEWVGRIRSKYNNYATYYADSV KDRFTISRDDSKNSLYLQMNSLKTEDTAVYYCV RHGNFGNSYVSWFAYWGQGTLVTVSSASTKGPS VFPLAPCSRSTSESTAALGCLVKDYFPEPVTVS WNSGALTSGVHTFPAVLQSSGLYSLSSVVTVPS SSLGTKTYTCNVDHKPSNTKVDKRVESKYGPPC PPCPAPEFEGGPSVFLFPPKPKDTLMISRTPEV TCVVVDVSQEDPEVQFNWYVDGVEVHNAKTKPR EEQFNSTYRVVSVLTVLHQDWLNGKEYKCKVSN KGLASSIEKTISKAKGQPREPQVYTLPPCQEEM TKNQVSLWCLVKGFYPSDIAVEWESNGQPENNY KTTPPVLDSDGSFFLYSKLTVDKSRWQEGNVFS CSVMHEALHNHYTQKSLSLSLGK |
| | Light chain: the amino acid sequence thereof is as shown in SEQ ID NO. 210, and a coding nucleic acid therefor is as shown in SEQ ID NO. 211 | Light chain: the amino acid sequence thereof is as shown in SEQ ID NO. 224, and a coding nucleic acid therefor is as shown in SEQ ID NO. 225 |
| | DIQMTQSPSSLSASVGDRVTITCRASQNVGTN VAWYQKKPGQAPKALIYSASYLNSGVPSRFSG SGSGTDFTLTISSLQPEDFATYFCQQYNSYLL TFGGGTKVEIKRTVAAPSVFIFPPSDEQLKSG TASVVCLLNNFYPREAKVQWKVDNALQSGNSQ ESVTEQDSKDSTYSLSSTLTLSKADYEKHKVY ACEVTHQGLSSPVTKSFNRGEC | QAVVTQEPSLTVSPGGTVTLTCRSSTGAVTTSN YANWVQEKPGQAPRGLIGGTNKRAPWTPARFSG SLLGGKAALTITGAQAEDEAEYYCVLWYSNLWV FGGGTKLTVLGQPKAAPSVTLFPPSSEELQANK ATLVCLISDFYPGAVTVAWKADSSPVKAGVETT TPSKQSNNKYAASSYLSLTPEQWKSHRSYSCQV THEGSTVEKTVAPTECS |
| 2F1×CD3 H4K7 | Heavy chain: the amino acid sequence thereof is as shown in SEQ ID NO. 200, and a coding nucleic acid therefor is as shown in SEQ ID NO. 201 | Heavy chain: the amino acid sequence thereof is as shown in SEQ ID NO. 222, and a coding nucleic acid therefor is as shown in SEQ ID NO. 223 |
| | QVQLVQSGAEVKKPGSSVKVSCKASGYSFTGY TMNWVREAPGQGLEWMGLINPYNGGTRYNQKF QGRVTITADESTSTAYMELSSLRSEDTAVYYC ARWALRYGMDYWGQGTLVTVSSASTKGPSVFP LAPCSRSTSESTAALGCLVKDYFPEPVTVSWN SGALTSGVHTFPAVLQSSGLYSLSSVVTVPSS SLGTKTYTCNVDHKPSNTKVDKRVESKYGPPC PPCPAPEFEGGPSVFLFPPKPKDTLMISRTPE VTCVVVDVSQEDPEVQFNWYVDGVEVHNAKTK PREEQFNSTYRVVSVLTVLHQDWLNGKEYKCK VSNKGLASSIEKTISKAKGQPREPQVCTLPPS QEEMTKNQVSLSCAVKGFYPSDIAVEWESNGQ PENNYKTTPPVLDSDGSFFLVSKLTVDKSRWQ EGNVFSCSVMHEALHNHYTQKSLSLSLGK | EVQLVESGGGLVQPGGSLRLSCAASGFTFNTYA MNWVRKAPGKGLEWVGRIRSKYNNYATYYADSV KDRFTISRDDSKNSLYLQMNSLKTEDTAVYYCV RHGNFGNSYVSWFAYWGQGTLVTVSSASTKGPS VFPLAPCSRSTSESTAALGCLVKDYFPEPVTVS WNSGALTSGVHTFPAVLQSSGLYSLSSVVTVPS SSLGTKTYTCNVDHKPSNTKVDKRVESKYGPPC PPCPAPEFEGGPSVFLFPPKPKDTLMISRTPEV TCVVVDVSQEDPEVQFNWYVDGVEVHNAKTKPR EEQFNSTYRVVSVLTVLHQDWLNGKEYKCKVSN KGLASSIEKTISKAKGQPREPQVYTLPPCQEEM TKNQVSLWCLVKGFYPSDIAVEWESNGQPENNY KTTPPVLDSDGSFFLYSKLTVDKSRWQEGNVFS CSVMHEALHNHYTQKSLSLSLGK |
| | Light chain: the amino acid sequence thereof is as shown in SEQ ID NO. 214, and a coding nucleic acid therefor is as shown in SEQ ID NO. 215 | Light chain: the amino acid sequence thereof is as shown in SEQ ID NO. 224, and a coding nucleic acid therefor is as shown in SEQ ID NO. 225 |
| | DIQMTQSPSSLSASVGDRVTITCQASQNVGTN VAWYQKKPGQAPKALIYSASYRNSGVPSRFSG SGSGTDFTFTISSLQPEDIATYFCQQYNSYLL TFGGGTKVEIKRTVAAPSVFIFPPSDEQLKSG TASVVCLLNNFYPREAKVQWKVDNALQSGNSQ ESVTEQDSKDSTYSLSSTLTLSKADYEKHKVY ACEVTHQGLSSPVTKSFNRGEC | QAVVTQEPSLTVSPGGTVTLTCRSSTGAVTTSN YANWVQEKPGQAPRGLIGGTNKRAPWTPARFSG SLLGGKAALTITGAQAEDEAEYYCVLWYSNLWV FGGGTKLTVLGQPKAAPSVTLFPPSSEELQANK ATLVCLISDFYPGAVTVAWKADSSPVKAGVETT TPSKQSNNKYAASSYLSLTPEQWKSHRSYSCQV THEGSTVEKTVAPTECS |
| 2F1×CD3 H4K8 | Heavy chain: the amino acid sequence thereof is as shown in SEQ ID NO. 200, and a coding nucleic acid therefor is as shown in SEQ ID NO. 201 | Heavy chain: the amino acid sequence thereof is as shown in SEQ ID NO. 222, and a coding nucleic acid therefor is as shown in SEQ ID NO. 223 |
| | QVQLVQSGAEVKKPGSSVKVSCKASGYSFTGY TMNWVREAPGQGLEWMGLINPYNGGTRYNQKF QGRVTITADESTSTAYMELSSLRSEDTAVYYC ARWALRYGMDYWGQGTLVTVSSASTKGPSVFP LAPCSRSTSESTAALGCLVKDYFPEPVTVSWN SGALTSGVHTFPAVLQSSGLYSLSSVVTVPSS SLGTKTYTCNVDHKPSNTKVDKRVESKYGPPC PPCPAPEFEGGPSVFLFPPKPKDTLMISRTPE VTCVVVDVSQEDPEVQFNWYVDGVEVHNAKTK PREEQFNSTYRVVSVLTVLHQDWLNGKEYKCK VSNKGLASSIEKTISKAKGQPREPQVCTLPPS QEEMTKNQVSLSCAVKGFYPSDIAVEWESNGQ PENNYKTTPPVLDSDGSFFLVSKLTVDKSRWQ EGNVFSCSVMHEALHNHYTQKSLSLSLGK | EVQLVESGGGLVQPGGSLRLSCAASGFTFNTYA MNWVRKAPGKGLEWVGRIRSKYNNYATYYADSV KDRFTISRDDSKNSLYLQMNSLKTEDTAVYYCV RHGNFGNSYVSWFAYWGQGTLVTVSSASTKGPS VFPLAPCSRSTSESTAALGCLVKDYFPEPVTVS WNSGALTSGVHTFPAVLQSSGLYSLSSVVTVPS SSLGTKTYTCNVDHKPSNTKVDKRVESKYGPPC PPCPAPEFEGGPSVFLFPPKPKDTLMISRTPEV TCVVVDVSQEDPEVQFNWYVDGVEVHNAKTKPR EEQFNSTYRVVSVLTVLHQDWLNGKEYKCKVSN KGLASSIEKTISKAKGQPREPQVYTLPPCQEEM TKNQVSLWCLVKGFYPSDIAVEWESNGQPENNY KTTPPVLDSDGSFFLYSKLTVDKSRWQEGNVFS CSVMHEALHNHYTQKSLSLSLGK |
| | Light chain: the amino acid sequence thereof is as shown in SEQ ID NO. 218, and a coding nucleic acid therefor is as shown in SEQ ID NO. 219 | Light chain: the amino acid sequence thereof is as shown in SEQ ID NO. 224, and a coding nucleic acid therefor is as shown in SEQ ID NO. 225 |
| | DIQMTQSPSSLSASVGDRVTITCRASQNVGTN VAWYQKKPGQAPKALIYSASYRNSGVPSRFSG SGSGTDFTLTISSLQPEDFATYFCQQYNSYLL TFGGGTKVEIKRTVAAPSVFIFPPSDEQLKSG TASVVCLLNNFYPREAKVQWKVDNALQSGNSQ ESVTEQDSKDSTYSLSSTLTLSKADYEKHKVY ACEVTHQGLSSPVTKSFNRGEC | QAVVTQEPSLTVSPGGTVTLTCRSSTGAVTTSN YANWVQEKPGQAPRGLIGGTNKRAPWTPARFSG SLLGGKAALTITGAQAEDEAEYYCVLWYSNLWV FGGGTKLTVLGQPKAAPSVTLFPPSSEELQANK ATLVCLISDFYPGAVTVAWKADSSPVKAGVETT TPSKQSNNKYAASSYLSLTPEQWKSHRSYSCQV THEGSTVEKTVAPTECS |

### (2) Binding of 2F1 GPRC5D×CD3 κλ bispecific antibody and variants thereof to GPRC5D⁺ tumor cell line

The cell suspension of MM.1R cells in logarithmic growth phase was adjusted to a concentration of 5 x 10⁵ cells/mL, and 100 µL per well was added to a 96-well U-shaped plate, and centrifuged at 300 g for 5 min. The supernatant was discarded. 100 µL per well of serially diluted antibody was added (initial concentration: 1800 nM, 3-fold dilution, 12 gradients), and incubated at 4°C for 60 min. The primary antibody was washed away, and 50 µL/well Alexa Fluro647 labeled goat anti-human IgG FC (1:300 dilution) was added, and incubated on ice for 20 min. After washing once, 50 µL/well of PI was added, incubated for 5 min, and detected by flow cytometry. The results are shown in Fig. 6. 2F1 GPRC5D×CD3κλ bispecific antibody and variants thereof can all recognize MM.1R cells, with an affinity slightly weaker than that of the parent 2F1×CD3 bispecific antibody, but they all show higher binding ability than GCDB72.

### (3) Binding of 2F1 GPRC5D×CD3 κλ bispecific antibody variants to human and Cynomolgus GPRC5D stably transfected cell lines

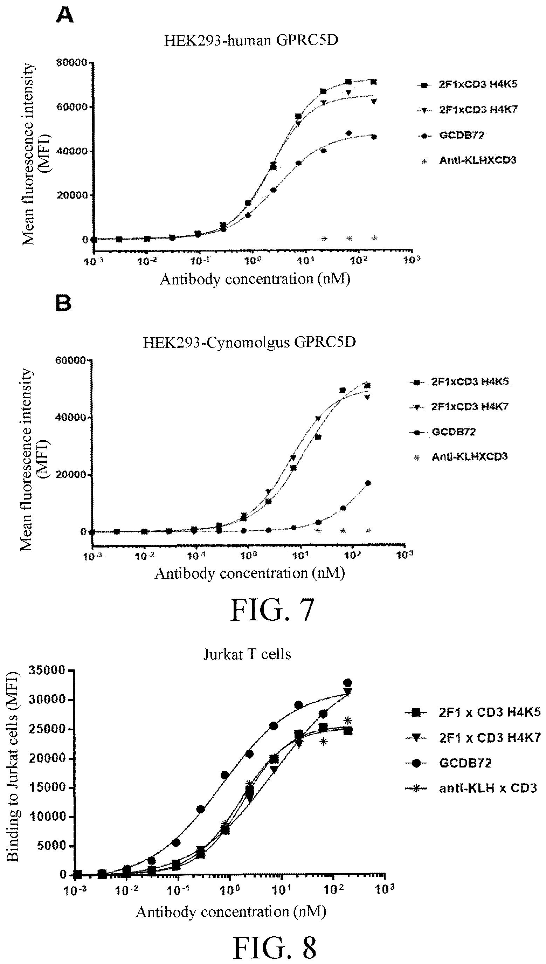

HEK293-hGPRC5D and HEK293-cyGPRC5D stably transfected cells in logarithmic growth phase were respectively adjusted to a cell density of 5×10⁵ cells/mL. Then, 100 µl/well of the cell suspension was added to a 96-well U-shaped plate, and centrifuged at 300 g for 5 min. The supernatant was discarded. 100 µL per well of serially diluted 2F1 GPRC5D×CD3 κλ bispecific antibody variants H4K5 and H4K7 and GCDB72 were added (initial concentration 1800 nM, 3-fold dilution, 12 gradients), and incubated at 4°C for 60 min. The secondary antibody 50 µL/well of Alexa Fluro647 labeled goat anti-human IgG Fc (1:300 dilution) was added, and incubated on ice for 20 min. After washing once, 50 µL/well of PI solution (1:300) was added, incubated for 5 min, and detected by flow cytometry. The results are shown in Fig. 7. The affinity of 2F1 GPRC5D×CD3 κλ bispecific antibody variants H4K5 and H4K7 binding human GPRC5D (Fig. 7A) and Cynomolgus GPRC5D (Fig. 7B) is similar (<5 times). Therefore, Cynomolgus can be used as a research-related species. In contrast, the binding between GCDB72 and Cynomolgus GPRC5D stably transfected cells is weak (Table 6).

### (4) Binding of 2F1 GPRC5D×CD3 κλ bispecific antibody variants to Jurkat T cells

Human leukemia T cell line Jurkat cells in logarithmic growth phase were added with 200 µg/mL mouse IgG (Jackson ImmunoResearch, 115-005-03), and allowed to stand in an ice bath for 30 min. The cell concentration was adjusted with 4% calf serum to 5×10⁵ cells/mL. 100 µL per well was added to a 96-well U-shaped plate, and centrifuged at 300 g. The supernatant was removed. 100 µL per well of serially diluted 2F1 GPRC5D×CD3κλ bispecific antibody variants H4K5 and H4K7 and GCDB72 were added (initial concentration 1800 nM, 3-fold dilution, 10 gradients), and incubated at 4°C for 60 min. The secondary antibody 50 µL/well of Alexa Fluro647 labeled goat anti-human IgG Fc (1:300 dilution) was added, and incubated on ice for 20 min. After washing once, 50 µL/well of PI was added, incubated for 5 min, and detected by flow cytometry. The detection results are shown in Fig. 8. The binding ability of 2F1 GPRC5D×CD3 κλ bispecific antibody variants to T cells is comparable, and significantly weaker than that of GCDB72. This reduces the retention of the GPRC5D×CD3κλ bispecific antibody in peripheral lymphoid organs, increases its enrichment in tumor sites, and allows it to more gently activate T cells and reduce the release of cytokines.

### (5) TDCC mediated by 2F1 GPRC5D×CD3 κλ bispecific antibody variants

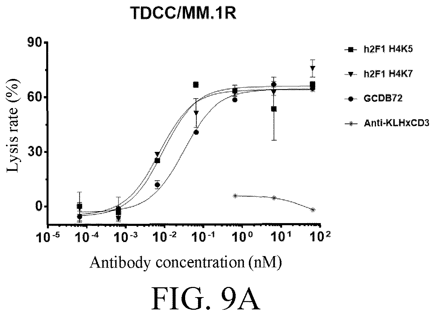

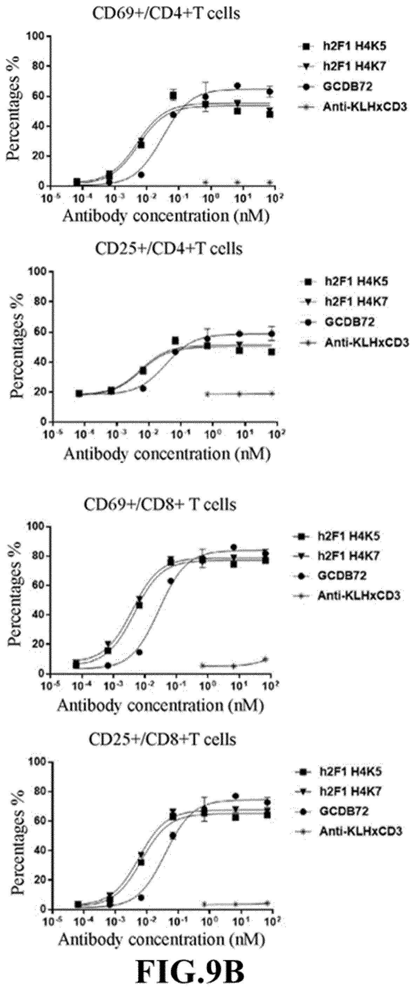

Freshly isolated PBMCs were mixed with MM.1R tumor cells in logarithmic growth phase at a ratio of effector cell: target cell =10:1. 50 µL per well of serially diluted antibody was added (initial antibody concentration: 66.7 nM, 10-fold dilution, 7 gradients), and cultured at 37°C with 5%CO₂ for 24 hrs. After culture, 50 µL of the supernatant was transferred to a new black microplate, and 50 µL/well of substrate for LDH detection was added. After 10 min, the reaction was terminated and the release of LDH was detected. The remaining cells in the well were washed twice with 4% calf serum, 100 µg/mL human IgG was added and incubated for 10 min, and an antibody for T cell activation and detection (CD25-PE, CD4-APC, CD69-FITC and CD8-APC) was added, and incubated on ice for 20 min. After washing, the supernatant was discarded. 60 µL/well of PI was added, incubated on ice for 5 min, and detected by flow cytometry. The detection results are shown in Fig. 9. 2F1 GPRC5D×CD3 κλ bispecific antibody variants can effectively kill tumor cells by mediating TDCC (Fig. 9A). The expression of T cell activation markers CD69 and CD25 is up-regulated during the killing process (Figure 9B) showing that CD8⁺ T cells and CD4⁺ T cells are effectively activated.

### Example 6. Activation of T cell activation pathway by GPRC5D×CD3κλbispecific antibody variants

Jurkat cells were transfected with luciferase reporter plasmid pGL4.30[luc2P/NFAT-RE/Hygro](Promega, E8481). 200 µg/ml hygromycin B was added to screen out a Jurkat-NFAT luc stably transformed reporter cell. GPRC5D⁺ target cells, GPRC5D×CD3 antibody and Jurkat-NFAT-luc reporter cells were co-incubated. The GPRC5D×CD3 antibody was firstly bound to the target cells, and then the other end was bound to the CD3 receptor on the surface of Jurkat cells, causing the aggregation of intracellular signaling motif of CD3. The activation signal was transmitted to the nucleus through NFAT pathway, which eventually led to the up-regulation of luciferase expression of the reporter gene.

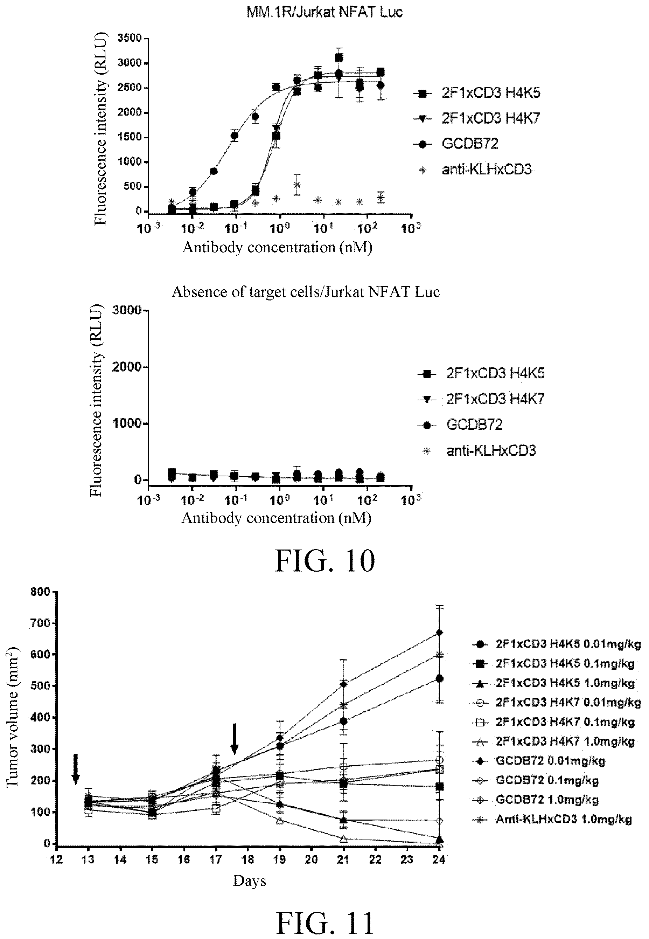

Jurkat-NFAT-luc reporter cells and MM.1R cells in logarithmic growth phase were centrifuged. The supernatant was discarded. The cells were re-suspended to give a density of 2×10⁶ cells/ml. The target cells were inoculated into a 96 well plate in an amount of 50 µl/well, and centrifuged at 300 g for 5 min. The supernatant was discarded. The Jurkat-NFAT-luc reporter cells were inoculated into a 96-well plate in an amount of 50 µl/well. 50 µL per well of a serially diluted GPRC5D×CD3κλ bispecific antibody variant or a control antibody was added (initial concentration 20 µg/ml, 10-fold dilution, 10 gradients), and incubated with 5%CO₂ at 37°C for 6 hrs. After culture, according to the instructions of ONE-Glo Luciferase Assay System, 100 µL per well of the detection reagent was added and left at room temperature for 3 min, and then the fluorescence signal was detected (Biotek Synergy HT). Fig. 10 shows the activation of Jurkat cells by the GPRC5D×CD3κλ bispecific antibody variant depends on the existence of target cells, and EC50 is about 0.66-0.77 nM, which is weaker than that of GCDB72 (EC50=0.066nM). Table 7 summarizes the in-vitro activity results of 2F1 GPRC5D×CD3 κλ bispecific antibody variants.

**Table 7. In-vitro activity of 2F1 GPRC5D×CD3 κλ bispecific antibody variants**

| EC50 (nM) | MM.1R | hGPRC5D | cyGPRC5D | Jurkat T | TDCC | Jurkat-NFAT-luc |
|---|---|---|---|---|---|---|
| 2F1×CD3 H4K5 | 15.7 | 2.8 | 12.8 | 1.9 | 0.008 | 0.662 |
| 2F1×CD3 H4K7 | 10.2 | 2.3 | 6.6 | 7.2 | 0.009 | 0.774 |
| GCDB72 | 54.2 | 3.0 | Weak | 0.7 | 0.032 | 0.066 |
| anti-KLH×CD3 | - | - | - | 1.6 | - | - |

### Example 7. Effect of GPRC5D×CD3κλ bispecific antibody variants on subcutaneous MM.1S transplanted tumor model of immunodeficient mice

Female B-NDG mice (Biosetu Biotechnology Co. Ltd) aged 6-8 weeks were subcutaneously inoculated with 6×10⁶ MM.1S cells. When the tumor grew to 130-140 mm³, the animals were randomized into groups, including 1.0 mg/kg, 0.1 mg/kg, 0.01 mg/kg administration groups and a negative control group with 1 mg/kg KLH×CD3. 1×10⁷ PBMC cells were injected into the tail vein of each mouse. The first administration was performed 6 days later. The administration interval was once every 5 days. A total of 2 administrations were performed. The tumor volume and weight of mice were monitored. After the experiment, the mice were sacrificed by cervical dislocation. The tumors were removed and weighed, and the weight was recorded.

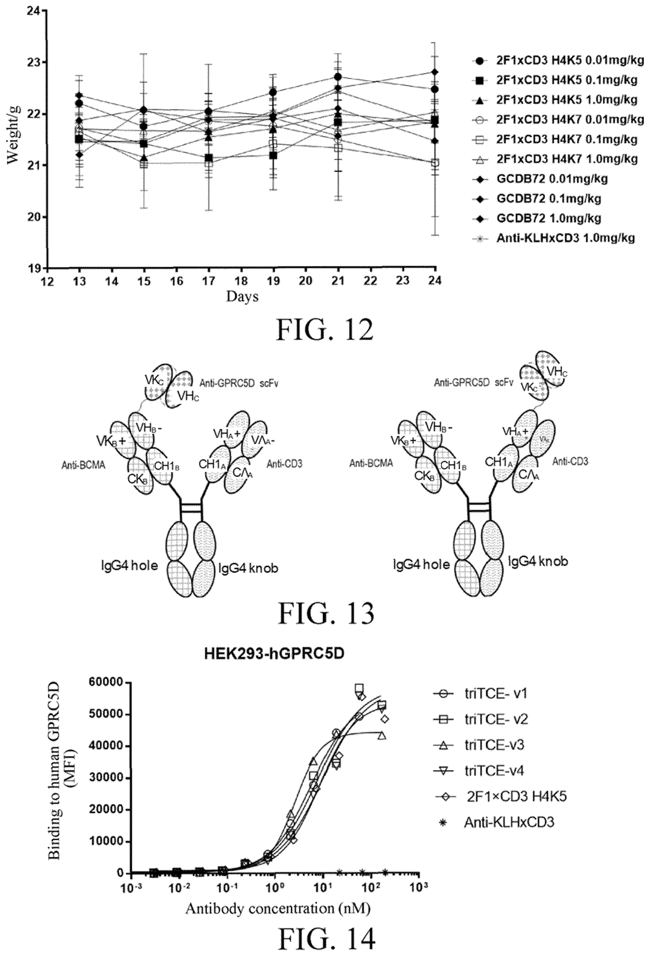

The results are shown in Fig. 11. The in-vivo efficacy of the GPRC5D×CD3κλ bispecific antibody variant is dose dependent, and the tumors in the high- and middle-dose groups are completely inhibited or relieved. At the high, middle, and low concentrations, h2F1 H4K5 and h2F1 H4K7 show a more potent antitumor effect than GCDB72. Fig. 12 shows that the tumor-bearing mice have good tolerance to the above doses and no adverse reactions such as weight loss.

### Example 8. Construction of GPRC5D×BCMA×CD3 triTCE trispecific antibody

### (1) Construction of GPRC5D×BCMA×CD3 triTCE antibody

The light and heavy chain variable regions of the humanized anti-GPRC5D antibody 2F1 H4K5 was linked by a flexible polypeptide chain GGGGSGGGGSGGGGS to the N terminus of the heavy chain of the BCMA arm or CD3 arm of the BCMA×CD3 κλ005 bispecific antibody (See Chinese Patent No. CN114573703A), to construct a new GPRC5D×BCMA×CD3 triTCE trispecific antibody, which could recognize two tumor antigens and recruit T cells. The structure of GPRC5D×BCMA×CD3 triTCE is schematically shown in Fig. 13.

In Tables 8 and 9, the amino acid sequence of the light chain of BCMA is as shown in SEQ ID NO. 226, the coding nucleic acid of which is as shown in SEQ ID NO 227, the light chain variable region of which is as shown in SEQ ID NO. 267, and the coding nucleic acid for the light chain variable region is as shown in SEQ ID NO. 269; and the amino acid sequence of the heavy chain isas shown in SEQ ID NO. 233, the coding nucleic acid of which is as shown in SEQ ID NO. 238, the heavy chain variable region is as shown in SEQ ID NO. 268, and the coding nucleic acid for the heavy chain variable region is as shown in SEQ ID NO. 270.

In Tables 8 and 9, the amino acid sequence of the light chain variable region (GPRC5D-VL) of GPRC5D is as shown in SEQ ID NO. 230, and the coding nucleic acid of which is as shown in SEQ ID NO. 271; the amino acid sequence of the heavy chain variable region (GPRC5D-VH) of GPRC5D is as shown in SEQ ID NO. 232, and a coding nucleic acid therefor is as shown in SEQ ID NO. 272.

**Table 8. Sequence of GPRC5D×BCMA×CD3 triTCE trispecific antibody**

| GPRC5D ×BCMA ×CD3 | Light chain 1 | | Heavy chain 1 | | | | | | | Light chain 2 | | Heavy chain 2 | |
|---|---|---|---|---|---|---|---|---|---|---|---|---|---|
| triTCE V1 | Amino acid sequence | Nucleotide sequence | Amino acid sequence | Nucleotide sequence | GPRC5D-VL | Linker | GPRC5D-VH | Linker | BCMA-Heavy | Amino acid sequence | Nucleotide sequence | Amino acid sequence | Nucleotide sequence |
| | 226 (BCMA-Light) | 227 | 228 (GPRC5D-VL ×GPRC5D-VH ×BCMA-Heavy) | 229 | 230 | 231 | 232 | 231 | 233 | 224 (CD3-Light) | 225 | 222 (CD3-Heavy) | 223 |
| triTCE V2 | Amino acid sequence | Nucleotide sequence | Amino acid sequence | Nucleotide sequence | GPRC5D-VH | Linker | GPRC5D-VL | Linker | BCMA-Heavy | Amino acid sequence | Nucleotide sequence | Amino acid sequence | Nucleotide sequence |
| | 226 (BCMA-Light) | 227 | 234 (GPRC5D-VH ×GPRC5D-VL ×BCMA-Heavy) | 235 | 232 | 231 | 230 | 231 | 233 | 224 (CD3-Light) | 225 | 222 (CD3-Heavy) | 223 |
| triTCE V3 | Amino acid sequence | Nucleotide sequence | Amino acid sequence | Nucleotide sequence | GPRC5D-VL | Linker | GPRC5D-VH | Linker | CD3-Heavy | Amino acid sequence | Nucleotide sequence | Amino acid sequence | Nucleotide sequence |
| | 224 (CD3-Light) | 225 | 236 (GPRC5D-VL ×GPRC5D-VH ×CD3-Heavy) | 237 | 230 | 231 | 232 | 231 | 222 | 226 (BCMA-Light) | 227 | 233 (BCMA-Heavy) | 238 |
| triTCE V4 | Amino acid sequence | Nucleotide sequence | Amino acid sequence | Nucleotide sequence | GPRC5D-VH | Linker | GPRC5D-VL | Linker | CD3-Heavy | Amino acid sequence | Nucleotide sequence | Amino acid sequence | Nucleotide sequence |
| | 224 (CD3-Light) | 225 | 239 (GPRC5D-VH ×GPRC5D-VL ×CD3-Heavy) | 240 | 232 | 231 | 230 | 231 | 222 | 226 (BCMA-Light) | 227 | 233 (BCMA-Heavy) | 238 |

**Table 9. Specific sequence of GPRC5D×BCMA×CD3 triTCE trispecific antibody**

| GPRC5D× BCMA× CD3 triTCE | Light chain 1 | Heavy chain 1 |
|---|---|---|
| triTCE-V1 | The amino acid sequence thereof is as shown in SEQ ID NO. 226, and a coding nucleic acid therefor is as shown in SEQ ID NO. 227 (BCMA-Light) | The amino acid sequence thereof is as shown in SEQ ID NO. 228, and a coding nucleic acid therefore is as shown in SEQ ID NO. 229 (GPRC5D-VL×GPRC5D-VH×BCMA-Heavy) |
| | DIVLTQSPASLAVSPGQRATITCRASKSVSTSG YSYMHWYQKKPGQPPKLLIYLASNLESGVPARF SGSGSGTDFTLTINPVEAEDTANYYCQHSRELP WTFGQGTKVEIKRTVAAPSVFIFPPSDEQLKSG TASVVCLLNNFYPREAKVQWKVDNALQSGNSQE SVTEQDSKDSTYSLSSTLTLSKADYEKHKVYAC EVTHQGLSSPVTKSFNRGEC | DIQMTQSPSSLSASVGDRVTITCQASQNVGTNVA WYQQKPGQAPKALIYSASYLNSGVPSRFSGSGSG TDFTFTISSLQPEDIATYFCQQYNSYLLTFGCGT KVEIKGGGGSGGGGSGGGGSQVQLVQSGAEVKKP GSSVKVSCKASGYSFTGYTMNWVRQAPGQCLEWM GLINPYNGGTRYNQKFQGRVTITADESTSTAYME LSSLRSEDTAVYYCARWALRYGMDYWGQGTLVTV SSGGGGSGGGGSGGGGSQVQLVQSGSELKKPGAS VKVSCKASGYIFTNFGMNWVREAPGQGLEWMGWI NTYTGEQIYADGFTGRFVFSLDTSVSTAYLQISS LKAEDTAVYFCARGEIYYGYDVGFVYWGQGTLVT VSSASTKGPSVFPLAPCSRSTSESTAALGCLVKD YFPEPVTVSWNSGALTSGVHTFPAVLQSSGLYSL SSVVTVPSSSLGTKTYTCNVDHKPSNTKVDKRVE SKYGPPCPPCPAPEFEGGPSVFLFPPKPKDTLMI SRTPEVTCVVVDVSQEDPEVQFNWYVDGVEVHNA KTKPREEQFNSTYRVVSVLTVLHQDWLNGKEYKC KVSNKGLASSIEKTISKAKGQPREPQVCTLPPSQ EEMTKNQVSLSCAVKGFYPSDIAVEWESNGQPEN NYKTTPPVLDSDGSFFLVSKLTVDKSRWQEGNVF SCSVMHEALHNHYTQKSLSLSLGK |
| triTCE-V2 | The amino acid sequence thereof is as shown in SEQ ID NO. 226, and a coding nucleic acid therefor is as shown in SEQ ID NO. 227 (BCMA-Light) | The amino acid sequence thereof is as shown in SEQ ID NO. 234, and a coding nucleic acid therefor is as shown in SEQ ID NO. 235 (GPRC5D-VH×GPRC5D-VL×BCMA-VH) |
| | DIVLTQSPASLAVSPGQRATITCRASKSVSTSG YSYMHWYQKKPGQPPKLLIYLASNLESGVPARF SGSGSGTDFTLTINPVEAEDTANYYCQHSRELP WTFGQGTKVEIKRTVAAPSVFIFPPSDEQLKSG TASVVCLLNNFYPREAKVQWKVDNALQSGNSQE SVTEQDSKDSTYSLSSTLTLSKADYEKHKVYAC EVTHQGLSSPVTKSFNRGEC | |
| | | QVQLVQSGAEVKKPGSSVKVSCKASGYSFTGYTM NWVRQAPGQCLEWMGLINPYNGGTRYNQKFQGRV TITADESTSTAYMELSSLRSEDTAVYYCARWALR YGMDYWGQGTLVTVSSGGGGSGGGGSGGGGSDIQ MTQSPSSLSASVGDRVTITCQASQNVGTNVAWYQ QKPGQAPKALIYSASYLNSGVPSRFSGSGSGTDF TFTISSLQPEDIATYFCQQYNSYLLTFGCGTKVE IKGGGGSGGGGSGGGGSQVQLVQSGSELKKPGAS VKVSCKASGYIFTNFGMNWVREAPGQGLEWMGWI NTYTGEQIYADGFTGRFVFSLDTSVSTAYLQISS LKAEDTAVYFCARGEIYYGYDVGFVYWGQGTLVT VSSASTKGPSVFPLAPCSRSTSESTAALGCLVKD YFPEPVTVSWNSGALTSGVHTFPAVLQSSGLYSL SSVVTVPSSSLGTKTYTCNVDHKPSNTKVDKRVE SKYGPPCPPCPAPEFEGGPSVFLFPPKPKDTLMI SRTPEVTCVVVDVSQEDPEVQFNWYVDGVEVHNA KTKPREEQFNSTYRVVSVLTVLHQDWLNGKEYKC KVSNKGLASSIEKTISKAKGQPREPQVCTLPPSQ EEMTKNQVSLSCAVKGFYPSDIAVEWESNGQPEN NYKTTPPVLDSDGSFFLVSKLTVDKSRWQEGNVF SCSVMHEALHNHYTQKSLSLSLGK |
| triTCE-V3 | The amino acid sequence thereof is as shown in SEQ ID NO. 224, and a coding nucleic acid therefor is as shown in SEQ ID NO. 225 (CD3-Light) | The amino acid sequence thereof is as shown in SEQ ID NO. 236, and a coding nucleic acid therefor is as shown in SEQ ID NO. 237 (GPRC5D-VL×GPRC5D-VH×CD3-Heavy) |
| | QAVVTQEPSLTVSPGGTVTLTCRSSTGAVTTSN YANWVQEKPGQAPRGLIGGTNKRAPWTPARFSG SLLGGKAALTITGAQAEDEAEYYCVLWYSNLWV FGGGTKLTVLGQPKAAPSVTLFPPSSEELQANK ATLVCLISDFYPGAVTVAWKADSSPVKAGVETT TPSKQSNNKYAASSYLSLTPEQWKSHRSYSCQV THEGSTVEKTVAPTECS | |
| | | DIQMTQSPSSLSASVGDRVTITCQASQNVGTNVA WYQQKPGQAPKALIYSASYLNSGVPSRFSGSGSG TDFTFTISSLQPEDIATYFCQQYNSYLLTFGCGT KVEIKGGGGSGGGGSGGGGSQVQLVQSGAEVKKP GSSVKVSCKASGYSFTGYTMNWVRQAPGQCLEWM GLINPYNGGTRYNQKFQGRVTITADESTSTAYME LSSLRSEDTAVYYCARWALRYGMDYWGQGTLVTV SSGGGGSGGGGSGGGGSEVQLVESGGGLVQPGGS LRLSCAASGFTFNTYAMNWVRKAPGKGLEWVGRI RSKYNNYATYYADSVKDRFTISRDDSKNSLYLQM NSLKTEDTAVYYCVRHGNFGNSYVSWFAYWGQGT LVTVSSASTKGPSVFPLAPCSRSTSESTAALGCL VKDYFPEPVTVSWNSGALTSGVHTFPAVLQSSGL YSLSSVVTVPSSSLGTKTYTCNVDHKPSNTKVDK RVESKYGPPCPPCPAPEFEGGPSVFLFPPKPKDT LMISRTPEVTCVVVDVSQEDPEVQFNWYVDGVEV HNAKTKPREEQFNSTYRVVSVLTVLHQDWLNGKE YKCKVSNKGLASSIEKTISKAKGQPREPQVYTLP PCQEEMTKNQVSLWCLVKGFYPSDIAVEWESNGQ PENNYKTTPPVLDSDGSFFLYSKLTVDKSRWQEG NVFSCSVMHEALHNHYTQKSLSLSLGK |
| triTCE-V4 | The amino acid sequence thereof is as shown in SEQ ID NO. 224, and a coding nucleic acid therefor is as shown in SEQ ID NO. 225 (CD3-Light) | The amino acid sequence thereof is as shown in SEQ ID NO. 239, and a coding nucleic acid therefor is as shown in SEQ ID NO. 240 (GPRC5D-VH×GPRC5D-VL×CD3-Heavy) |
| | QAVVTQEPSLTVSPGGTVTLTCRSSTGAVTTSN YANWVQEKPGQAPRGLIGGTNKRAPWTPARFSG SLLGGKAALTITGAQAEDEAEYYCVLWYSNLWV FGGGTKLTVLGQPKAAPSVTLFPPSSEELQANK ATLVCLISDFYPGAVTVAWKADSSPVKAGVETT TPSKQSNNKYAASSYLSLTPEQWKSHRSYSCQV THEGSTVEKTVAPTECS | |
| | | QVQLVQSGAEVKKPGSSVKVSCKASGYSFTGYTM NWVRQAPGQCLEWMGLINPYNGGTRYNQKFQGRV TITADESTSTAYMELSSLRSEDTAVYYCARWALR YGMDYWGQGTLVTVSSGGGGSGGGGSGGGGSDIQ MTQSPSSLSASVGDRVTITCQASQNVGTNVAWYQ QKPGQAPKALIYSASYLNSGVPSRFSGSGSGTDF TFTISSLQPEDIATYFCQQYNSYLLTFGCGTKVE IKGGGGSGGGGSGGGGSEVQLVESGGGLVQPGGS LRLSCAASGFTFNTYAMNWVRKAPGKGLEWVGRI RSKYNNYATYYADSVKDRFTISRDDSKNSLYLQM NSLKTEDTAVYYCVRHGNFGNSYVSWFAYWGQGT LVTVSSASTKGPSVFPLAPCSRSTSESTAALGCL VKDYFPEPVTVSWNSGALTSGVHTFPAVLQSSGL YSLSSVVTVPSSSLGTKTYTCNVDHKPSNTKVDK RVESKYGPPCPPCPAPEFEGGPSVFLFPPKPKDT LMISRTPEVTCVVVDVSQEDPEVQFNWYVDGVEV HNAKTKPREEQFNSTYRVVSVLTVLHQDWLNGKE YKCKVSNKGLASSIEKTISKAKGQPREPQVYTLP PCQEEMTKNQVSLWCLVKGFYPSDIAVEWESNGQ PENNYKTTPPVLDSDGSFFLYSKLTVDKSRWQEG NVFSCSVMHEALHNHYTQKSLSLSLGK |
| | | |

| | Light chain 2 | Heavy chain 2 |
|---|---|---|
| triTCE-V1 | The amino acid sequence thereof is as shown in SEQ ID NO. 224, and a coding nucleic acid therefor is as shown in SEQ ID NO. 225 (CD3-Light) | The amino acid sequence thereof is as shown in SEQ ID NO. 222, and a coding nucleic acid therefor is as shown in SEQ ID NO. 223 (CD3-Heavy) |
| | QAVVTQEPSLTVSPGGTVTLTCRSSTGAVTTSN YANWVQEKPGQAPRGLIGGTNKRAPWTPARFSG SLLGGKAALTITGAQAEDEAEYYCVLWYSNLWV FGGGTKLTVLGQPKAAPSVTLFPPSSEELQANK ATLVCLISDFYPGAVTVAWKADSSPVKAGVETT TPSKQSNNKYAASSYLSLTPEQWKSHRSYSCQV THEGSTVEKTVAPTECS | EVQLVESGGGLVQPGGSLRLSCAASGFTFNTYAM NWVRKAPGKGLEWVGRIRSKYNNYATYYADSVKD RFTISRDDSKNSLYLQMNSLKTEDTAVYYCVRHG NFGNSYVSWFAYWGQGTLVTVSSASTKGPSVFPL APCSRSTSESTAALGCLVKDYFPEPVTVSWNSGA LTSGVHTFPAVLQSSGLYSLSSVVTVPSSSLGTK TYTCNVDHKPSNTKVDKRVESKYGPPCPPCPAPE FEGGPSVFLFPPKPKDTLMISRTPEVTCVVVDVS QEDPEVQFNWYVDGVEVHNAKTKPREEQFNSTYR VVSVLTVLHQDWLNGKEYKCKVSNKGLASSIEKT ISKAKGQPREPQVYTLPPCQEEMTKNQVSLWCLV KGFYPSDIAVEWESNGQPENNYKTTPPVLDSDGS FFLYSKLTVDKSRWQEGNVFSCSVMHEALHNHYT QKSLSLSLGK |
| triTCE-V2 | The amino acid sequence thereof is as shown in SEQ ID NO. 224, and a coding nucleic acid therefor is as shown in SEQ ID NO. 225 (CD3) | The amino acid sequence thereof is as shown in SEQ ID NO. 222, and a coding nucleic acid therefor is as shown in SEQ ID NO. 223 (CD3-Heavy) |
| | QAVVTQEPSLTVSPGGTVTLTCRSSTGAVTTSN YANWVQEKPGQAPRGLIGGTNKRAPWTPARFSG SLLGGKAALTITGAQAEDEAEYYCVLWYSNLWV FGGGTKLTVLGQPKAAPSVTLFPPSSEELQANK ATLVCLISDFYPGAVTVAWKADSSPVKAGVETT TPSKQSNNKYAASSYLSLTPEQWKSHRSYSCQV THEGSTVEKTVAPTECS | EVQLVESGGGLVQPGGSLRLSCAASGFTFNTYAM NWVRKAPGKGLEWVGRIRSKYNNYATYYADSVKD RFTISRDDSKNSLYLQMNSLKTEDTAVYYCVRHG NFGNSYVSWFAYWGQGTLVTVSSASTKGPSVFPL APCSRSTSESTAALGCLVKDYFPEPVTVSWNSGA LTSGVHTFPAVLQSSGLYSLSSVVTVPSSSLGTK TYTCNVDHKPSNTKVDKRVESKYGPPCPPCPAPE FEGGPSVFLFPPKPKDTLMISRTPEVTCVVVDVS QEDPEVQFNWYVDGVEVHNAKTKPREEQFNSTYR VVSVLTVLHQDWLNGKEYKCKVSNKGLASSIEKT ISKAKGQPREPQVYTLPPCQEEMTKNQVSLWCLV KGFYPSDIAVEWESNGQPENNYKTTPPVLDSDGS FFLYSKLTVDKSRWQEGNVFSCSVMHEALHNHYT QKSLSLSLGK |
| triTCE-V3 | The amino acid sequence thereof is as shown in SEQ ID NO. 226, and a coding nucleic acid therefor is as shown in SEQ ID NO. 227 (BCMA-Light) | The amino acid sequence thereof is as shown in SEQ ID NO. 233, and a coding nucleic acid therefor is as shown in SEQ ID NO. 238 (BCMA-Heavy) |
| | DIVLTQSPASLAVSPGQRATITCRASKSVSTSG YSYMHWYQKKPGQPPKLLIYLASNLESGVPARF SGSGSGTDFTLTINPVEAEDTANYYCQHSRELP WTFGQGTKVEIKRTVAAPSVFIFPPSDEQLKSG TASVVCLLNNFYPREAKVQWKVDNALQSGNSQE SVTEQDSKDSTYSLSSTLTLSKADYEKHKVYAC EVTHQGLSSPVTKSFNRGEC | QVQLVQSGSELKKPGASVKVSCKASGYIFTNFGM NWVREAPGQGLEWMGWINTYTGEQIYADGFTGRF VFSLDTSVSTAYLQISSLKAEDTAVYFCARGEIY YGYDVGFVYWGQGTLVTVSSASTKGPSVFPLAPC SRSTSESTAALGCLVKDYFPEPVTVSWNSGALTS GVHTFPAVLQSSGLYSLSSVVTVPSSSLGTKTYT CNVDHKPSNTKVDKRVESKYGPPCPPCPAPEFEG GPSVFLFPPKPKDTLMISRTPEVTCVVVDVSQED PEVQFNWYVDGVEVHNAKTKPREEQFNSTYRVVS VLTVLHQDWLNGKEYKCKVSNKGLASSIEKTISK AKGQPREPQVCTLPPSQEEMTKNQVSLSCAVKGF YPSDIAVEWESNGQPENNYKTTPPVLDSDGSFFL VSKLTVDKSRWQEGNVFSCSVMHEALHNHYTQKS LSLSLGK |
| triTCE-V4 | The amino acid sequence thereof is as shown in SEQ ID NO. 226, and a coding nucleic acid therefor is as shown in SEQ ID NO. 227 (BCMA-Light) | The amino acid sequence thereof is as shown in SEQ ID NO. 233, and a coding nucleic acid therefor is as shown in SEQ ID NO. 238 (BCMA-Heavy) |
| | DIVLTQSPASLAVSPGQRATITCRASKSVSTSG YSYMHWYQKKPGQPPKLLIYLASNLESGVPARF SGSGSGTDFTLTINPVEAEDTANYYCQHSRELP WTFGQGTKVEIKRTVAAPSVFIFPPSDEQLKSG TASVVCLLNNFYPREAKVQWKVDNALQSGNSQE SVTEQDSKDSTYSLSSTLTLSKADYEKHKVYAC EVTHQGLSSPVTKSFNRGEC | QVQLVQSGSELKKPGASVKVSCKASGYIFTNFGM NWVREAPGQGLEWMGWINTYTGEQIYADGFTGRF VFSLDTSVSTAYLQISSLKAEDTAVYFCARGEIY YGYDVGFVYWGQGTLVTVSSASTKGPSVFPLAPC SRSTSESTAALGCLVKDYFPEPVTVSWNSGALTS GVHTFPAVLQSSGLYSLSSVVTVPSSSLGTKTYT CNVDHKPSNTKVDKRVESKYGPPCPPCPAPEFEG GPSVFLFPPKPKDTLMISRTPEVTCVVVDVSQED PEVQFNWYVDGVEVHNAKTKPREEQFNSTYRVVS VLTVLHQDWLNGKEYKCKVSNKGLASSIEKTISK AKGQPREPQVCTLPPSQEEMTKNQVSLSCAVKGF YPSDIAVEWESNGQPENNYKTTPPVLDSDGSFFL VSKLTVDKSRWQEGNVFSCSVMHEALHNHYTQKS LSLSLGK |

### (2) Expression and purification of GPRC5D×BCMA×CD3 triTCE antibody

Plasmids encoding CD3-GPRC5D scfv arm and BCMA arm (or encoding BCMA-GPRC5D scfv arm and CD3 arm) were mixed at a ratio of 1: 2: 1: 2 (heavy chain: light chain =1:2), and transfected into CHO-S cells. After 5-6 days of expression at 37°C, the culture supernatant was collected, subjected to Protein A and Capto S ImpAct ion exchange chromatography, and eluted over gradients with phosphoric acid-citric acid/0.5M NaCl pH 7.5. The elution peaks with monomer purity of over 90% were combined, concentrated and exchanged into 10 mM acetic acid/150mM arginine pH 4.8 buffer. The product was determined by LC-MS (Agilent 1290 Infinity II; Q-TOF 6545XT) to be the GPRC5D×BCMA×CD3 triTCE trispecific antibody.

### (3) Binding activity of GPRC5D×BCMA×CD3 triTCE antibody

### (i) Affinity assay by BLI

The affinity of GPRC5D×BCMA×CD3 triTCE antibody to BCMA and CD3 recombinant antigens was detected by Bio-layer interferometry (BLI/Gator prime). Biotinylated BCMA antigen or CD3e antigen was captured by SA probe, and then the signals of binding to and dissociation from the probe were detected by using the triTCE antibody as an analyte. The result shows that the fusion expression of GPRC5D scFv on the N-terminus of the BCMA arm or CD3 arm has little influence on the binding of BCMA or CD3 (Table 10).

### (ii) Binding with GPRC5D and BCMA stably transformed cell lines

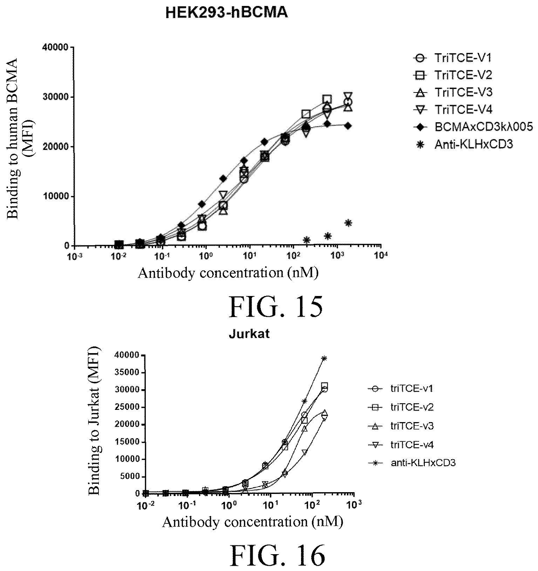

With the full-length sequence of human BCMA (Sino biological, HG10620-M) as a template, a lentiviral vector plasmid containing the full-length sequence of human BCMA was constructed. Following the instructions of a Lenti-Pac HIV Expression Packaging Kit (Gene Copoeia, product number: HPK-LvTR-20), the constructed lentiviral plasmid and a packaging plasmid were co-transfected into HEK293T cells (ATCC CRL-11268), for lentiviral packaging. 10 µL supernatant containing lentiviral particles was used to infect 1 x 10⁶ HEK293T cells. After 48 hrs, 8 µg/ml puromycin was added for stressed screening, and finally stably transfected HEK-hBCMA cells with high expression of human BCMA were isolated. Cell suspensions of HEK293-hGPRC5D and HEK293-hBCMA stably transfected cells in logarithmic growth phase were adjusted to 5x10⁵ cells/ml. Then, 100 µl/well of the cell suspension was added to a 96-well U-shaped plate, and centrifuged at 300g for 5 min. The supernatant was discarded. 100 µL per well of serially diluted antibody was added, and incubated at 4°C for 60 min. The secondary antibody 50 µL/well of Alexa Fluro647 labeled goat anti-human IgG Fc (1:300 dilution) was added, and incubated on ice for 20 min. After washing once, 50 µL/well of PI solution was added, incubated for 5 min, and detected by flow cytometry. The result shows that the GPRC5D×BCMA×CD3 triTCE trispecific antibody can bind to both the GPRC5D receptor and BCMA receptor on the cell membrane (Fig. 14, and Fig. 15), with an affinity (EC50) of about 2.7-14.1 nM (Table 10).

### (iii) Binding with Jurkat cells

Jurkat cells in logarithmic growth phase were added with 200 µg/mL mouse IgG, and allowed to stand in an ice bath for 30 min. The cells were adjusted with 4% calf serum to 5×10⁵ cells/mL. 100 µL per well was added to a 96-well U-shaped plate, and centrifuged at 300 g. The supernatant was removed. 100 µL per well of serially diluted antibody was added, and incubated at 4°C for 60 min. The secondary antibody 50 µL/well of Alexa Fluro647 labeled goat anti-human IgG Fc (1:300 dilution) was added, and incubated on ice for 20 min. After washing once, 50 µL/well of PI was added, incubated for 5 min, and detected by flow cytometry. The result shows that The GPRC5D×BCMA×CD3 triTCE trispecific antibody can bind to Jurkat cells with a low affinity. At the highest concentration of 200 nM, the antibody binding can still not reach a plateau (Fig. 16).

The binding data from BLI and FACS shows that compared with the GPRC5D×CD3κλ bispecific antibody or BCMA×CD3κλ bispecific antibody, the affinity of the GPRC5D×BCMA×CD3 triTCE trispecific antibody binding to BCMA, GPRC5D and CD3 is basically the same (Table 10).

**Table 10. Binding activity of GPRC5D×BCMA×CD3 triTCE antibody**

| Bi-, multi-specific antibody | BCMA KD (nM) | CD3εγ KD (nM) | Human GPRC5D EC50(nM) | Human BCMA EC50 (nM) | Jurkat FACS |
|---|---|---|---|---|---|
| GPRC5D×BCMA ×CD3 triTCE-v1 | 1.8 | Not detected | 5.7 | 11.8 | ++ |
| GPRC5D×BCMA ×CD3 triTCE-v2 | 1.5 | Not detected | 7.9 | 14.1 | ++ |
| GPRC5D×BCMA | Not | 58.2 | 2.7 | 9.1 | ++ |
| ×CD3 triTCE-v3 | detected | | | | |
| GPRC5D×BCMA ×CD3 triTCE-v4 | Not detected | 64.4 | 9.9 | 10.2 | ++ |
| 2F1×CD3 H4K5 | Not detected | Not detected | 8.4 | Not detected | Not detected |
| BCMA×CD3 κλ005 | 1.1 | 45.4 | Not detected | 1.9 | Not detected |
| anti-KLH×CD3 | Not detected | Not detected | - | - | ++ |

### Example 9. Killing of GPRC5D+ and BCMA+ cell lines by GPRC5D×BCMA×CD3 triTCE trispecific antibody

Freshly isolated PBMCs were respectively mixed with the target cells NCI-H929 (purchased from ATCC; product number: CRL-9068), MM.1S, HEK293-hGPRC5D or HEK293-hBCMA stably transfected cells in logarithmic growth phase, at a ratio of effector cell: target cell =10:1. 50 µL per well of serially diluted antibody was added (initial antibody concentration: 66.7 nM, 10-fold dilution, 8 gradients), and cultured at 37°C with 5%CO₂ for 24 hrs. After culture, 50 µL of the supernatant was transferred to a new black microplate, and 50 µL/well of substrate for LDH detection was added. After 10 min, the reaction was terminated and the release of LDH was detected. The results are shown in Fig. 17. GPRC5D×BCMA×CD3 triTCE trispecific antibody can effectively kill BCMA⁺ monopositive target cells (Fig. 17A), GPRC5D⁺ monopositive target cells (Fig. 17B), and myeloma cell lines MM.1S (Fig. 17C) and NCI-H929 (Fig. 17D) expressing both GPRC5D and BCMA receptors (Verkleij et al., Preclinical activity and determinants of response of the GPRC5DxCD3 bispecific antibody talquetamab in multiple myeloma, Blood Adv. 2021 Apr 27;5(8); Pillarisetti et al., A T-cell-redirecting bispecific G-protein-coupled receptor class 5 member D x CD3 antibody to treat multiple myeloma, Blood. 2020;135(15); and Pillarisetti et al., Teclistamab is an active T cell-redirecting bispecific antibody against B-cell maturation antigen for multiple myeloma, Blood Adv. 2020 Sep 22;4(18)). The killing activity mediated by GPRC5D×BCMA×CD3 triTCE-v1 and v2 against the tumor cells is obviously superior to that of the parent antibody GPRC5D×CD3 bispecific antibody or BCMA×CD3 bispecific antibody (Table 11).

**Table 11. TDCC activity of GPRC5D×BCMA×CD3 triTCE**

| TDCC EC50 (nM) | HEK293-hBCMA | HEK293-hGPRC5D | MM.1S | NCI-H929 |
|---|---|---|---|---|
| GPRC5D×BCMA ×CD3 triTCE-v1 | 0.016 | 0.074 | 0.006 | 0.006 |
| GPRC5D×BCMA ×CD3 triTCE-v2 | 0.037 | 0.045 | 0.005 | 0.007 |
| GPRC5D×BCMA ×CD3 triTCE-v3 | 0.033 | 0.278 | 1.880 | 0.185 |
| GPRC5D×BCMA ×CD3 triTCE-v4 | 0.019 | 0.446 | 2.060 | 0.269 |
| 2F1×CD3 H4K5 | Not detected | 0.014 | 0.017 | 0.036 |
| BCMA×CD3 κλ005 | 0.015 | Not detected | 0.072 | 0.021 |

## Claims

1. An antibody binding GPRC5D antigen or an antigen binding fragment thereof, comprising:
(1) a heavy chain variable region and a light chain variable region, wherein
(i) the heavy chain variable region comprises:
(a) an HCDR1 sequence having a formula of:
TSYNMH (SEQ ID NO. 5);
(b) an HCDR2 sequence having a formula of:
YIYPGNGGTNYNQX₁FX₂X₃ (SEQ ID NO. 242), in which
X₁ is K or N, X₂ is K or Q, and X₃ is A or G; and
(c) an HCDR3 sequence having a formula of:
GYGYRYWYFDV (SEQ ID NO. 7); and
(ii) the light chain variable region comprises:
(a) an LCDR1 sequence having a formula of:
X₄ASSSVX₅X₆MH (SEQ ID NO. 243), in which
X₄ is S or R, X₅ is S or R, and X₆ is Y or F;
(b) an LCDR2 sequence having a formula of:
DTSKX₇AS (SEQ ID NO. 244), in which
X₇ is V or L; and
(c) an LCDR3 sequence having a formula of:
QQWX₈X₉X₁₀PX₁₁T (SEQ ID NO.245), in which
X₈ is S or N, X₉ is S or N, X₁₀ is N, YorK, and X₁₁ is L or P; or
(2) a heavy chain variable region and a light chain variable region, wherein
(i) the heavy chain variable region comprises:
(a) an HCDR1 sequence having a formula of:
TGYTMN (SEQ ID NO. 25);
(b) an HCDR2 sequence having a formula of:
LINPYNGGX₁₂X₁₃YNX₁₄KFX₁₅G (SEQ ID NO.246), in which
X₁₂ is S or T, X₁₃ is R, S or N, X₁₄ is Q or L, and X₁₅ is K or Q; and
(c) an HCDR3 sequence having a formula of:
X₁₆X₁₇X₁₈RX₁₉X₂₀MDY (SEQ ID NO. 247), in which
X₁₆ is W or L, X₁₇ is A, P or G, X₁₈ is I or L, X₁₉ is Y or N, and X₂₀ is A or G; and
(ii) the light chain variable region comprises:
(a) an LCDR1 sequence having a formula of:
X₂₁ASQX₂₂X₂₃X₂₄X₂₅NX₂₆X₂₇ (SEQ ID NO. 248), in which
X₂₁ is K, R or Q, X₂₂ is N or S, X₂₃ is V or I, X₂₄ is G or S, X₂₅ is T or N, X₂₆ is V or L, and X₂₇ is A or H;
(b) an LCDR2 sequence having a formula of:
X₂₈ASX₂₉X₃₀X₃₁S (SEQ ID NO. 249), in which
X₂₈ is F, Y or S, X₂₉ is Y, L or Q, X₃₀ is R, L or S, and X₃₁ is N or I; and
(c) an LCDR3 sequence having a formula of:
QQX₃₂NSX₃₃X₃₄X₃₅T (SEQ ID NO. 250), in which
X₃₂ is Y or S, X₃₃ is Y or W, X₃₄ is L or P, and X₃₅ is L or Q; or
(3) a heavy chain variable region and a light chain variable region, wherein
(i) the heavy chain variable region comprises:
(a) an HCDR1 sequence having a formula of:
TNFPIE (SEQ ID NO. 42);
(b) an HCDR2 sequence having a formula of:
NFHPYNDVTKYNEKFX₃₆G (SEQ ID NO. 251), in which
X₃₆ is K or Q; and
(c) an HCDR3 sequence having a formula of:
ITRGY (SEQ ID NO. 44); and
(ii) the light chain variable region comprises:
(a) an LCDR1 sequence having a formula of:
KSSQSLLYSRNQKNSLA (SEQ ID NO. 47);
(b) an LCDR2 sequence having a formula of:
WASTRES (SEQ ID NO. 48); and
(c) an LCDR3 sequence having a formula of:
QQYYSYPLT (SEQ ID NO.49); or
(4) a heavy chain variable region and a light chain variable region, wherein
(i) the heavy chain variable region comprises:
(a) an HCDR1 sequence having a formula of:
TSYX₃₇MY (SEQ ID NO. 252), in which
X₃₇ is Y or T;
(b) an HCDR2 sequence having a formula of:
X₃₈INPSX₃₉GX₄₀TX₄₁YNX₄₂KFX₄₃X₄₄ (SEQ ID NO. 253), in which
X₃₈ is G or Y, X₃₉ is N or S, X₄₀ is G or Y, X₄₁ is K or N, X₄₂ is Q or E, X₄₃ is K or Q, and X₄₄ is S, D or G; and
(c) an HCDR3 sequence having a formula of:
X₄₅X₄₆X₄₇X₄₈X₄₉X₅₀X₅₁YX₅₂X₅₃X₅₄X₅₅DY (SEQ ID NO. 254), in which
X₄₅ is G or W, X₄₆ is G or S, X₄₇ is G or L, X₄₈ is R or N, X₄₉ is E or S, X₅₀ is Y or R, X₅₁ is Y or L, X₅₂ is A or Y, X₅₃ is Y or M, X₅₄ is A or absent, and X₅₅ is M or absent; and
(ii) the light chain variable region comprises:
(a) an LCDR1 sequence having a formula of:
X₅₆ASX₅₇X₅₈X₅₉X₆₀X₆₁YX₆₂X₆₃ (SEQ ID NO. 255), in which
X₅₆ is R, Q or S, X₅₇ is Q or S, X₅₈ is D or S, X₅₉ is I or V, X₆₀ is S or absent, X₆₁ is N or S, X₆₂ is L or M, and X₆₃ is N or Y;
(b) an LCDR2 sequence having a formula of:
X₆₄TSX₆₅LX₆₆S (SEQ ID NO. 256), in which
X₆₄ is Y or G, X₆₅ is R or N, and X₆₆ is H or A; and
(c) an LCDR3 sequence having a formula of:
QQX₆₇X₆₈SX₆₉PX₇₀T (SEQ ID NO.257), in which
X₆₇ is R or G, X₆₈ is Y or S, X₆₉ is L or Y, and X₇₀ is Y or L; or
(5) a heavy chain variable region and a light chain variable region, wherein
(i) the heavy chain variable region comprises:
(a) an HCDR1 sequence having a formula of:
TX₇₁YVMX₇₂ (SEQ ID NO. 258), in which
X₇₁ is S or N, and X₇₂ is Y or H;
(b) an HCDR2 sequence having a formula of:
YINPYNDGTKYNEX₇₃FX₇₄G (SEQ ID NO. 259), in which
X₇₃ is K or R, and X₇₄ is K, T or Q; and
(c) an HCDR3 sequence having a formula of:
GGX₇₅X₇₆X₇₇X₇₈X₇₉X₈₀X₈₁X₈₂X₈₃Y (SEQ ID NO. 260), in which
X₇₅ is M or V, X₇₆ is L or R, X₇₇ is T or R, X₇₈ is T or Y, X₇₉ is R or F, X₈₀ is S or absent, X₈₁ is L or absent, X₈₂ is F or absent, and X₈₃ is A, T, V, L or D; and
(ii) the light chain variable region comprises:
(a) an LCDR1 sequence having a formula of:
RASQX₈₄IX₈₅X₈₆X₈₇X₈₈X₈₉ (SEQ ID NO. 261), in which
X₈₄ is R, E or D, X₈₅ is G or S, X₈₆ is G or S, X₈₇ is Y or N, X₈₈ is L or F, and X₈₉ is N or S;
(b) an LCDR2 sequence having a formula of:
AX₉₀SX₉₁LDS (SEQ ID NO. 262), in which
X₉₀ is A or T, and X₉₁ is T or S; and
(c) an LCDR3 sequence having a formula of:
LQYAX₉₂X₉₃PX₉₄T(SEQ ID NO. 263), in which
X₉₂ is S or N, X₉₃ is Y or F, and X₉₄ is P or Y; or
(6) a heavy chain variable region and a light chain variable region, wherein
(i) the heavy chain variable region comprises:
(a) an HCDR1 sequence having a formula of:
SSYGMS (SEQ ID NO. 58);
(b) an HCDR2 sequence having a formula of:
TISSGGSYTYYPDSVKG (SEQ ID NO. 59); and
(c) an HCDR3 sequence having a formula of:
HX₉₅X₉₆X₉₇X₉₈X₉₉X₁₀₀X₁₀₁X₁₀₂DY (SEQ ID NO. 264), in which
X₉₅ is G or E, X₉₆ is G or A, X₉₇ is S or R, X₉₈ is S or A, X₉₉ is S or T, X₁₀₀ is Y or F, X₁₀₁ is V or absent, and X₁₀₂ is M or absent; and
(ii) the light chain variable region comprises:
(a) an LCDR1 sequence having a formula of:
RSSKSLLHSNGNTYLY (SEQ ID NO. 63);
(b) an LCDR2 sequence having a formula of:
RMSNLAS (SEQ ID NO. 64); and
(c) an LCDR3 sequence having a formula of:
MQHLEYPFT (SEQ ID NO. 65); or
(7) a heavy chain variable region and a light chain variable region, wherein
(i) the heavy chain variable region comprises:
(a) an HCDR1 sequence having a formula of:
TRYWMH (SEQ ID NO. 15);
(b) an HCDR2 sequence having a formula of:
EFNPSNGRINYNEKFX₁₀₃X₁₀₄ (SEQ ID NO. 265), in which
X₁₀₃ is K or Q, and X₁₀₄ is N or G; and
(c) an HCDR3 sequence having a formula of:
GFAY (SEQ ID NO. 17); and
(ii) the light chain variable region comprises:
(a) an LCDR1 sequence having a formula of:
X₁₀₅ASSTVSYIH (SEQ ID NO. 266), in which
X₁₀₅ is S or R;
(b) an LCDR2 sequence having a formula of:
DTSKLAS (SEQ ID NO. 21); and
(c) an LCDR3 sequence having a formula of:
QQWTTNPWT (SEQ ID NO. 22).

2. The antibody or antigen binding fragment thereof according to claim 1, comprising a heavy chain CDR1 selected from an amino acid sequence as shown in SEQ ID NO. 5, 15, 25, 42, 58, 68, 98, 108, 119, or 241, or any variant thereof, a heavy chain CDR2 selected from an amino acid sequence as shown in SEQ ID NO. 6, 16, 26, 35, 43, 52, 59, 69, 78, 89, 99, 115, 133, or 147, or any variant thereof, and a heavy chain CDR3 selected from an amino acid sequence as shown in SEQ ID NO. 7, 17, 27, 44, 60, 70, 79, 90, 100, 109, 116, 120, 134, 139, or 148, or any variant thereof; and a light chain CDR1 selected from an amino acid sequence as shown in SEQ ID NO. 10, 20, 30, 38, 47, 63, 73, 82, 93, 103, 112, 123, 130, 167, 173, 179, 190, or 208, or any variant thereof, a light chain CDR2 selected from an amino acid sequence as shown in SEQ ID NO. 11, 21, 31, 48, 64, 74, 83, 94, 104, 124, 151, or 209, or any variant thereof, and a light chain CDR3 selected from an amino acid sequence as shown in SEQ ID NO. 12, 22, 32, 39, 49, 55, 65, 75, 84, 95, 105, 125, or 144, or any variant thereof.

3. The antibody or antigen binding fragment thereof according to claim 1 or 2, comprising a combination of heavy chain and light chain CDRs selected from:
(1) comprising heavy chain CDR1, CDR2, and CDR3 sequences having SEQ ID NOs. 5, 6, and 7 respectively, and comprising light chain CDR1, CDR2, and CDR3 sequences having SEQ ID NOs. 10, 11, and 12 respectively;
(2) comprising heavy chain CDR1, CDR2, and CDR3 sequences having SEQ ID NOs. 15, 16, and 17 respectively, and comprising light chain CDR1, CDR2, and CDR3 sequences having SEQ ID NOs. 20, 21, and 22 respectively;
(3) comprising heavy chain CDR1, CDR2, and CDR3 sequences having SEQ ID NOs. 25, 26, and 27 respectively, and comprising light chain CDR1, CDR2, and CDR3 sequences having SEQ ID NOs. 30, 31, and 32 respectively;
(4) comprising heavy chain CDR1, CDR2, and CDR3 sequences having SEQ ID NOs. 5, 35, and 7 respectively, and comprising light chain CDR1, CDR2, and CDR3 sequences having SEQ ID NOs. 38, 21, and 39 respectively;
(5) comprising heavy chain CDR1, CDR2, and CDR3 sequences having SEQ ID NOs. 42, 43, and 44 respectively, and comprising light chain CDR1, CDR2, and CDR3 sequences having SEQ ID NOs. 47, 48, and 49 respectively;
(6) comprising heavy chain CDR1, CDR2, and CDR3 sequences having SEQ ID NOs. 5, 52, and 7 respectively, and comprising light chain CDR1, CDR2, and CDR3 sequences having SEQ ID NOs. 38, 21, and 55 respectively;
(7) comprising heavy chain CDR1, CDR2, and CDR3 sequences having SEQ ID NOs. 58, 59, and 60 respectively, and comprising light chain CDR1, CDR2, and CDR3 sequences having SEQ ID NOs. 63, 64, and 65 respectively;
(8) comprising heavy chain CDR1, CDR2, and CDR3 sequences having SEQ ID NOs. 68, 69, and 70 respectively, and comprising light chain CDR1, CDR2, and CDR3 sequences having SEQ ID NOs. 73, 74, and 75 respectively;
(9) comprising heavy chain CDR1, CDR2, and CDR3 sequences having SEQ ID NOs. 241, 78, and 79 respectively, and comprising light chain CDR1, CDR2, and CDR3 sequences having SEQ ID NOs. 82, 83, and 84 respectively;
(10) comprising heavy chain CDR1, CDR2, and CDR3 sequences having SEQ ID NOs. 42, 43, and 44 respectively, and comprising light chain CDR1, CDR2, and CDR3 sequences having SEQ ID NOs. 47, 48, and 49 respectively;
(11) comprising heavy chain CDR1, CDR2, and CDR3 sequences having SEQ ID NOs. 25, 89, and 90 respectively, and comprising light chain CDR1, CDR2, and CDR3 sequences having SEQ ID NOs. 93, 94, and 95 respectively;
(12) comprising heavy chain CDR1, CDR2, and CDR3 sequences having SEQ ID NOs. 98, 99, and 100 respectively, and comprising light chain CDR1, CDR2, and CDR3 sequences having SEQ ID NOs. 103, 104, and 105 respectively;
(13) comprising heavy chain CDR1, CDR2, and CDR3 sequences having SEQ ID NOs. 108, 99, and 109 respectively, and comprising light chain CDR1, CDR2, and CDR3 sequences having SEQ ID NOs. 112, 104, and 105 respectively;
(14) comprising heavy chain CDR1, CDR2, and CDR3 sequences having SEQ ID NOs. 98, 115, and 116 respectively, and comprising light chain CDR1, CDR2, and CDR3 sequences having SEQ ID NOs. 112, 104, and 105 respectively;
(15) comprising heavy chain CDR1, CDR2, and CDR3 sequences having SEQ ID NOs. 119, 99, and 120 respectively, and comprising light chain CDR1, CDR2, and CDR3 sequences having SEQ ID NOs. 123, 124, and 125 respectively;
(16) comprising heavy chain CDR1, CDR2, and CDR3 sequences having SEQ ID NOs. 108, 99, and 116 respectively, and comprising light chain CDR1, CDR2, and CDR3 sequences having SEQ ID NOs. 130, 104, and 105 respectively;
(17) comprising heavy chain CDR1, CDR2, and CDR3 sequences having SEQ ID NOs. 108, 133, and 134 respectively, and comprising light chain CDR1, CDR2, and CDR3 sequences having SEQ ID NOs. 112, 104, and 105 respectively;
(18) comprising heavy chain CDR1, CDR2, and CDR3 sequences having SEQ ID NOs. 58, 59, and 139 respectively, and comprising light chain CDR1, CDR2, and CDR3 sequences having SEQ ID NOs. 63, 64, and 65 respectively;
(19) comprising heavy chain CDR1, CDR2, and CDR3 sequences having SEQ ID NOs. 98, 99, and 109 respectively, and comprising light chain CDR1, CDR2, and CDR3 sequences having SEQ ID NOs. 103, 104, and 105 respectively;
(20) comprising heavy chain CDR1, CDR2, and CDR3 sequences having SEQ ID NOs. 98, 99, and 109 respectively, and comprising light chain CDR1, CDR2, and CDR3 sequences having SEQ ID NOs. 103, 104, and 144 respectively;
(21) comprising heavy chain CDR1, CDR2, and CDR3 sequences having SEQ ID NOs. 25, 147, and 148 respectively, and comprising light chain CDR1, CDR2, and CDR3 sequences having SEQ ID NOs. 93, 151, and 95 respectively;
(22) comprising heavy chain CDR1, CDR2, and CDR3 sequences having SEQ ID NOs. 15, 164, and 17 respectively, and comprising light chain CDR1, CDR2, and CDR3 sequences having SEQ ID NOs. 167, 21, and 22 respectively;
(23) comprising heavy chain CDR1, CDR2, and CDR3 sequences having SEQ ID NOs. 25, 170, and 27 respectively, and comprising light chain CDR1, CDR2, and CDR3 sequences having SEQ ID NOs. 173, 31, and 32 respectively;
(24) comprising heavy chain CDR1, CDR2, and CDR3 sequences having SEQ ID NOs. 5, 176, and 7 respectively, and comprising light chain CDR1, CDR2, and CDR3 sequences having SEQ ID NOs. 179, 21, and 39 respectively;
(25) comprising heavy chain CDR1, CDR2, and CDR3 sequences having SEQ ID NOs. 42, 182, and 44 respectively, and comprising light chain CDR1, CDR2, and CDR3 sequences having SEQ ID NOs. 47, 48, and 49 respectively;
(26) comprising heavy chain CDR1, CDR2, and CDR3 sequences having SEQ ID NOs. 68, 187, and 70 respectively, and comprising light chain CDR1, CDR2, and CDR3 sequences having SEQ ID NOs. 190, 74, and 75 respectively;
(27) comprising heavy chain CDR1, CDR2, and CDR3 sequences having SEQ ID NOs. 119, 193, and 120 respectively, and comprising light chain CDR1, CDR2, and CDR3 sequences having SEQ ID NOs. 123, 124, and 125 respectively;
(28) comprising heavy chain CDR1, CDR2, and CDR3 sequences having SEQ ID NOs. 25, 170, and 27 respectively, and comprising light chain CDR1, CDR2, and CDR3 sequences having SEQ ID NOs. 208, 209, and 32 respectively;
(29) comprising heavy chain CDR1, CDR2, and CDR3 sequences having SEQ ID NOs. 25, 170, and 27 respectively, and comprising light chain CDR1, CDR2, and CDR3 sequences having SEQ ID NOs. 173, 209, and 32 respectively; and
(30) comprising heavy chain CDR1, CDR2, and CDR3 sequences having SEQ ID NOs. 25, 170, and 27 respectively, and comprising light chain CDR1, CDR2, and CDR3 sequences having SEQ ID NOs. 208, 31, and 32 respectively.

4. The antibody or antigen binding fragment thereof according to any one of claims 1 to 3, comprising a heavy chain variable region selected from an amino acid sequence as shown in SEQ ID NO. 3, 13, 23, 33, 40, 50, 56, 66, 76, 87, 96, 106, 113, 117, 126, 131, 140, 145, 162, 168, 174, 180, 185, 191, 202, or 232, or any variant thereof, and a light chain variable region selected from an amino acid sequence as shown in SEQ ID NO. 8, 18, 28, 36, 45, 53, 61, 71, 80, 85, 91, 101, 110, 121, 128, 135, 142, 149, 165, 171, 177, 183, 188, 194, 206, 212, 216, 220, or 230 or any variant thereof;
preferably, comprising a heavy chain variable region having an amino acid sequence as shown in SEQ ID NO. 3 or any variant thereof, and a light chain variable region having an amino acid sequence as shown in SEQ ID NO. 8 or any variant thereof;
preferably, comprising a heavy chain variable region having an amino acid sequence as shown in SEQ ID NO. 13 or any variant thereof, and a light chain variable region having an amino acid sequence as shown in SEQ ID NO. 18 or any variant thereof;
preferably, comprising a heavy chain variable region having an amino acid sequence as shown in SEQ ID NO. 23 or any variant thereof, and a light chain variable region having an amino acid sequence as shown in SEQ ID NO. 28 or any variant thereof;
preferably, comprising a heavy chain variable region having an amino acid sequence as shown in SEQ ID NO. 33 or any variant thereof, and a light chain variable region having an amino acid sequence as shown in SEQ ID NO. 36 or any variant thereof;
preferably, comprising a heavy chain variable region having an amino acid sequence as shown in SEQ ID NO. 40 or any variant thereof, and a light chain variable region having an amino acid sequence as shown in SEQ ID NO. 45 or any variant thereof;
preferably, comprising a heavy chain variable region having an amino acid sequence as shown in SEQ ID NO. 50 or any variant thereof, and a light chain variable region having an amino acid sequence as shown in SEQ ID NO. 53 or any variant thereof;
preferably, comprising a heavy chain variable region having an amino acid sequence as shown in SEQ ID NO. 56 or any variant thereof, and a light chain variable region having an amino acid sequence as shown in SEQ ID NO. 61 or any variant thereof;
preferably, comprising a heavy chain variable region having an amino acid sequence as shown in SEQ ID NO. 66 or any variant thereof, and a light chain variable region having an amino acid sequence as shown in SEQ ID NO. 71 or any variant thereof;
preferably, comprising a heavy chain variable region having an amino acid sequence as shown in SEQ ID NO. 76 or any variant thereof, and a light chain variable region having an amino acid sequence as shown in SEQ ID NO. 80 or any variant thereof;
preferably, comprising a heavy chain variable region having an amino acid sequence as shown in SEQ ID NO. 40 or any variant thereof, and a light chain variable region having an amino acid sequence as shown in SEQ ID NO. 85 or any variant thereof;
preferably, comprising a heavy chain variable region having an amino acid sequence as shown in SEQ ID NO. 87 or any variant thereof, and a light chain variable region having an amino acid sequence as shown in SEQ ID NO. 91 or any variant thereof;
preferably, comprising a heavy chain variable region having an amino acid sequence as shown in SEQ ID NO. 96 or any variant thereof, and a light chain variable region having an amino acid sequence as shown in SEQ ID NO. 101 or any variant thereof;
preferably, comprising a heavy chain variable region having an amino acid sequence as shown in SEQ ID NO. 106 or any variant thereof, and a light chain variable region having an amino acid sequence as shown in SEQ ID NO. 110 or any variant thereof;
preferably, comprising a heavy chain variable region having an amino acid sequence as shown in SEQ ID NO. 113 or any variant thereof, and a light chain variable region having an amino acid sequence as shown in SEQ ID NO. 110 or any variant thereof;
preferably, comprising a heavy chain variable region having an amino acid sequence as shown in SEQ ID NO. 117 or any variant thereof, and a light chain variable region having an amino acid sequence as shown in SEQ ID NO. 121 or any variant thereof;
preferably, comprising a heavy chain variable region having an amino acid sequence as shown in SEQ ID NO. 126 or any variant thereof, and a light chain variable region having an amino acid sequence as shown in SEQ ID NO. 128 or any variant thereof;
preferably, comprising a heavy chain variable region having an amino acid sequence as shown in SEQ ID NO. 131 or any variant thereof, and a light chain variable region having an amino acid sequence as shown in SEQ ID NO. 135 or any variant thereof;
preferably, comprising a heavy chain variable region having an amino acid sequence as shown in SEQ ID NO. 137 or any variant thereof, and a light chain variable region having an amino acid sequence as shown in SEQ ID NO. 61 or any variant thereof;
preferably, comprising a heavy chain variable region having an amino acid sequence as shown in SEQ ID NO. 140 or any variant thereof, and a light chain variable region having an amino acid sequence as shown in SEQ ID NO. 101 or any variant thereof;
preferably, comprising a heavy chain variable region having an amino acid sequence as shown in SEQ ID NO. 140 or any variant thereof, and a light chain variable region having an amino acid sequence as shown in SEQ ID NO. 142 or any variant thereof;
preferably, comprising a heavy chain variable region having an amino acid sequence as shown in SEQ ID NO. 145 or any variant thereof, and a light chain variable region having an amino acid sequence as shown in SEQ ID NO. 149 or any variant thereof;
preferably, comprising a heavy chain variable region having an amino acid sequence as shown in SEQ ID NO. 162 or any variant thereof, and a light chain variable region having an amino acid sequence as shown in SEQ ID NO. 165 or any variant thereof;
preferably, comprising a heavy chain variable region having an amino acid sequence as shown in SEQ ID NO. 168 or any variant thereof, and a light chain variable region having an amino acid sequence as shown in SEQ ID NO. 171 or any variant thereof;
preferably, comprising a heavy chain variable region having an amino acid sequence as shown in SEQ ID NO. 174 or any variant thereof, and a light chain variable region having an amino acid sequence as shown in SEQ ID NO. 177 or any variant thereof;
preferably, comprising a heavy chain variable region having an amino acid sequence as shown in SEQ ID NO. 180 or any variant thereof, and a light chain variable region having an amino acid sequence as shown in SEQ ID NO. 183 or any variant thereof;
preferably, comprising a heavy chain variable region having an amino acid sequence as shown in SEQ ID NO. 185 or any variant thereof, and a light chain variable region having an amino acid sequence as shown in SEQ ID NO. 188 or any variant thereof;
preferably, comprising a heavy chain variable region having an amino acid sequence as shown in SEQ ID NO. 191 or any variant thereof, and a light chain variable region having an amino acid sequence as shown in SEQ ID NO. 194 or any variant thereof;
preferably, comprising a heavy chain variable region having an amino acid sequence as shown in SEQ ID NO. 202 or any variant thereof, and a light chain variable region having an amino acid sequence as shown in SEQ ID NO. 206 or any variant thereof;
preferably, comprising a heavy chain variable region having an amino acid sequence as shown in SEQ ID NO. 202 or any variant thereof, and a light chain variable region having an amino acid sequence as shown in SEQ ID NO. 212 or any variant thereof;
preferably, comprising a heavy chain variable region having an amino acid sequence as shown in SEQ ID NO. 202 or any variant thereof, and a light chain variable region having an amino acid sequence as shown in SEQ ID NO. 216 or any variant thereof;
preferably, comprising a heavy chain variable region having an amino acid sequence as shown in SEQ ID NO. 202 or any variant thereof, and a light chain variable region having an amino acid sequence as shown in SEQ ID NO. 220 or any variant thereof; and
preferably, comprising a heavy chain variable region having an amino acid sequence as shown in SEQ ID NO. 232 or any variant thereof, and a light chain variable region having an amino acid sequence as shown in SEQ ID NO. 230 or any variant thereof;
wherein preferably, the antibody or antigen binding fragment thereof is humanized.

5. A nucleic acid encoding the antibody or antigen binding fragment thereof according to any one of claims 1 to 4,
preferably, the nucleic acid comprising a nucleic acid sequence selected from SEQ ID NO. 4, 14, 24, 34, 41, 51, 57, 67, 77, 88, 97, 107, 114, 118, 127, 132, 138, 141, 146, 163, 169, 175, 181, 186, 192, or 203, or any variant thereof, which encodes the heavy chain variable region of the antibody; and a nucleic acid sequence selected from SEQ ID NO. 9, 19, 29, 37, 46, 54, 62, 72, 81, 86, 92, 102, 111, 122, 129, 136, 143, 150, 166, 172, 178, 184, 189, 195, 207, 213, 217, 221, or any variant thereof, which encodes the light chain variable region of the antibody;
further preferably the nucleic acid comprising a nucleotide sequence as shown in SEQ ID NO. 4 or any variant thereof, which encodes the heavy chain variable region, and a nucleotide sequence as shown in SEQ ID NO. 9 or any variant thereof, which encodes the light chain variable region;
further preferably the nucleic acid comprising a nucleotide sequence as shown in SEQ ID NO. 14 or any variant thereof, which encodes the heavy chain variable region, and a nucleotide sequence as shown in SEQ ID NO. 19 or any variant thereof, which encodes the light chain variable region;
further preferably the nucleic acid comprising a nucleotide sequence as shown in SEQ ID NO. 24 or any variant thereof, which encodes the heavy chain variable region, and a nucleotide sequence as shown in SEQ ID NO. 29 or any variant thereof, which encodes the light chain variable region;
further preferably the nucleic acid comprising a nucleotide sequence as shown in SEQ ID NO. 34 or any variant thereof, which encodes the heavy chain variable region, and a nucleotide sequence as shown in SEQ ID NO. 37 or any variant thereof, which encodes the light chain variable region;
further preferably the nucleic acid comprising a nucleotide sequence as shown in SEQ ID NO. 41 or any variant thereof, which encodes the heavy chain variable region, and a nucleotide sequence as shown in SEQ ID NO. 46 or any variant thereof, which encodes the light chain variable region;
further preferably the nucleic acid comprising a nucleotide sequence as shown in SEQ ID NO. 51 or any variant thereof, which encodes the heavy chain variable region, and a nucleotide sequence as shown in SEQ ID NO. 54 or any variant thereof, which encodes the light chain variable region;
further preferably the nucleic acid comprising a nucleotide sequence as shown in SEQ ID NO. 57 or any variant thereof, which encodes the heavy chain variable region, and a nucleotide sequence as shown in SEQ ID NO. 62 or any variant thereof, which encodes the light chain variable region;
further preferably the nucleic acid comprising a nucleotide sequence as shown in SEQ ID NO. 67 or any variant thereof, which encodes the heavy chain variable region, and a nucleotide sequence as shown in SEQ ID NO. 72 or any variant thereof, which encodes the light chain variable region;
further preferably the nucleic acid comprising a nucleotide sequence as shown in SEQ ID NO. 77 or any variant thereof, which encodes the heavy chain variable region, and a nucleotide sequence as shown in SEQ ID NO. 81 or any variant thereof, which encodes the light chain variable region;
further preferably the nucleic acid comprising a nucleotide sequence as shown in SEQ ID NO. 41 or any variant thereof, which encodes the heavy chain variable region, and a nucleotide sequence as shown in SEQ ID NO. 86 or any variant thereof, which encodes the light chain variable region;
further preferably the nucleic acid comprising a nucleotide sequence as shown in SEQ ID NO. 88 or any variant thereof, which encodes the heavy chain variable region, and a nucleotide sequence as shown in SEQ ID NO. 92 or any variant thereof, which encodes the light chain variable region;
further preferably the nucleic acid comprising a nucleotide sequence as shown in SEQ ID NO. 97 or any variant thereof, which encodes the heavy chain variable region, and a nucleotide sequence as shown in SEQ ID NO. 102 or any variant thereof, which encodes the light chain variable region;
further preferably the nucleic acid comprising a nucleotide sequence as shown in SEQ ID NO. 107 or any variant thereof, which encodes the heavy chain variable region, and a nucleotide sequence as shown in SEQ ID NO. 111 or any variant thereof, which encodes the light chain variable region;
further preferably the nucleic acid comprising a nucleotide sequence as shown in SEQ ID NO. 114 or any variant thereof, which encodes the heavy chain variable region, and a nucleotide sequence as shown in SEQ ID NO. 111 or any variant thereof, which encodes the light chain variable region;
further preferably the nucleic acid comprising a nucleotide sequence as shown in SEQ ID NO. 118 or any variant thereof, which encodes the heavy chain variable region, and a nucleotide sequence as shown in SEQ ID NO. 122 or any variant thereof, which encodes the light chain variable region;
further preferably the nucleic acid comprising a nucleotide sequence as shown in SEQ ID NO. 127 or any variant thereof, which encodes the heavy chain variable region, and a nucleotide sequence as shown in SEQ ID NO. 129 or any variant thereof, which encodes the light chain variable region;
further preferably the nucleic acid comprising a nucleotide sequence as shown in SEQ ID NO. 132 or any variant thereof, which encodes the heavy chain variable region, and a nucleotide sequence as shown in SEQ ID NO. 136 or any variant thereof, which encodes the light chain variable region;
further preferably the nucleic acid comprising a nucleotide sequence as shown in SEQ ID NO. 138 or any variant thereof, which encodes the heavy chain variable region, and a nucleotide sequence as shown in SEQ ID NO. 62 or any variant thereof, which encodes the light chain variable region;
further preferably the nucleic acid comprising a nucleotide sequence as shown in SEQ ID NO. 141 or any variant thereof, which encodes the heavy chain variable region, and a nucleotide sequence as shown in SEQ ID NO. 102 or any variant thereof, which encodes the light chain variable region;
further preferably the nucleic acid comprising a nucleotide sequence as shown in SEQ ID NO. 141 or any variant thereof, which encodes the heavy chain variable region, and a nucleotide sequence as shown in SEQ ID NO. 143 or any variant thereof, which encodes the light chain variable region;
further preferably the nucleic acid comprising a nucleotide sequence as shown in SEQ ID NO. 146 or any variant thereof, which encodes the heavy chain variable region, and a nucleotide sequence as shown in SEQ ID NO. 150 or any variant thereof, which encodes the light chain variable region;
further preferably the nucleic acid comprising a nucleotide sequence as shown in SEQ ID NO. 163 or any variant thereof, which encodes the heavy chain variable region, and a nucleotide sequence as shown in SEQ ID NO. 166 or any variant thereof, which encodes the light chain variable region;
further preferably the nucleic acid comprising a nucleotide sequence as shown in SEQ ID NO. 169 or any variant thereof, which encodes the heavy chain variable region, and a nucleotide sequence as shown in SEQ ID NO. 172 or any variant thereof, which encodes the light chain variable region;
further preferably the nucleic acid comprising a nucleotide sequence as shown in SEQ ID NO. 175 or any variant thereof, which encodes the heavy chain variable region, and a nucleotide sequence as shown in SEQ ID NO. 178 or any variant thereof, which encodes the light chain variable region;
further preferably the nucleic acid comprising a nucleotide sequence as shown in SEQ ID NO. 181 or any variant thereof, which encodes the heavy chain variable region, and a nucleotide sequence as shown in SEQ ID NO. 184 or any variant thereof, which encodes the light chain variable region;
further preferably the nucleic acid comprising a nucleotide sequence as shown in SEQ ID NO. 186 or any variant thereof, which encodes the heavy chain variable region, and a nucleotide sequence as shown in SEQ ID NO. 189 or any variant thereof, which encodes the light chain variable region;
further preferably the nucleic acid comprising a nucleotide sequence as shown in SEQ ID NO. 192 or any variant thereof, which encodes the heavy chain variable region, and a nucleotide sequence as shown in SEQ ID NO. 195 or any variant thereof, which encodes the light chain variable region;
further preferably the nucleic acid comprising a nucleotide sequence as shown in SEQ ID NO. 203 or any variant thereof, which encodes the heavy chain variable region, and a nucleotide sequence as shown in SEQ ID NO. 207 or any variant thereof, which encodes the light chain variable region;
further preferably the nucleic acid comprising a nucleotide sequence as shown in SEQ ID NO. 203 or any variant thereof, which encodes the heavy chain variable region, and a nucleotide sequence as shown in SEQ ID NO. 213 or any variant thereof, which encodes the light chain variable region;
further preferably the nucleic acid comprising a nucleotide sequence as shown in SEQ ID NO. 203 or any variant thereof, which encodes the heavy chain variable region, and a nucleotide sequence as shown in SEQ ID NO. 217 or any variant thereof, which encodes the light chain variable region; and
further preferably the nucleic acid comprising a nucleotide sequence as shown in SEQ ID NO. 203 or any variant thereof, which encodes the heavy chain variable region, and a nucleotide sequence as shown in SEQ ID NO. 221 or any variant thereof, which encodes the light chain variable region.

6. A multispecific antibody or an antigen binding fragment thereof, at least comprising:
(a) an antibody binding GPRC5D antigen or an antigen binding fragment thereof according to any one of claims 1 to 4, which is a first antigen binding portion, wherein the GPRC5D antigen is a first antigen, the first antigen binding portion comprises a first heavy chain variable region and a first light chain variable region, and the first antigen binding portion comprises a first binding domain binding the first antigen, wherein
preferably, the first binding domain comprises a first heavy chain CDR selected from an amino acid sequence as shown in SEQ ID NO. 25, 170, or 27, or any variant thereof, a first light chain CDR selected from an amino acid sequence as shown in SEQ ID NO. 31, 32, 173, 208, or 209, or any variant thereof; and
(b) an antibody binding CD3 antigen or an antigen binding fragment thereof, which is a second antigen binding portion, wherein the CD3 antigen is a second antigen, the second antigen binding portion comprises a second heavy chain variable region and a second light chain variable region, and the second antigen binding portion comprises a second binding domain binding the second antigen, wherein the second binding domain comprises a second heavy chain CDR1 selected from an amino acid sequence as shown in SEQ ID NO. 159 or any variant thereof, a second heavy chain CDR2 selected from an amino acid sequence as shown in SEQ ID NO. 160 or any variant thereof, and a second heavy chain CDR3 selected from an amino acid sequence as shown in SEQ ID NO. 161 or any variant thereof; and a second light chain CDR1 selected from an amino acid sequence as shown in SEQ ID NO. 154 or any variant thereof, a second light chain CDR2 selected from an amino acid sequence as shown in SEQ ID NO. 155 or any variant thereof, and a second light chain CDR3 selected from an amino acid sequence as shown in SEQ ID NO. 156 or any variant thereof;
preferably, the second binding domain comprises a second heavy chain variable region selected from an amino acid sequence as shown in SEQ ID NO. 157 or any variant thereof, and a second light chain variable region selected from an amino acid sequence as shown in SEQ ID NO. 152 or any variant thereof;
preferably, the second heavy chain of the second antigen binding portion comprises an amino acid sequence as shown in SEQ ID NO. 222, and a second light chain of the second antigen binding portion comprises an amino acid sequence as shown in SEQ ID NO. 224;
preferably, the first binding domain comprises a first heavy chain CDR1 selected from an amino acid sequence as shown in SEQ ID NO. 25 or any variant thereof, a first heavy chain CDR2 selected from an amino acid sequence as shown in SEQ ID NO. 170 or any variant thereof, and a first heavy chain CDR3 selected from an amino acid sequence as shown in SEQ ID NO. 27 or any variant thereof; a first light chain CDR1 selected from an amino acid sequence as shown in SEQ ID NO. 173, or 208, or any variant thereof, a first light chain CDR2 selected from an amino acid sequence as shown in SEQ ID NO. 31, or 209, or any variant thereof, and a first light chain CDR3 selected from an amino acid sequence as shown in SEQ ID NO. 32 or any variant thereof;
preferably, the first light chain CDR of the first binding domain is selected from: first light chain CDR1, CDR2 and CDR3 sequences comprising an amino acid sequence as shown in SEQ ID NOs. 173, 31, and 32 respectively; first light chain CDR1, CDR2 and CDR3 sequences comprising an amino acid sequence as shown in SEQ ID NOs. 208, 209, and 32 respectively; first light chain CDR1, CDR2 and CDR3 sequences comprising an amino acid sequence as shown in SEQ ID NOs. 173, 209, and 32 respectively; or first light chain CDR1, CDR2 and CDR3 sequences comprising an amino acid sequence as shown in SEQ ID NOs. 208, 31, and 32 respectively; and the first heavy chain CDR of the first binding domain is selected from: first heavy chain CDR1, CDR2 and CDR3 sequences comprising an amino acid sequence as shown in SEQ ID NOs. 25, 170, and 27 respectively;
preferably, the first binding domain comprises a first heavy chain variable region selected from an amino acid sequence as shown in SEQ ID NO. 168, 202, or 232, or any variant thereof, and a first light chain variable region selected from an amino acid sequence as shown in SEQ ID NO. 171, 206, 212, 216, 220, or 230, or any variant thereof;
preferably, the first binding domain comprises a first heavy chain variable region selected from an amino acid sequence as shown in SEQ ID NO. 168 or any variant thereof, and a first light chain variable region selected from an amino acid sequence as shown in SEQ ID NO. 171 or any variant thereof;
preferably, the first binding domain comprises a first heavy chain variable region selected from an amino acid sequence as shown in SEQ ID NO. 202 or any variant thereof, and a first light chain variable region selected from an amino acid sequence as shown in SEQ ID NO. 206 or any variant thereof;
preferably, the first binding domain comprises a first heavy chain variable region selected from an amino acid sequence as shown in SEQ ID NO. 202 or any variant thereof, and a first light chain variable region selected from an amino acid sequence as shown in SEQ ID NO. 212 or any variant thereof;
preferably, the first binding domain comprises a first heavy chain variable region selected from an amino acid sequence as shown in SEQ ID NO. 202 or any variant thereof, and a first light chain variable region selected from an amino acid sequence as shown in SEQ ID NO. 216 or any variant thereof;
preferably, the first binding domain comprises a first heavy chain variable region selected from an amino acid sequence as shown in SEQ ID NO. 202 or any variant thereof, and a first light chain variable region selected from an amino acid sequence as shown in SEQ ID NO. 220 or any variant thereof;
preferably, the first binding domain comprises a first heavy chain variable region selected from an amino acid sequence as shown in SEQ ID NO. 232 or any variant thereof, and a first light chain variable region selected from an amino acid sequence as shown in SEQ ID NO. 230 or any variant thereof;
preferably, the first heavy chain of the first antigen binding portion is selected from an amino acid sequence as shown in SEQ ID NO. 196 or 200, and the first light chain of the first antigen binding portion is selected from an amino acid sequence as shown in SEQ ID NO. 198, 204, 210, 214, or 218; further preferably, the first heavy chain of the first antigen binding portion comprises an amino acid sequence as shown in SEQ ID NO. 196, and the first light chain of the first antigen binding portion comprises an amino acid sequence as shown in SEQ ID NO. 198; further preferably, the first heavy chain of the first antigen binding portion comprises an amino acid sequence as shown in SEQ ID NO. 200, and the first light chain of the first antigen binding portion comprises an amino acid sequence as shown in SEQ ID NO. 204; further preferably, the first heavy chain of the first antigen binding portion comprises an amino acid sequence as shown in SEQ ID NO. 200 and the first light chain of the first antigen binding portion comprises an amino acid sequence as shown in SEQ ID NO. 210; further preferably, the first heavy chain of the first antigen binding portion comprises an amino acid sequence as shown in SEQ ID NO. 200, and the first light chain of the first antigen binding portion comprises an amino acid sequence as shown in SEQ ID NO. 214; further preferably, the first heavy chain of the first antigen binding portion comprises an amino acid sequence as shown in SEQ ID NO. 200, and the first light chain of the first antigen binding portion comprises an amino acid sequence as shown in SEQ ID NO. 218;
further preferably, the multispecific antibody or antigen binding fragment thereof further comprising:
(c) an antibody binding BCMA antigen or an antigen binding fragment thereof, which is a third antigen binding portion, wherein the BCMA antigen is a third antigen, the third antigen binding portion comprises a third heavy chain variable region and a third light chain variable region, and the third antigen binding portion comprises a third binding domain binding the third antigen, wherein
preferably, the third binding domain comprises a third heavy chain variable region selected from an amino acid sequence as shown in SEQ ID NO.268 or any variant thereof, and a third light chain variable region selected from an amino acid sequence as shown in SEQ ID NO. 267 or any variant thereof;
preferably, the third antigen binding portion comprises a third heavy chain having an amino acid sequence as shown in SEQ ID NO. 233 or any variant thereof, and a third light chain having an amino acid sequence as shown in SEQ ID NO. 226 or any variant thereof;
preferably, the multispecific antibody is a trispecific antibody, wherein any two of the first heavy chain variable region, the second heavy chain variable region, and the third heavy chain variable region are connected to a constant region, the remaining heavy chain variable region is not connected to the constant region, and the heavy chain variable region that is not connected to the constant region is connected to any one of the two heavy chain variable regions connected to the constant region, preferably, by a linker; preferably, the heavy chain variable region that is not connected to the constant region is connected to a corresponding light chain variable region by a linker;
preferably, the first heavy chain variable region or first light chain variable region of the first antigen binding portion is connected to the second heavy chain variable region or second light chain variable region of the second antigen binding portion by a linker; preferably, the first heavy chain variable region and the first light chain variable region of the first antigen binding portion are connected by a linker; further preferably, the first light chain variable region of the first antigen binding portion is connected to the second heavy chain variable region of the second antigen binding portion;
preferably, the first heavy chain variable region or first light chain variable region of the first antigen binding portion is connected to the third heavy chain variable region or third light chain variable region of the third antigen binding portion by a linker; preferably, the first heavy chain variable region and the first light chain variable region of the first antigen binding portion are connected by a linker;
preferably, the linker is a flexible linker; further preferably, the linker is (GGGGS)ₙ, in which n=2-5, and specifically the linker is GGGGSGGGGSGGGGS (SEQ ID NO: 231), GSTSGSGKSSEGKG (SEQ ID NO: 273), GSGGGSG (SEQ ID NO: 274), or GGGSGGSG (SEQ ID NO: 275);
further preferably, the trispecific antibody comprises a light chain 1, a heavy chain 1, a light chain 2, and a heavy chain 2;
further preferably, the light chain 1 comprises a third light chain variable region having an amino acid sequence as shown in SEQ ID NO. 267, and the heavy chain 1 comprises a first light chain variable region having an amino acid sequence as shown in SEQ ID NO. 230, a first heavy chain variable region having an amino acid sequence as shown in SEQ ID NO. 232, and a third heavy chain variable region having an amino acid sequence as shown in SEQ ID NO. 268, the light chain 2 comprises a second light chain variable region having an amino acid sequence as shown in SEQ ID NO. 152, and the heavy chain 2 comprises a second heavy chain variable region having an amino acid sequence as shown in SEQ ID NO. 157; further preferably, the light chain 1 comprises an amino acid sequence as shown in SEQ ID NO.226, the heavy chain 1 comprises an amino acid sequence as shown in SEQ ID NO. 228, the light chain 2 comprises an amino acid sequence as shown in SEQ ID NO. 224, and the heavy chain 2 comprises an amino acid sequence as shown in SEQ ID NO. 222;
further preferably, the light chain 1 comprises a third light chain variable region having an amino acid sequence as shown in SEQ ID NO. 267, the heavy chain 1 comprises a first heavy chain variable region having an amino acid sequence as shown in SEQ ID NO. 232, a first light chain variable region having an amino acid sequence as shown in SEQ ID NO. 230, and a third heavy chain variable region having an amino acid sequence as shown in SEQ ID NO. 268, the light chain 2 comprises a second light chain variable region having an amino acid sequence as shown in SEQ ID NO. 152, and the heavy chain 2 comprises a second heavy chain variable region having an amino acid sequence as shown in SEQ ID NO. 157; further preferably, the light chain 1 comprises an amino acid sequence as shown in SEQ ID NO.226, the heavy chain 1 comprises an amino acid sequence as shown in SEQ ID NO. 234, the light chain 2 comprises an amino acid sequence as shown in SEQ ID NO. 224, and the heavy chain 2 comprises an amino acid sequence as shown in SEQ ID NO. 222;
further preferably, the light chain 1 comprises a second light chain variable region having an amino acid sequence as shown in SEQ ID NO. 152, the heavy chain 1 comprises a first light chain variable region having an amino acid sequence as shown in SEQ ID NO. 230, a first heavy chain variable region having an amino acid sequence as shown in SEQ ID NO. 232 and a second heavy chain variable region having amino acid sequence as shown in SEQ ID NO. 157, the light chain 2 comprises a third light chain variable region having an amino acid sequence as shown in SEQ ID NO. 267, and the heavy chain 2 comprises a third heavy chain variable region having an amino acid sequence as shown in SEQ ID NO. 268; further preferably, the light chain 1 comprises an amino acid sequence as shown in SEQ ID NO. 224, the heavy chain 1 comprises an amino acid sequence as shown in SEQ ID NO. 236, the light chain 2 comprises an amino acid sequence as shown in SEQ ID NO. 226, and the heavy chain 2 comprises an amino acid sequence as shown in SEQ ID NO. 233;
further preferably, the light chain 1 comprises a second light chain variable region having an amino acid sequence as shown in SEQ ID NO. 152, the heavy chain 1 comprises a first heavy chain variable region having an amino acid sequence as shown in SEQ ID NO. 232, a first light chain variable region having an amino acid sequence as shown in SEQ ID NO. 230, and a second heavy chain variable region having an amino acid sequence as shown in SEQ ID NO. 157, the light chain 2 comprises a third light chain variable region having an amino acid sequence as shown in SEQ ID NO. 267, and the heavy chain 2 comprises a third heavy chain variable region having an amino acid sequence as shown in SEQ ID NO. 268; and further preferably, the light chain 1 comprises an amino acid sequence as shown in SEQ ID NO.224, the heavy chain 1 comprises an amino acid sequence as shown in SEQ ID NO. 239, the light chain 2 comprises an amino acid sequence as shown in SEQ ID NO. 226, and the heavy chain 2 comprises an amino acid sequence as shown in SEQ ID NO. 233.

7. A nucleic acid encoding the multispecific antibody or antigen binding fragment thereof according to claim 6,
wherein preferably, the multispecific antibody is a bispecific antibody, wherein the nucleic acid encoding the first heavy chain variable region of the first antigen binding portion of the bispecific antibody selected from a nucleotide sequence as shown in SEQ ID NO. 169 or SEQ ID NO. 203, the nucleic acid encoding the first light chain variable region of the first antigen binding portion selected from a nucleotide sequence as shown in SEQ ID NO. 172, SEQ ID NO. 207, SEQ ID NO. 213, SEQ ID NO. 217, or SEQ ID NO. 221, the nucleic acid encoding the second heavy chain variable region of the second antigen binding portion comprises a nucleotide sequence as shown in SEQ ID NO. 158, and the nucleic acid encoding the second light chain variable region of the second antigen binding portion comprises a nucleotide sequence as shown in SEQ ID NO. 153; further preferably, the nucleic acid encoding the first heavy chain of the first antigen binding portion of the bispecific antibody selected from a nucleotide sequence as shown in SEQ ID NO. 197 or SEQ ID NO. 201, the nucleic acid encoding the first light chain of the first antigen binding portion selected from a nucleotide sequence as shown in SEQ ID NO. 199, SEQ ID NO. 205, SEQ ID NO. 211, SEQ ID NO. 215, or SEQ ID NO. 219, the nucleic acid encoding the second heavy chain of the second antigen binding portion comprises a nucleotide sequence as shown in SEQ ID NO. 223, and the nucleic acid encoding the second light chain of the second antigen binding portion comprises a nucleotide sequence as shown in SEQ ID NO. 225;
further preferably, the nucleic acid encoding the first heavy chain variable region of the first antigen binding portion of the bispecific antibody comprises a nucleotide sequence as shown in SEQ ID NO. 169, the nucleic acid encoding the first light chain variable region of the first antigen binding portion comprises a nucleotide sequence as shown in SEQ ID NO. 172, the nucleic acid encoding the second heavy chain variable region of the second antigen binding portion comprises a nucleotide sequence as shown in SEQ ID NO. 158, and the nucleic acid encoding the second light chain variable region of the second antigen binding portion comprises a nucleotide sequence as shown in SEQ ID NO. 153; further preferably, the nucleic acid encoding the first heavy chain of the first antigen binding portion of the bispecific antibody comprises a nucleotide sequence as shown in SEQ ID NO. 197, the nucleic acid encoding the first light chain of the first antigen binding portion comprises a nucleotide sequence as shown in SEQ ID NO. 199, the nucleic acid encoding the second heavy chain of the second antigen binding portion comprises a nucleotide sequence as shown in SEQ ID NO. 223, and the nucleic acid encoding the second light chain of the second antigen binding portion comprises a nucleotide sequence as shown in SEQ ID NO. 225;
further preferably, the nucleic acid encoding the first heavy chain variable region of the first antigen binding portion of the bispecific antibody comprises a nucleotide sequence as shown in SEQ ID NO. 203, the nucleic acid encoding the first light chain variable region of the first antigen binding portion comprises a nucleotide sequence as shown in SEQ ID NO. 207, the nucleic acid encoding the second heavy chain variable region of the second antigen binding portion comprises a nucleotide sequence as shown in SEQ ID NO. 158, and the nucleic acid encoding the second light chain variable region of the second antigen binding portion comprises a nucleotide sequence as shown in SEQ ID NO. 153; further preferably, the nucleic acid encoding the first heavy chain of the first antigen binding portion of the bispecific antibody has comprises a nucleotide sequence as shown in SEQ ID NO. 201, the nucleic acid encoding the first light chain of the first antigen binding portion has comprises a nucleotide sequence as shown in SEQ ID NO. 205, the nucleic acid encoding the second heavy chain of the second antigen binding portion comprises a nucleotide sequence as shown in SEQ ID NO. 223, and the nucleic acid encoding the second light chain of the second antigen binding portion comprises a nucleotide sequence as shown in SEQ ID NO. 225;
further preferably, the nucleic acid encoding the first heavy chain variable region of the first antigen binding portion of the bispecific antibody comprises a nucleotide sequence as shown in SEQ ID NO. 203, the nucleic acid encoding the first light chain variable region of the first antigen binding portion comprises a nucleotide sequence as shown in SEQ ID NO. 213, the nucleic acid encoding the second heavy chain variable region of the second antigen binding portion comprises a nucleotide sequence as shown in SEQ ID NO. 158, and the nucleic acid encoding the second light chain variable region of the second antigen binding portion comprises a nucleotide sequence as shown in SEQ ID NO. 153; further preferably, the nucleic acid encoding the first heavy chain of the first antigen binding portion of the bispecific antibody comprises a nucleotide sequence as shown in SEQ ID NO. 201, the nucleic acid encoding the first light chain of the first antigen binding portion comprises a nucleotide sequence as shown in SEQ ID NO. 211, the nucleic acid encoding the second heavy chain of the second antigen binding portion comprises a nucleotide sequence as shown in SEQ ID NO. 223, and the nucleic acid encoding the second light chain of the second antigen binding portion comprises a nucleotide sequence as shown in SEQ ID NO. 225;
further preferably, the nucleic acid encoding the first heavy chain variable region of the first antigen binding portion of the bispecific antibody comprises a nucleotide sequence as shown in SEQ ID NO. 203, the nucleic acid encoding the first light chain variable region of the first antigen binding portion comprises a nucleotide sequence as shown in SEQ ID NO. 217, the nucleic acid encoding the second heavy chain variable region of the second antigen binding portion comprises a nucleotide sequence as shown in SEQ ID NO. 158, and the nucleic acid encoding the second light chain variable region of the second antigen binding portion comprises a nucleotide sequence as shown in SEQ ID NO. 153; further preferably, the nucleic acid encoding the first heavy chain of the first antigen binding portion of the bispecific antibody comprises a nucleotide sequence as shown in SEQ ID NO. 201, the nucleic acid encoding the first light chain of the first antigen binding portion comprises a nucleotide sequence as shown in SEQ ID NO. 215, the nucleic acid encoding the second heavy chain of the second antigen binding portion comprises a nucleotide sequence as shown in SEQ ID NO. 223, and the nucleic acid encoding the second light chain of the second antigen binding portion comprises a nucleotide sequence as shown in SEQ ID NO. 225;
further preferably, the nucleic acid encoding the first heavy chain variable region of the first antigen binding portion of the bispecific antibody comprises a nucleotide sequence as shown in SEQ ID NO. 203, the nucleic acid encoding the first light chain variable region of the first antigen binding portion comprises a nucleotide sequence as shown in SEQ ID NO. 221, the nucleic acid encoding the second heavy chain variable region of the second antigen binding portion comprises a nucleotide sequence as shown in SEQ ID NO. 158, and the nucleic acid encoding the second light chain variable region of the second antigen binding portion comprises a nucleotide sequence as shown in SEQ ID NO. 153; further preferably, the nucleic acid encoding the first heavy chain of the first antigen binding portion of the bispecific antibody comprises a nucleotide sequence as shown in SEQ ID NO. 201, the nucleic acid encoding the first light chain of the first antigen binding portion comprises a nucleotide sequence as shown in SEQ ID NO. 219, the nucleic acid encoding the second heavy chain of the second antigen binding portion comprises a nucleotide sequence as shown in SEQ ID NO. 223, and the nucleic acid encoding the second light chain of the second antigen binding portion comprises a nucleotide sequence as shown in SEQ ID NO. 225;
and preferably, the multispecific antibody is a trispecific antibody, and the trispecific antibody comprises a light chain 1, a heavy chain 1, a light chain 2, and a heavy chain 2,
wherein further preferably the nucleic acid encoding the light chain 1 comprises a nucleotide sequence as shown in SEQ ID NO. 269, the nucleic acid encoding the heavy chain 1 comprises a nucleotide sequence as shown in SEQ ID NO. 271, a nucleotide sequence as shown in SEQ ID NO. 272, or a nucleotide sequence as shown in SEQ ID NO. 270, the nucleic acid encoding the light chain 2 comprises a nucleotide sequence as shown in SEQ ID NO. 153, and the nucleic acid encoding the heavy chain 2 comprises a nucleotide sequence as shown in SEQ ID NO. 158; further preferably, the nucleic acid encoding the light chain 1 of the trispecific antibody comprises a nucleotide sequence as shown in SEQ ID NO. 227, the nucleic acid encoding the heavy chain 1 comprises a nucleotide sequence as shown in SEQ ID NO. 229, the nucleic acid encoding the light chain 2 comprises a nucleotide sequence as shown in SEQ ID NO. 225, and the nucleic acid encoding the heavy chain 2 comprises a nucleotide sequence as shown in SEQ ID NO. 223;
further preferably, the nucleic acid encoding the light chain 1 comprises a nucleotide sequence as shown in SEQ ID NO. 269, the nucleic acid encoding the heavy chain 1 comprises a nucleotide sequence as shown in SEQ ID NO. 272, a nucleotide sequence as shown in SEQ ID NO. 271, or a nucleotide sequence as shown in SEQ ID NO. 270, the nucleic acid encoding the light chain 2 comprises a nucleotide sequence as shown in SEQ ID NO. 153, and the nucleic acid encoding the heavy chain 2 comprises a nucleotide sequence as shown in SEQ ID NO. 158; further preferably, the nucleic acid encoding the light chain 1 of the multispecific antibody comprises a nucleotide sequence as shown in SEQ ID NO. 227, the nucleic acid encoding the heavy chain 1 comprises a nucleotide sequence as shown in SEQ ID NO. 235, the nucleic acid encoding the light chain 2 comprises a nucleotide sequence as shown in SEQ ID NO. 225, and the nucleic acid encoding the heavy chain 2 comprises a nucleotide sequence as shown in SEQ ID NO. 223;
further preferably, the nucleic acid encoding the light chain 1 comprises a nucleotide sequence as shown in SEQ ID NO. 153, the nucleic acid encoding the heavy chain 1 comprises a nucleotide sequence as shown in SEQ ID NO. 271, a nucleotide sequence as shown in SEQ ID NO. 272, or a nucleotide sequence as shown in SEQ ID NO. 158, the nucleic acid encoding the light chain 2 comprises a nucleotide sequence as shown in SEQ ID NO. 269, and the nucleic acid encoding the heavy chain 2 comprises a nucleotide sequence as shown in SEQ ID NO. 270; further preferably, the nucleic acid encoding the light chain 1 of the multispecific antibody comprises a nucleotide sequence as shown in SEQ ID NO. 225, the nucleic acid encoding the heavy chain 1 comprises a nucleotide sequence as shown in SEQ ID NO. 237, the nucleic acid encoding the light chain 2 comprises a nucleotide sequence as shown in SEQ ID NO. 227, and the nucleic acid encoding the heavy chain 2 comprises a nucleotide sequence as shown in SEQ ID NO. 238;
further preferably, the nucleic acid encoding the light chain 1 comprises a nucleotide sequence as shown in SEQ ID NO. 153, the nucleic acid encoding the heavy chain 1 comprises a nucleotide sequence as shown in SEQ ID NO. 272, a nucleotide sequence as shown in SEQ ID NO. 271, or a nucleotide sequence as shown in SEQ ID NO. 158, the nucleic acid encoding the light chain 2 comprises a nucleotide sequence as shown in SEQ ID NO. 269, and the nucleic acid encoding the heavy chain 2 comprises a nucleotide sequence as shown in SEQ ID NO. 270; and further preferably the nucleic acid encoding the light chain 1 of the multispecific antibody comprises a nucleotide sequence as shown in SEQ ID NO. 225, the nucleic acid encoding the heavy chain 1 comprises a nucleotide sequence as shown in SEQ ID NO. 240, the nucleic acid encoding the light chain 2 comprises a nucleotide sequence as shown in SEQ ID NO. 227, and the nucleic acid encoding the heavy chain 2 comprises a nucleotide sequence as shown in SEQ ID NO. 238.

8. A vector comprising the nucleic acid according to claim 5 or 7.

9. A cell comprising the nucleic acid according to claim 5 or 7, or the vector according to claim 8.

10. A composition, comprising the GPRC5D antibody or antigen binding fragment thereof according to any one of claims 1 to 4, the multispecific antibody or antigen binding fragment thereof according to claim 6, the nucleic acid according to claim 5 or 7, the vector according to claim 8, and/or the cell according to claim 9.

11. A kit, comprising the GPRC5D antibody or antigen binding fragment thereof according to any one of claims 1 to 4, the multispecific antibody or antigen binding fragment thereof according to claim 6, the nucleic acid according to claim 5 or 7, the vector according to claim 8, the cell according to claim 9, and/or the composition according to claim 10.

12. Use of the GPRC5D antibody or antigen binding fragment thereof according to any one of claims 1 to 4, the multispecific antibody or antigen binding fragment thereof according to claim 6, the nucleic acid according to claim 5 or 7, the vector according to claim 8, the cell according to claim 9, and/or the composition according to claim 10 in the preparation of a drug or a kit for diagnosing, treating or preventing cancers or autoimmune diseases,
wherein preferably, the cancers are B-cell related cancers, selected from multiple myeloma, malignant plasmacytoma, Hodgkin's lymphoma, nodular lymphocyte predominant Hodgkin's lymphoma, Kahler's disease, myeloid leukemia, plasmacytic leukemia, plasmacytoma, B-cell prolymphocytic leukemia, hairy cell leukemia, B-cell non-Hodgkin's lymphoma (NHL), acute myelogenous leukemia (AML), chronic lymphocytic leukemia (CLL), acute lymphocytic leukemia (ALL), chronic myelogenous leukemia (CML), follicular lymphoma, Burkitt's lymphoma, marginal zone lymphoma, mantle cell lymphoma, large cell lymphoma, precursor B-cell lymphocytic lymphoma, myeloid leukemia, Waldenström's macroglobulinaemia, diffuse large B-cell lymphoma, follicular lymphoma, marginal zone lymphoma, mucosa-associated lymphoid tissue lymphoma, small lymphocytic lymphoma, mantle cell lymphoma, Burkitt's lymphoma, primary mediastinal (thymic) large B-cell lymphoma, lymphoplasmacytic lymphoma, Waldenström's macroglobulinaemia, nodal marginal zone B-cell lymphoma, splenic marginal zone lymphoma, intravascular large B-cell lymphoma, primary effusion lymphoma, lymphomatoid granulomatosis, T-cell/histiocyte-rich large B-cell lymphoma, primary central nervous system lymphoma, primary cutaneous diffuse large B-cell lymphoma (leg type), EBV-positive diffuse large B-cell lymphoma of the elderly, inflammation-related diffuse large B- cell lymphoma\, intravascular large B-cell lymphoma, ALK-positive large B-cell lymphoma, plasmablastic lymphoma, large B-cell lymphoma produced in HHV8-associated multicentric Castleman's disease, unclassifiable B-cell lymphoma with intermediate features between diffuse large B-cell lymphoma and Burkitt's lymphoma, unclassifiable B-cell lymphoma with intermediate features between diffuse large B-cell lymphoma and classical Hodgkin lymphoma, and other B-cell associated lymphoma; and
preferably, the autoimmune diseases are selected from immune nephropathy, systemic lupus erythematosus or rheumatoid arthritis.
